# EUROPEAN PATENT SPECIFICATION

(11) **EP 1 098 986 B1**
(45) Date of publication and mention of the grant of the patent: **28.12.2011**
(21) Application number: 99934910.3
(22) Date of filing: 21.07.1999
(51) Int. Cl.: C12N 15/85, C12N 5/10, C12Q 1/68, A61K 48/00, C12N 15/00, A01K 67/027, C12N 15/11, C12N 15/86

(54) **A POLYNUCLEOTIDE COMPRISING A UBIQUITOUS CHROMATIN OPENING ELEMENT (UCOE)**
EIN EIN ALLGEGENWÄRTIGES CHROMATIN-ÖFFNENDES ELEMENT (UCOE) ENTHALTENDES POLYNUCLEOTID
POLYNUCLEOTIDE COMPORTANT UN ELEMENT D'OUVERTURE DE LA CHROMATINE UBIQUISTE (UCOE)

(30) Priority: 21.07.1998 GB 9815879; 09.11.1998 US 107688 P; 23.03.1999 GB 9906712; 01.04.1999 US 127410 P; 23.04.1999 GB 9909494; 12.05.1999 US 134016 P
(43) Date of publication of application: 16.05.2001
(60) Divisional application: 10183981.9
(73) Proprietor: MILLIPORE CORPORATION, Billerica Massachusetts 01821 (US)
(72) Inventor: ANTONIOU, Michael, Dept. of Experimental Pathology, London Bridge, London SE1 9RT (GB); CROMBIE, Robert, Cobra Therapeutics Limited, Keele ST5 5SP (GB)
(74) Representative: Irvine, Jonquil Claire
(86) International application number: PCT/GB1999/002357
(87) International publication number: WO 2000/005393

(56) References cited:
- EP-A2- 0 848 061
- WO-A-94/13273
- WO-A-94/13273
- ORTIZ B D ET AL: "ADJACENT DNA ELEMENTS DOMINANTLY RESTRICT THE UBIQUITOUS ACTIVITY OF A NOVEL CHROMATIN-OPENING REGION TO SPECIFIC TISSUES" EMBO JOURNAL, OXFORD UNIVERSITY PRESS, SURREY, GB, vol. 16, no. 16, 15 August 1997 (1997-08-15), pages 5037-5045, XP000867213 ISSN: 0261-4189
- TALBOT D, DESCOMBES P, SCHIBLER U.: "The 5' flanking region of the rat LAP (C/EBP beta) gene can direct high-level, position-independent, copy number-dependent expression in multiple tissues in transgenic mice." NUCLEIC ACIDS RES., vol. 22, no. 5, 11 March 1994 (1994-03-11), pages 756-766, XP000867294
- LAVIA ET AL.: "Coincident start sites for divergent transcripts at a randomly selected CpG-rich island of mouse" EMBO JOURNAL, vol. 6, 1987, pages 2773-2779, XP000867291 EYNSHAM, OXFORD GB
- DUHIG T, RUHRBERG C, MOR O, FRIED M.: "The human Surfeit locus." GENOMICS, vol. 52, no. 1, 15 August 1998 (1998-08-15), pages 72-78, XP000870301
- HUXLEY C, FRIED M.: "The mouse surfeit locus contains a cluster of six genes associated with four CpG-rich islands in 32 kilobases of genomic DNA." MOL CELL BIOL., vol. 10, no. 2, February 1990 (1990-02), pages 605-614, XP000870104
- RYAN M T ET AL: "The genes encoding mammalian chaperonin 60 and chaperonin 10 are linked head-to-head and share a bidirectional promoter" GENE: AN INTERNATIONAL JOURNAL ON GENES AND GENOMES,GB,ELSEVIER SCIENCE PUBLISHERS, BARKING, vol. 196, no. 1-2, 1 September 1997 (1997-09-01), pages 9-17, XP004126323 ISSN: 0378-1119
- GAVALAS A, DIXON JE, BRAYTON KA, ZALKIN H.: "Coexpression of two closely linked avian genes for purine nucleotide synthesis from a bidirectional promoter." MOL CELL BIOL., vol. 13, no. 8, August 1993 (1993-08), pages 4784-4792, XP000870098
- OHBAYASHI T, SCHMIDT EE, MAKINO Y, KISHIMOTO T, NABESHIMA Y, MURAMATSU M, TAMURA T: "Promoter structure of the mouse TATA-binding protein (TBP) gene." BIOCHEM BIOPHYS RES COMMUN, vol. 225, no. 1, August 1996 (1996-08), pages 275-280, XP000876687
- FESTENSTEIN, R. ET AL.: "Locus Control Region function and heterochromatin-induced position effect variegation" SCIENCE., vol. 271, 23 February 1996 (1996-02-23), pages 1123-1125, XP000876598 AAAS. LANCASTER, PA., US
- KOZU T., HENRICH B., SCHAFER K.P.: "Structure and expression of the gene (HNRPA2B1) encoding the human hnRNP protein A2/B1" GENOMICS 25:365-371(1995)., vol. 25, no. 2, 20 January 1995 (1995-01-20), pages 365-371, XP000870070
- KAMMA HIROSHI ET AL: "Molecular characterization of the hnRNP A2/B1 proteins: Tissue-specific expression and novel isoforms" EXPERIMENTAL CELL RESEARCH, vol. 246, no. 2, 1 February 1999 (1999-02-01), pages 399-411, XP002369363 ISSN: 0014-4827
- ARMSTRONG J A ET AL: "TRANSCRIPTION OF CHROMATIN: THESE ARE COMPLEX TIMES" CURRENT OPINION IN GENETICS & DEVELOPMENT, CURRENT BIOLOGY LTD, vol. 8, no. 2, April 1998 (1998-04), pages 165-172, XP000878773 ISSN: 0959-437X
- KRAMER ET AL.: "Differentiation: the selective potentiation of chromatin domains." DEVELOPMENT., vol. 125, no. 23, December 1998 (1998-12), pages 4749-4755, XP000870263
- DAVEY C ET AL: "CPG METHYLATION REMODELS CHROMATIN STRUCTURE IN VITRO" JOURNAL OF MOLECULAR BIOLOGY, LONDON, GB, vol. 267, 1997, pages 276-288, XP000878942 ISSN: 0022-2836
- NAKAMURA T ET AL: "CLONING AND HETEROGENEOUS IN VIVO EXPRESSION OF TAT BINDING PROTEIN-1 (TBP-1) IN THE MOUSE" BIOCHIMICA ET BIOPHYSICA ACTA, AMSTERDAM, NL, vol. 1399, no. 1, 1998, pages 93-100, XP000870257 ISSN: 0006-3002
- WILLIAMS T ET AL: "THE MOUSE SURFEIT LOCUS CONTAINS A VERY TIGHT CLUSTER OF FOUR HOUSEKEEPING GENES THAT IS CONSERVED THROUGH EVOLUTION" PROCEEDINGS OF THE NATIONAL ACADEMY OF SCIENCES OF USA, NATIONAL ACADEMY OF SCIENCE, WASHINGTON, DC, US, vol. 85, no. 10, May 1988 (1988-05), pages 3527-3530, XP000876703 ISSN: 0027-8424
- BIAMONTI G ET AL: "TWO HOMOLOGOUS GENES, ORIGINATED BY DUPLICATION, ENCODE THE HUMAN HNRNP PROTEINS A2 AND A1" NUCLEIC ACIDS RESEARCH, OXFORD UNIVERSITY PRESS, SURREY, GB, vol. 22, no. 11, 1994, pages 1996-2002, XP002024996 ISSN: 0305-1048
- ORTIZ, BENJAMIN D. ET AL.: " Adjacent DNA elements dominantly restrict the ubiquitous activity of a novel chromatin - opening region to specific tissues." EMBO (EUROPEAN MOLECULAR BIOLOGY ORGANIZATION) JOURNAL, (1997) VOL. 16, no. 16, pages 5037-5045, XP000867213 cited in the application
- TALBOT D, DESCOMBES P, SCHIBLER U.: "The 5' flanking region of the rat LAP (C/EBP beta) gene can direct high-level, position-independent, copy number-dependent expression in multiple tissues in transgenic mice." NUCLEIC ACIDS RES., vol. 22, no. 5, 11 March 1994 (1994-03-11), pages 756-766, XP000867294 cited in the application
- LAVIA ET AL.: EMBO JOURNAL, vol. 6, 1987, pages 2773-2779, XP000867291 EYNSHAM, OXFORD GB cited in the application
- DUHIG T, RUHRBERG C, MOR O, FRIED M.: "The human Surfeit locus." GENOMICS, vol. 52, no. 1, 15 August 1998 (1998-08-15), pages 72-78, XP000870301 cited in the application
- HUXLEY C, FRIED M.: "The mouse surfeit locus contains a cluster of six genes associated with four CpG-rich islands in 32 kilobases of genomic DNA." MOL CELL BIOL., vol. 10, no. 2, February 1990 (1990-02), pages 605-614, XP000870104 cited in the application
- RYAN M T ET AL: "The genes encoding mammalian chaperonin 60 and chaperonin 10 are linked head-to-head and share a bidirectional promoter" GENE: AN INTERNATIONAL JOURNAL ON GENES AND GENOMES,GB,ELSEVIER SCIENCE PUBLISHERS, BARKING, vol. 196, no. 1-2, page 9-17 XP004126323 ISSN: 0378-1119 cited in the application
- GAVALAS A, DIXON JE, BRAYTON KA, ZALKIN H.: "Coexpression of two closely linked avian genes for purine nucleotide synthesis from a bidirectional promoter." MOL CELL BIOL., vol. 13, no. 8, August 1993 (1993-08), pages 4784-4792, XP000870098 cited in the application
- OHBAYASHI T, SCHMIDT EE, MAKINO Y, KISHIMOTO T, NABESHIMA Y, MURAMATSU M, TAMURA T: "Promoter structure of the mouse TATA-binding protein (TBP) gene." BIOCHEM BIOPHYS RES COMMUN, vol. 225, no. 1, August 1996 (1996-08), pages 275-280, XP000876687
- FESTENSTEIN, R. ET AL.: "Locus Control Region function and heterochromatin-induced position effect variegation" SCIENCE., vol. 271, 23 February 1996 (1996-02-23), pages 1123-1125, XP000876598 AAAS. LANCASTER, PA., US cited in the application
- SOND W. ET AL.: "Molecular cloning of the gene encoding the Rhesus monkey beta-amyloid precursor protein:structural characterization and a comparative study with oter species", GENE, vol. 217, 1998, pages 151-164,
- GARDINER-GARDEN M. ET AL.: "CpG Islands in Vertebrate Genomes", J. MOL. BIOL., vol. 196, 1987, pages 261-282,
- LARSEN F. ET AL.: "CpG Islands as Gene Markers in the Human Genome", GENOMICS, vol. 13, 1992, pages 1095-1107,
- SHE X. ET AL.: "Definition, coservation and epigenetics of housekeeping and tissue-enriched genes", BMC GENOMICS, vol. 10, 2009, pages 269-269,
- SAXONOV S. ET AL.: "A genome-wide analysis of CpG dinucleotides in the human genome distinguishes two distinct classes of promoters", PNAS, vol. 103, no. 5, 2006, pages 1412-1417,
- QVIST H. ET AL.: "The TATA-Less, CG-Rich Porcine Dipeptidylpeptidase IV (DPPIV) Promoter Shows Bidirectional Activity", BIOL. CHEM., vol. 379, 1998, pages 75-81, XP009126615,
- BÖHM S. K. ET AL.: "Human dipeptidyl peptidase IV gene promoter: tissue-specific regulation from a TATA-less CG-rich sequence characteristic of a house-keeping gene promoter", BIOCHEM. J., vol. 311, 1995, pages 835-843,
- BIRD A. P.: "CpG-rich islands and the function of DNA methylation", NATURE, vol. 321, 1986, pages 209-213,
- TAZI J. BIRD A.: "Alternative Chromatin Structure at CpG Islands", CELL, vol. 60, 1990, pages 909-920,

## Description

The present invention relates to a polynucleotide comprising a ubiquitous chromatin opening element (UCOE) which is not derived from an LCR. The present invention also relates to a vector comprising the polynucleotide sequence, a host cell comprising the vector, and use of the polynucleotide in therapy.

The current model of chromatin structure in higher eukaryotes postulates that genes are organised in "domains" (Dillon and Grosveld, 1994). Chromatin domains can consist of groups of genes that are expressed in a strictly tissue specific manner such as the human β-globin family (Grosveld *et al.,* 1993), genes that are expressed ubiquitously such as the human TBP/C5 locus (Trachtulec, Z. *et al*., 1997), or a mixture of tissue specific and ubiquitously expressed genes such as murine γ/δ TCR/*dad-1* locus, (Hong *et al*., 1997; Ortiz *et al*., 1997) and the human α-globin locus, (Vyas *et al*., 1992). Genes with two different tissue specificities may also be closely linked. For example, the human growth hormone and chorionic somatomammotropin genes (Jones *et al.,* 1995). Chromatin domains are envisaged to exist in either a closed, "condensed", transcriptionally silent state or in a "de-condensed", open and transcriptionally competent configuration. The establishment of an open chromatin structure characterised by DNase I sensitivity, DNA hypomethylation and histone hyperacetylation, is seen as a pre-requisite to the commencement of gene expression.

The discovery of tissue-specific transcriptional regulatory elements known as locus control regions (LCRs) has provided novel insights into the mechanisms by which a transcriptionally competent, open chromatin domain is established and maintained in certain cases. LCRs are defined by their ability to confer on a gene linked in *cis* host cell type-restricted, integration site independent, copy number-dependent expression of the gene (Grosveld *et al.,* 1987; Lang *et al*., 1988; Greaves *et al.,* 1989; Diaz *et al*., 1994; Carson and Wiles, 1993; Bonifer *et al.,* 1990; Montoliu *et al.,* 1996; Raguz *et al.,* 1998; EP-A-0 332 667) especially as single copy transgenes (Ellis *et al.,* 1996; Raguz *et al.,* 1998). LCRs are able to obstruct the spread of heterochromatin and prevent position effect variegation (Festenstein *et al.,* 1996; Milot *et al*., 1996). This pattern of expression conferred by LCRs suggests that these elements possess a powerful chromatin remodelling capability and are able to establish and maintain a transcriptionally competent, open chromatin domain. In addition, LCRs have been found to possess an inherent transcriptional activating capability that allows them to confer tissue-specific gene expression independent of their cognate promoter (Blom van Assendelft *et al.,* 1989; Collis *et al.,* 1990; Antoniou and Grosveld, 1990; Greaves *et al.,* 1989).

All LCRs are associated with gene domains with a prominent tissue-specific or tissue restricted component and are associated with a series of DNase I hypersensitive sites which can be located either 5' (Grosveld *et al.,* 1987; Carson and Wiles, 1993; Bonifer *et al.,* 1994; Jones *et al.,* 1995; Montoliu *et al.,* 1996) or 3' (Greaves *et al*., 1989) of genes which they regulate. In addition, LCR elements have recently been found to exist between closely spaced genes (Hong *et al.,* 1997; Ortiz *et al*., 1997). An LCR-like element has also been reported to have an intronic location within a gene (Aronow *et al*., 1995). In the few cases that have been investigated, these elements correspond to large clusters of tissue-specific and ubiquitous transcription factor binding sites (Talbot *et al.,* 1990; Philipsen *et al*., 1990; Pruzina *et al.,* 1991; Lake *et al*., 1990; Jarman *et al.,* 1991; Aronow *et al*., 1995).

The discovery of LCRs suggests that the regulatory elements that control tissue-specific gene expression from a given chromatin domain are organised in a hierarchical fashion. The LCR would appear to act as a master switch wherein its activation results in the establishment of an open chromatin structure that has to precede any gene expression. Transcription at the physiologically required level can then be achieved through a direct chromatin interaction between the LCR and the local promoter and enhancer elements of an individual gene via looping out of the intervening DNA (Hanscombe *et al.,* 1991; Wijgerde *et al*., 1995; Dillon *et al*., 1997).

As indicated above, an essential feature of an LCR is its tissue specificity. The tissue specificity of an LCR has been investigated by Ortiz *et al*., (1997), wherein a number of DNase I hypersensitive sites of the T-cell receptor alpha (TCRα) LCR were deleted and an LCR derived element, which opens chromatin in a number of tissues identified. Talbot et al., (1994, NAR, 22, 756-766) describe an LCR-like element that is considered to allow expression of a linked gene in a number of tissues. However, reproducible expression of the linked gene is not obtained. The levels of expression are indicated as having a standard deviation of between 74% from the average value on a per-gene-copy basis where the gene is expressed where transgene copy number is 3 or more. When the copy number is 1 or 2, the gene expression levels are 10 times lower and have a standard deviation of 49% from the average value on a per-gene-copy basis where the gene is expressed. The element disclosed by Talbot *et al*., does not give reproducible expression of a linked gene. This and the high variability of the system clearly limits the use of this system.

The long-term correction of genetically inherited disorders by gene therapy requires the maintenance and sustained expression of the transcription unit at sufficiently high levels to be of therapeutic value. This, may be achieved by one of two approaches. Firstly, transcription units can be stably integrated into the host cell genome using, for example, retroviral (Miller, 1992; Miller *et al*., 1993) or adeno-associated viral (AAV) vectors (Muzyczka, 1992; Kotin, 1994; Flotte and Carter, 1995). Alternatively, therapeutic genes can be incorporated within self-replicating episomal vectors comprising viral origins of replication such as those from EBV (Yates *et al.,* 1985), human papovavirus BK (De Benedetti and Rhoads, 1991; Cooper and Miron, 1993) and BPV-1 (Piirsoo *et al.,* 1996).

Unfortunately, the level of expression that is normally seen from genes that are integrated into the genome is too low or short in duration to be of therapeutic value in most cases. This is due to what are generally known as "position effects". The transcription of the introduced gene is dependent upon its site of integration where it comes under the influence of either competing activating (promoters/enhancers) or more frequently, repressing (chromatin silencing) elements. Position effects continue to impose substantial constraints on the therapeutic efficacy of integrating virus-based vectors of retroviral and adeno-associated viral (AAV) origin. Viral transcriptional regulatory elements are notoriously susceptible to silencing by chromatin elements in the vicinity of integration sites. The inclusion of classical promoter and enhancer elements from highly expressed genes as part of the viral constructs has not solved this major problem (Dai *et al.,* 1992; Lee *et al.,* 1993).

The inclusion of a fully functional LCR as part of the transcription unit overcomes this deficiency since this element can be used to drive a predictable, physiological and sustained level of expression of the desired gene in a specif cell type (see Yeoman and Mellor, 1992; Brines and Klaus, 1993; Needham *et al.* 1992 and 1993; Tewari *et al.,* 1998; Zhumabekov *et al*., 1995). This degree of predictability of expression is vital for a safe and successful gene therapy strategy.

The use of replicating episomal vectors (REVs) offers an attractive alternative to integrating viral vectors for producing long-term gene expression. Firstly, REVs do not pose the same size limitations on the therapeutic transcription unit as do viral vectors, with inserts in excess of 300kb being a possibility (Sun *et al.,* 1994). Secondly, being episomal, REVs do not suffer from potential hazards associated with insertional mutagenesis that is an inherent problem with integrating viral vectors. Lastly, REVs are introduced into the target cells using non-viral delivery systems that can be produced more cheaply at scale than with viral vectors.

It has been demonstrated that both non-replicating, transiently transfected plasmids (Reeves *et al.,* 1985; Archer *et al*., 1992) and REVs (Reeves *et al.,* 1985; Smith *et al.,* 1993) assemble nucleosomes. Assembly on REVs is more organised and resembles native chromatin whereas nucleosomes on transient plasmids are less well ordered and may allow some access of transcription factors to target sequences although gene expression can be inhibited (Archer *et al.,* 1992). It has recently been demonstrated that LCRs are able to confer long-term, tissue-specific gene expression from within REVs (International Patent Application WO 98/07876).

The generation of cultured mammalian cell lines producing high levels of a therapeutic protein product is a major developing industry. Chromatin position effects make this a difficult, time consuming and expensive process. The most commonly used approach to the production of such mammalian "cell factories" relies on gene amplification induced by a combination of a drug resistance gene (e.g. DHFR, glutamine synthetase (Kaufman, 1990)) and high toxic drug concentrations which have to be maintained at all times. The use of vectors possessing LCRs from highly expressed gene domains, greatly simplifies the generation of these cell lines (Needham *et al*., 1992; Needham *et al.,* 1995).

LCRs are also disclosed in the context of transgene expression in Talbot D, et al. (The 5' flanking region of the rat LAP (C/EBPβ) gene can direct high-level, position-independent, copy number-dependent expression in multiple tissues in transgenic mice. Nucleic Acids Research (1994) 22(5), 756-766).

A problem with the use of LCRs is that they are tissue specific and reproducible expression is only obtained in the specific cell type. Accordingly one could not obtain reproducible expression in a tissue type or a number of tissue types for which there is no LCR. Accordingly, there is a need for a UCOE, which is not derived from an LCR.

As indicated above, Ortiz *et al.,* (1997) discloses an LCR derived element, which opens chromatin in a number of tissues. There are a number of problems with the LCR derived element of Ortiz *et al.,* (1997). In particular, the element has to be carefully constructed using recombinant DNA techniques to contain the necessary regions of the LCR and also the element does not give reproducible levels of expression of a linked gene in cells of different types, especially when the element is at single or low (less than 3) transgene copy number.

European Patent Application EP 0 848 061 is directed to control of transgenic expression, and discloses a conceptual system in which the expression of one gene produces a transcription regulatory factor that acts upon a promoter driving another particular gene of interest.

CpG islands are disclosed by Song W. and Lahiri D.K. (Molecular cloning of the gene encoding the Rhesus monkey beta-amyloid precursor protein: structural characterization and a comparative study with other species. Gene, (1998) 217, 151-164). Tazi J. and Bird A. discuss nonmethylated CpG islands including promoters and 5' transcribed regions of genes as representing a model for active chromatin structure (Alternative Chromatin Structure at CpG Islands. Cell, (1990) 60, 909-920).

Ohbayashi T. et al. discuss the promoter structure of the mouse and human TATA-binding protein gene, teaching that ubiquitously-expressed genes often lack a TATA-box but have GC-boxes within the promoters, that in some cases, GC-boxes are known to play a role in transcriptional activation, and that TATA-less promoters frequently direct transcription from multiple sites (Promoter Structure of the Mouse TATA-Binding Protein (TBP) Gene. Biochemical and Biophysical Research Communications, (1996) 225, 275-280). The authors find that vertebrate TBP promoters fall into the class of ubiquitously active, TATA-less and GC-box-containing promoters, and conclude that their findings will be important for understanding how somatic cells regulate TBP gene expression and thus maintain homeostasis.

Bird A.P. discusses CpG islands associated with vertebrate genes, considering the properties of clustered non-methylated CpG islands and their relationship to genes (CpG-rich islands and the function of DNA methylation. Nature, (1986) 321, 209-213).

In Gardiner-Garden M. and Frommer M., sequences of vertebrate genes were screened for the presence of CpG islands, which were analysed in terms of length, nucleotide composition, frequency of CpG dinucleotides, and location relative to the transcription unit of the associated gene (CpG Islands in Vertebrate Genomes. J. Mol. Biol. (1987) 196, 261-282). CpG islands were found to be associated with housekeeping genes and tissue-specific genes. Gardiner-Garden and Frommer discussed possible functions of CpG islands in transcriptional and post-transcriptional regulation of gene expression, and these functions were related by the authors to theories for the maintenance of CpG islands as "methylation-free zones" in germline DNA.

Qvist H. et al. analysed the promoter activity of the porcine DPPIV gene on the basis of transient transfection assays (The TATA-Less, CG-Rich Porcine Dipeptidylpeptidase IV (DPPIV) Promoter Shows Bidirectional Activity. Biol. Chem. (1998) 379, 75-81). In a similar vein, Bohm, S.K. et al. analysed the promoter activity of human DPPIV on the basis of transient transfection assays in a sense orientation (Human dipeptidyl peptidase IV gene promoter: tissue-specific regulation from a TATA-less CG-rich sequence characteristic of a housekeeping gene promoter. Biochem. J. (1995) 311, 835-843).

Elements comprising bi-directional promoters and methylation-free CpG islands have been disclosed; however, there is no disclosure or indication that the elements opens chromatin or maintain chromatin in an open state and facilitate reproducible expression of an operably-linked gene in cells of at least two different tissue types.

The human Surfeit locus spans approximately 60kb and is located on chromosome 9q34.2. The locus comprises bi-directional promoters between the SURF5 and SURF3 genes and between the SURF1 and SURF2 genes (Huxley et al., Mol. Cell. Biol., 10, 605-614, 1990; Duhig et al., Genomics, 52, 72-78, 1998; Williams et al., Mol. Cell. Biol., 6, 4558-4569, 1986). There is no indication that these regions open chromatin or maintain chromatin in an open state and facilitate reproducible expression of an operably-linked gene in cells of at least two different tissue types.

A bi-directional promoter is also disclosed by Brayton et al., (J. Biol. Chem., 269, 5313-5321, 1994) between the avian GPAT and AIRC genes. Again there is no indication that the region opens chromatin or maintain chromatin in an open state and facilitate reproducible expression of an operably-linked gene in cells of at least two different tissue types.

A bi-directional promoter is disclosed by Ryan et al. (Gene, 196, 9-17, 1997) between the mitochondrial chaeronin 60 and chaperonin 10 genes. Again there is no indication that the region opens chromatin or maintain chromatin in an open state and facilitate reproducible expression of an operably-linked gene in cells of at least two different tissue types,

A bi-directional promoter is also disclosed associated with the murine HTF9 gene. Again there is no indication that the region opens chromatin or maintain chromatin in an open state and facilitate reproducible expression of an operably-linked gene in cells of at least two different tissue types.

Palmiter et al.,(PNAS USA, 95, 8428-8430, 1998) and International Patent Application WO 94/13273 disclose an element associated with the metallothionein genes. The element comprises DNase I hypersensitive sites which are not associated with promoters. Furthermore, there is no evidence demonstrating that the element does not open chromatin or maintain chromatin in an open state and facilitate reproducible expression of an operably-linked gene in cells of at least two different tissue types.

The use of non-replicating, transiently transfected plasmids to achieve gene expression by transfecting cells is well known. It is also known that only short term expression (generally less than 72 hours) is achieved using non-replicating, transiently transfected plasmids. The short term of expression is generally considered to be due to the breakdown of the plasmid or loss of the plasmid from the cell. In view of this drawback the use of such plasmids is limited.

A polynucleotide is disclosed comprising a UCOE which opens chromatin or maintains chromatin in an open state and facilitates reproducible expression of an operably-linked gene in cells of at least two different tissue types, wherein the polynucleotide is not derived from a locus control region.

A "locus control region" (LCR) is defined as a genetic element which is obtained from a tissue-specific locus of a eukaryotic host cell and which, when linked to a gene of interest and integrated into a chromosome of a host cell, confers tissue-specific, integration site- independent, copy number-dependent expression on the gene of interest. A polynucleotide derived from an LCR can be any part or parts of an LCR. Preferably, a polynucleotide derived from an LCR is any part of an LCR that functions to open chromatin. An LCR is associated with one or more DNase I hypersensitive (HS) sites that are not associated with a promoter and it is preferred that the UCOE does not comprise HS sites that are not associated with a promoter. HS sites are well known to those skilled in the art and can be identified based on the standard techniques, which are described herein.

The term "facilitates reproducible expression" refers to the capability of the UCOE to facilitate reproducible activation of transcription of the operably-linked gene. The process is believed to involve the ability of the UCOE to render the region of the chromatin encompassing the gene (or at least the transcription factor binding sites) accessible to transcription factors. Reproducible expression preferably means that the polynucleotide when operably-linked to an expressible gene gives substantially the same level of expression of the operably-linked gene irrespective of its chromatin environment and preferably irrespective of the cell tissue type. Preferably, substantially the same level of expression means a level of expression which has a standard deviation from an average value of less than 48%, more preferably less than 40% and most preferably, less than 25% on a per-gene-copy basis. Alternatively, substantially the same level of expression preferably means that the level of expression varies by less than 10 fold, more preferably less than 5 fold and most preferably less than 3 fold on a per gene copy basis. The level of expression is preferably the level of expression measured in a non-human transgenic animal. It is especially preferred that the UCOE facilitates reproducible expression of an operably-linked gene when present at a single or low (less than 3) copy number.

As used herein, "linked" refers to a *cis*-linkage in which the gene and the UCOE are present in a *cis* relationship on the same nucleic acid molecule. The term "operatively linked" refers to a *cis*-linkage in which the gene is subject to expression facilitated by the UCOE.

Open chromatin or chromatin in an open state refers to chromatin in a de-condensed state and is also referred to as euchromatin. Condensed chromatin is also referred to as heterochromatin. As indicated above, chromatin in a closed (condensed) state is transcriptionally silent. Chromatin in an open (de-condensed) state is transcriptionally competent. The establishment of an open chromatin structure is characterised by DNase I sensitivity, DNA hypomethylation and histone hyperacetylation. Standard methods for identifying open chromatin are well known to those skilled in the art and are described in Wu, 1989, Meth. Enzymol., 170, 269-289; Crane-Robinson et al., 1997, Methods, 12, 48-56; Rein et al., 1998, N.A.R., 26, 2255-2264.

The term "cells of two or more tissue types" refers to cells of at least two, preferably at least 4 and more preferably all of the following different tissue types: heart, kidney, lung, liver, gut, skeletal muscle, gonads, spleen, brain and thymus tissue. Preferably, the polynucleotide facilitates reproducible expression non-tissue specifically, i.e. with no tissue specificity. It is further referred that the polynucleotide of the present invention facilitates reproducible expression in at least 50% and more preferably in all tissue types where active gene expression occurs.

Preferably, the polynucleotide of the present invention facilitates reproducible expression of an operably-linked gene at a physiological level. By physiological level, it is meant a level of gene expression at which expression in a cell, population of cells or a patient exhibits a physiological effect. Preferably, the physiological level is an optimal physiological level depending on the desired result. Preferably, the physiological level is equivalent to the level of expression of an equivalent endogenous gene.

The UCOE disclosed herein can be any element, which opens chromatin or maintains chromatin in an open state and facilitates reproducible expression of an operably-linked gene in cells of at least two different tissue types provided it is not derived from an LCR. In a preferred embodiment, the UCOE comprises an extended methylation-free, CpG-island. CpG-islands have an average GC content of approximately 60%, compared with a 40% average in bulk DNA. One skilled in the art can easily identify CpG-islands using standard techniques such as using restriction enzymes specific for C and G sequences. Such techniques are described in Larsen *et al.,* 1992 and Kolsto *et al.,* 1986. An extended methylation-free CpG island is a methylation-free CpG island that extends across a region encompassing more than one transcriptional start site and/or extends for more than 300bp and preferably more than 500bp.

Preferably, the UCOE is derived from a sequence that in its natural endogenous position is associated with, more preferably, located adjacent to, a ubiquitously expressed gene. It is further preferred that the UCOE comprises at least one transcription factor binding site. Transcription factor binding sites include promoter sequences and enhancer sequences. Preferably, the UCOE comprises dual or bi-directional promoters that are divergently transcribed. Dual promoters are defined herein as two or more promoters which are independent from each other so that one of the promoters can be activated or deactivated without effecting the other promoter or promoters. A bi-directional promoter is defined herein as a region that can act as a promoter in both directions but cannot be activated or deactivated in one direction only. Preferably, the UCOE comprises dual promoters. Preferably, the UCOE comprises dual or bi-directional promoters that transcribe divergently (i.e. can lead to transcription in opposite directions) and which in their natural endogenous positions are associated with ubiquitously expressed genes. Preferably, the UCOE comprises dual promoters that are transcribe divergently. The UCOE may comprise a heterologous promoter, i.e. a promoter that is not naturally associated with the other sequences of the UCOE. For example, it is possible to use the CMV promoter with the UCOE associated with the hnRNP A2 and the HP1H-γ promoters, which is discussed further below. Also provided herein is a UCOE comprising one or more heterologous promoters. The heterologous promoter or promoters can replace of one or more of the endogenous promoters of the UCOE or can be used in addition to the one or more endogenous promoters of the UCOE. The heterologous promoter may be any promoter including tissue specific promoters such as tumour-specific promoters and ubiquitous promoters. Preferably the heterologous promoter is a substantially ubiquitous promoter and most preferably is the CMV promoter.

Preferably, the UCOE is not the 3725bp *Eco*RI fragments comprising the bi-directional promoter of the *Hpa*II tiny fragment (HTF) island HTF9 as described in Lavia et al., EMBO J., 6, 2773-2779, (1987).

Preferably, the UCOE is not the 149bp MES-1 element located within a 800bp *Bam*HI genomic fragment located between the murine SURF1 and SURF2 genes of the Surfeit locus (Williams et al., Mol. Cell. Biol, 13, 4784-4792, 1993). Preferably, the UCOE is not the bi-directional promoter located between the SURF5 and the SURF3 genes of the Surfeit locus (Williams et al., Mol. Cell. Biol, 13, 4784-4792, 1993). It is further preferred that the UCOE is not derived from the human surfeit gene locus which spans 60kb and is located on chromosome 9q34.2 as defined in Duhig et al., Genomics, 52, 72-78, (1998) or the corresponding murine locus (Huxley et al., Mol. Cell. Biol., 10, 605-614,1990). Preferably, the UCOE is not the bi-directional promoter region located between the avian GPAT and AIRC genes contained in the 1350bp *Sma*I fragment deposited in the GenBank database (accession no. L12533) (Gavalas et al., Mol. Cell. Biol., 13, 4784-4792, 1993) or the corresponding human equivalent (Brayton et al., J. Biol. Chem., 269, 5313-5321, 1994). Preferably, the UCOE is not the 13894 bp genomic DNA fragment (GenBank accession no. U68562) comprising the rat mitochondrial chaperonin 60 and chaperonin 10 genes. It is also preferred that the UCOE is not the 581bp fragment containing the bi-directional promoter located in the intergenic region between the rat mitochondrial chaperonin 60 and chaperonin 10 genes (Ryan et al., Gene, 196, 9-17, 1997).

Preferably, the UCOE is a 44kb DNA fragment spanning the human TATA binding protein (TBP) gene and 12kb each of the 5' and 3' flanking sequence, or a functional homologue or fragment thereof.

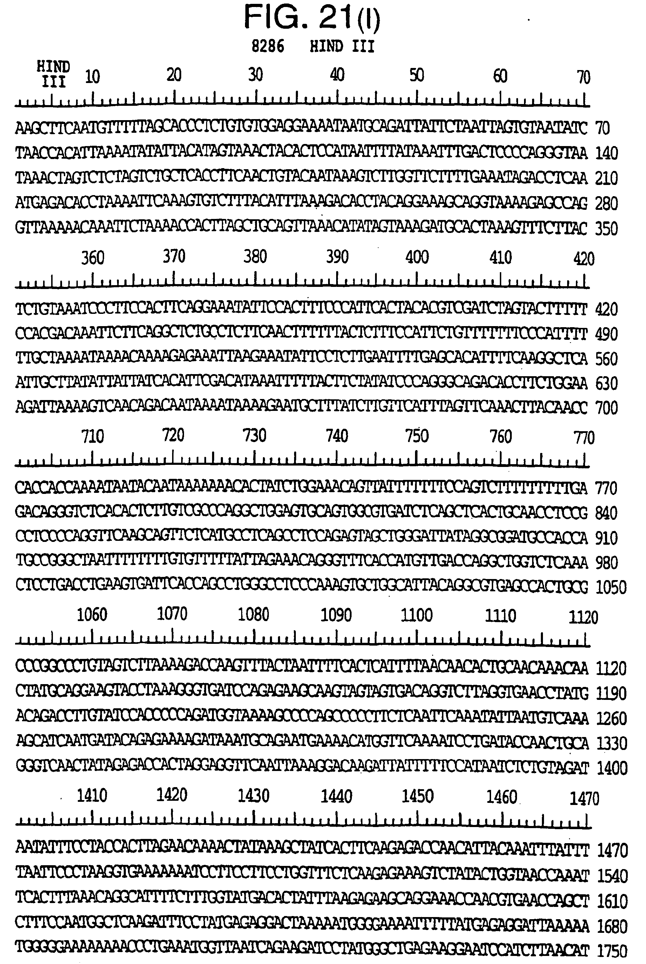

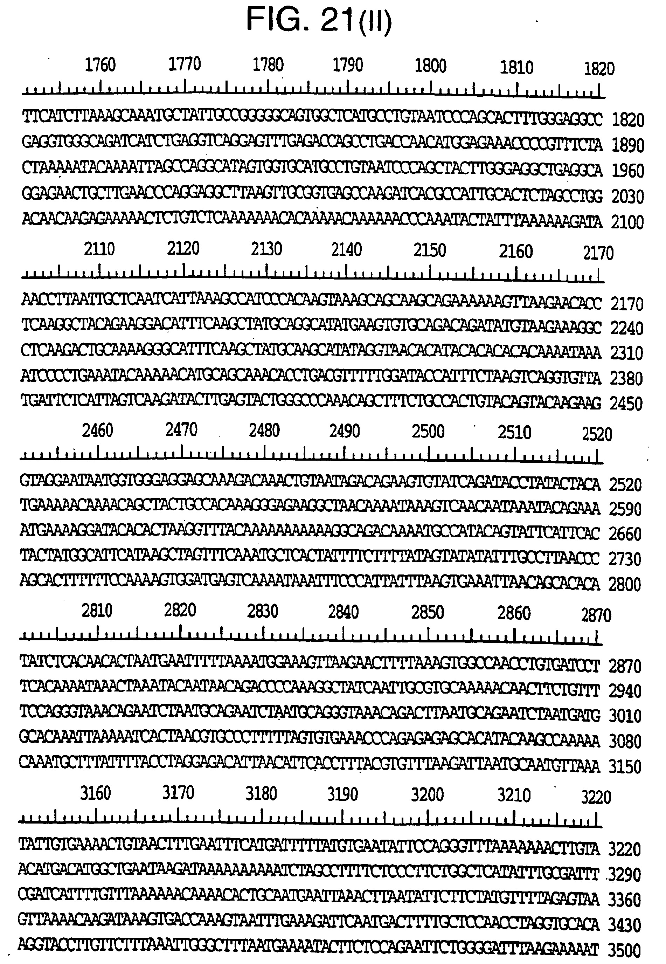

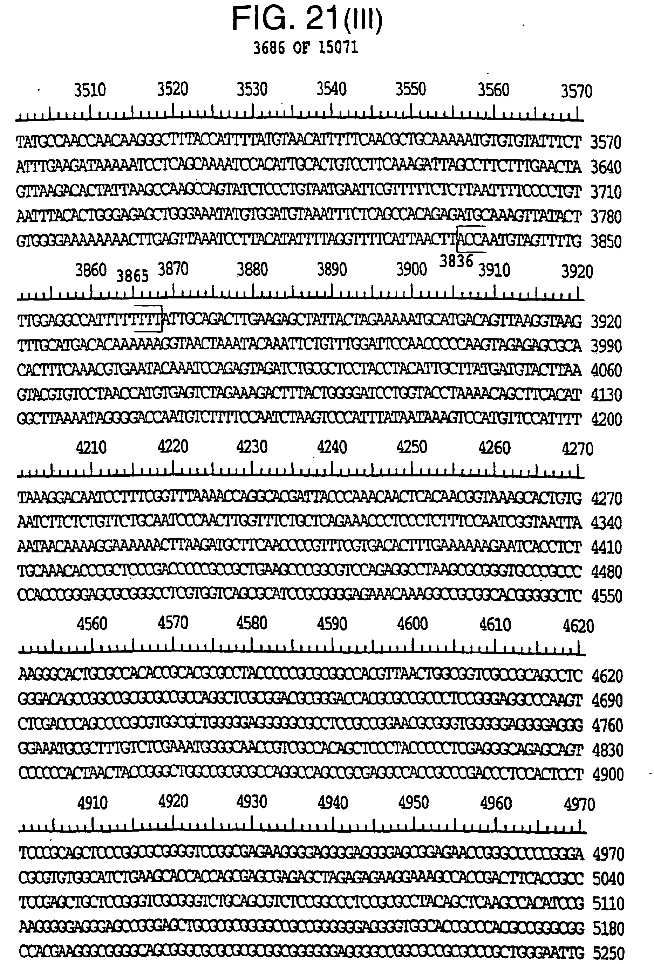

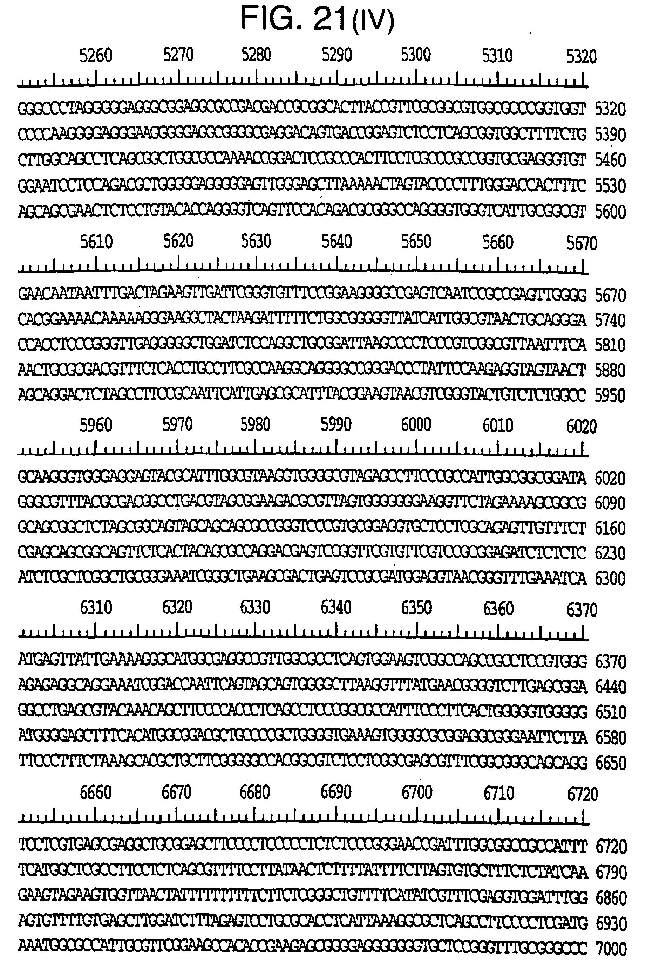

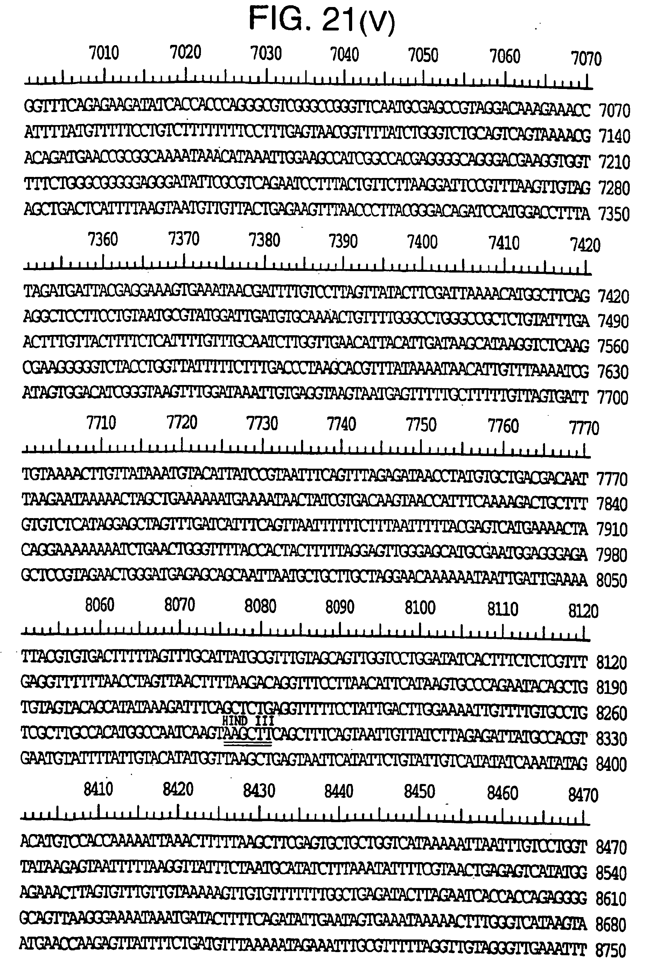

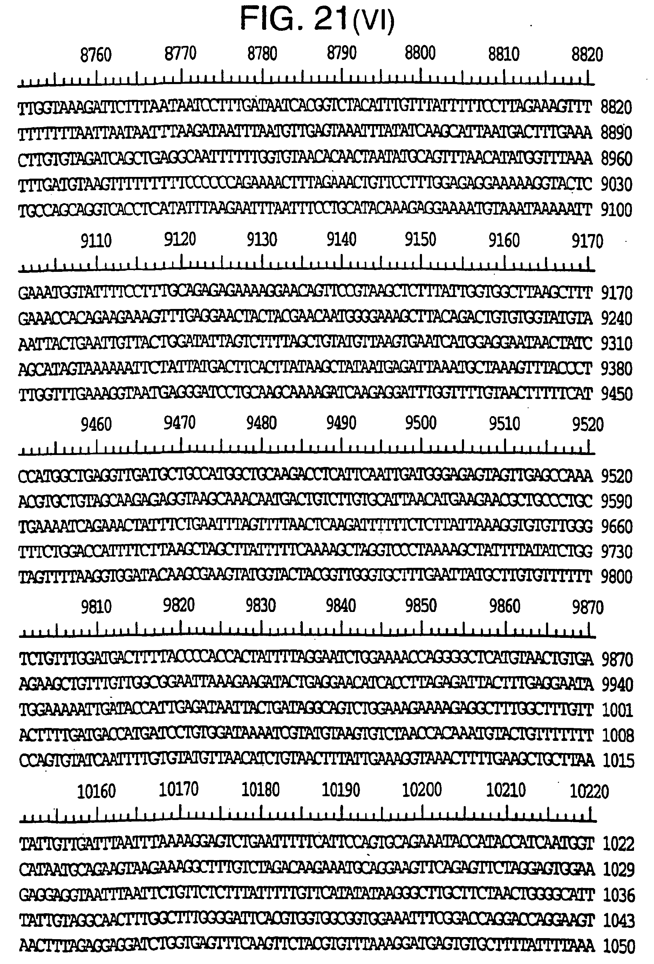

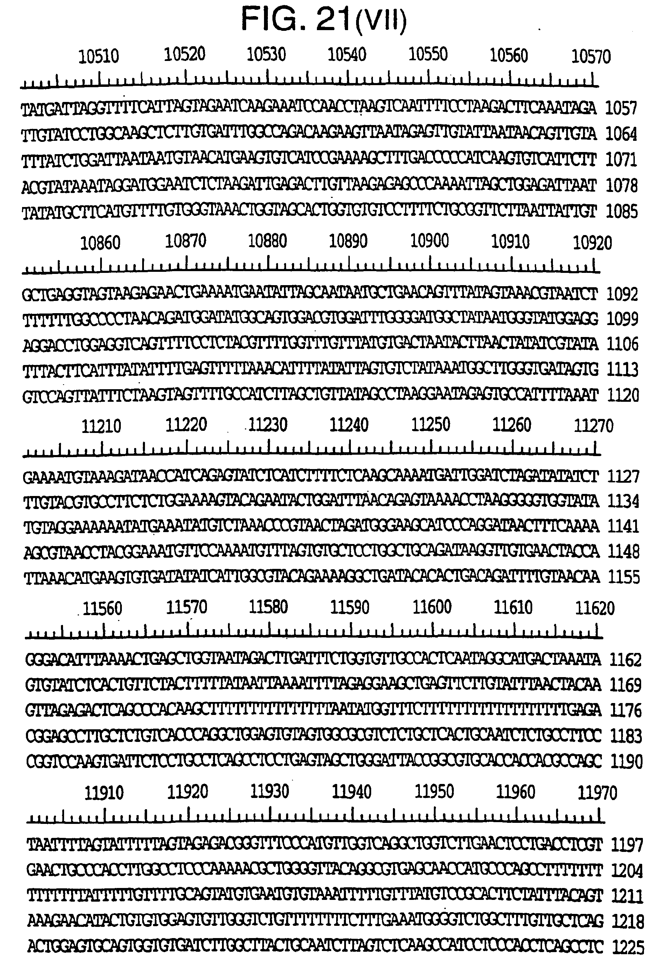

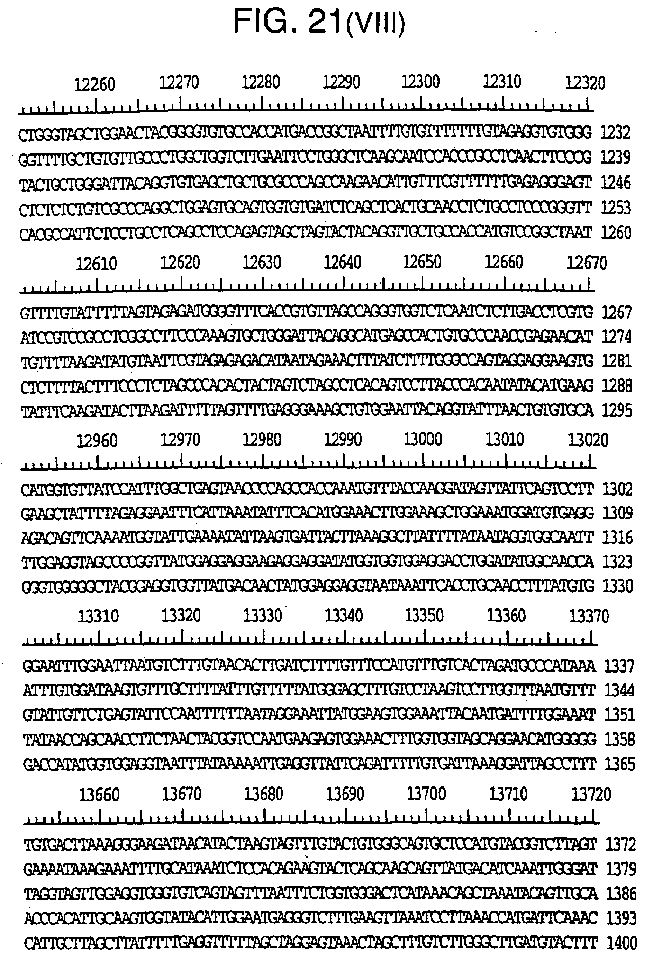

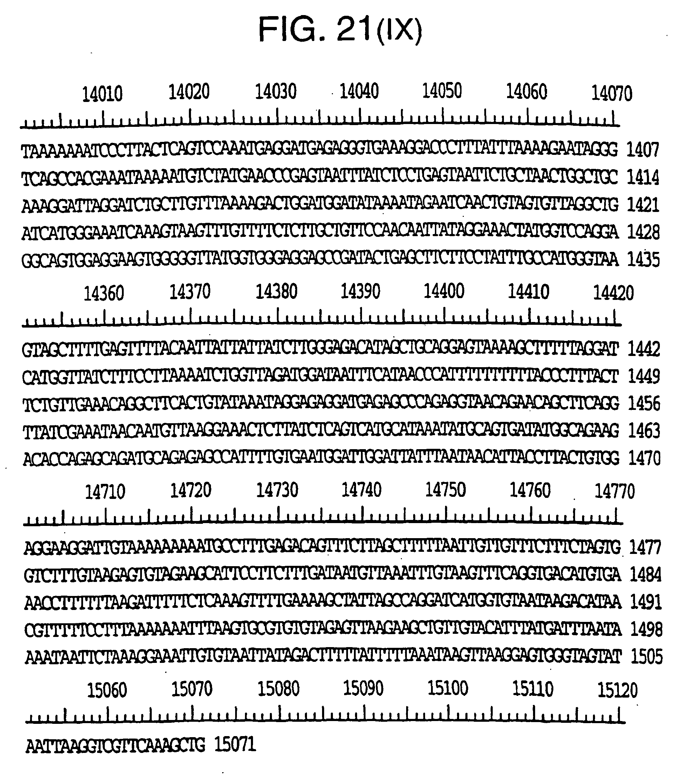

Preferably the UCOE is a 60kb DNA fragment spanning the human hnRNP A2 gene with 30kb 5' flanking sequence and 20kb 3' flanking sequence, or a functional homologue or fragment thereof. In a further preferred embodiment, the UCOE comprises the sequence of Figure 21 between nucleotides 1 to 6264 or a functional homologue or fragment thereof. This sequence encompasses the hnRNP A2 promoter (nucleotides 5636 to 6264) and 5.5kb 5' flanking sequence comprising the HP1H-γ promoter.

Preferably the UCOE is a 25kb DNA fragment spanning the human TBP gene with 1kb 5' and 5 kb 3'flanking sequence, or a functional homologue or fragment thereof.

Preferably the UCOE is a 16kb DNA fragment spanning the human hnRNP A2 gene with 5kb 5' and 1.5kb 3' flanking sequence, or a functional homologue or fragment thereof.

Preferably the UCOE. comprises the sequence of Figure 21 between nucleotides 1 and 5636 (the 5.5kb 5' flanking sequence of the hnRNP A2 promoter) and the CMV promoter or a functional homologue or fragment thereof.

Preferably the UCOE comprises the sequence of Figure 21 between nucleotides 4102 and 8286 or a functional homologue or fragment thereof. This sequence encompasses both the hnRNP A2 and HP1H-γ promoters.

Preferably the UCOE comprises the sequence of Figure 21 between nucleotides 1 and 7627 or a functional homologue or fragment thereof. This sequence encompasses both the hnRNP A2 and HP1H-γ promoters and exon 1 of the hnRNP A2 gene.

Preferably the UCOE comprises the sequence of Figure 21 between nucleotides 1 and 9127 or a functional homologue or fragment thereof. This sequence encompasses both the hnRNP A2 and HP1H-γ promoters and the 3' flanking sequence of the hnRNP A2 promoter up to but not including exon 2 of the hnRNP A2 gene.

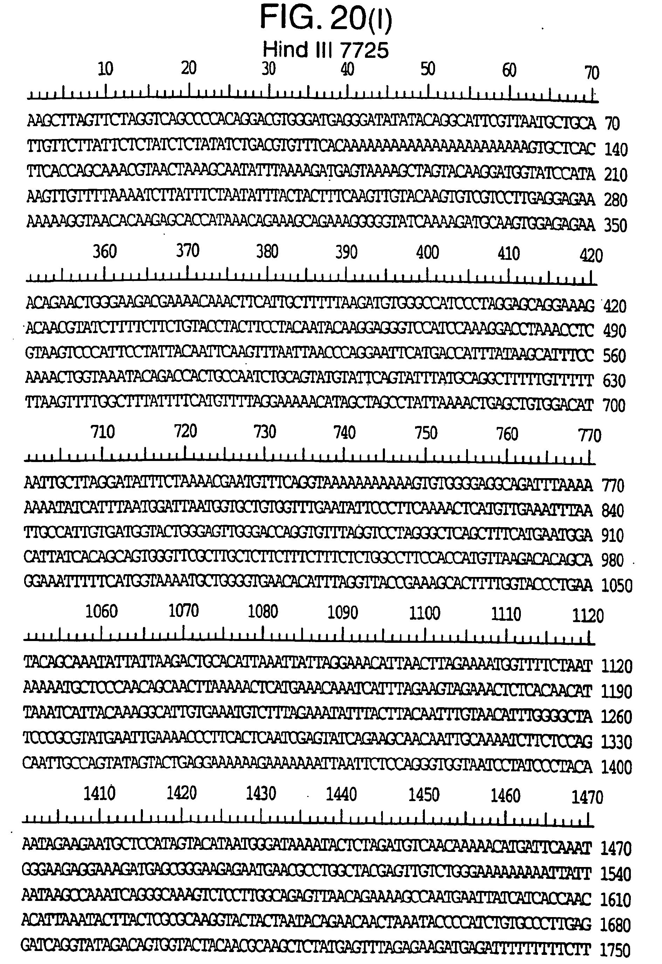

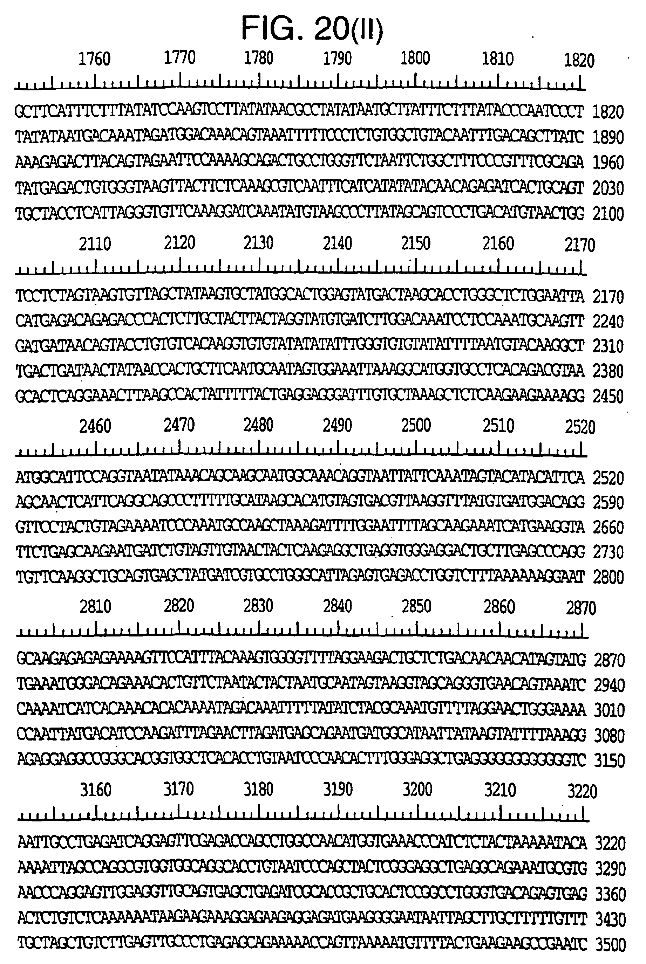

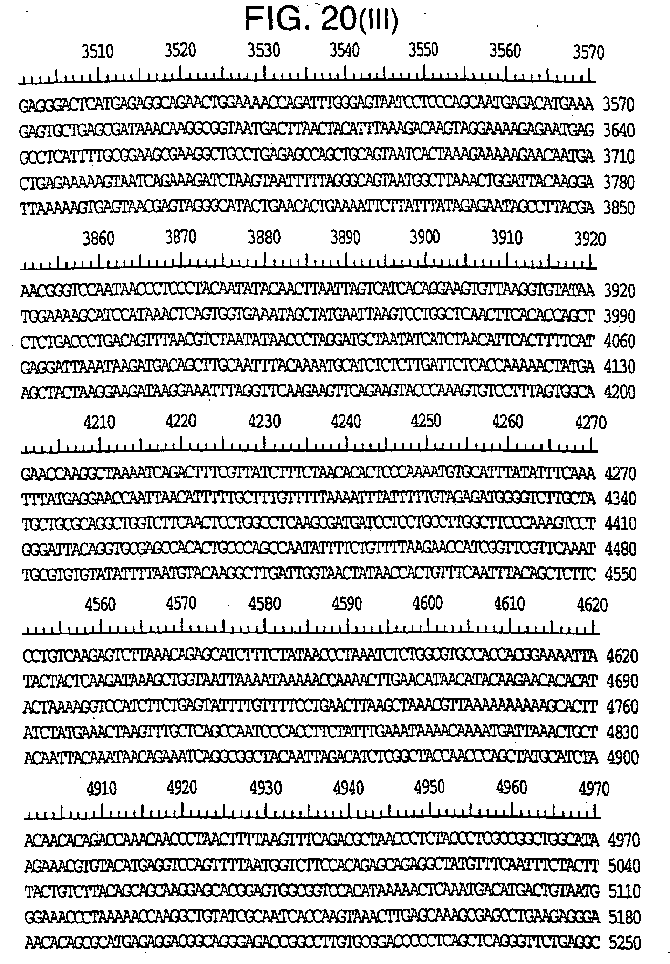

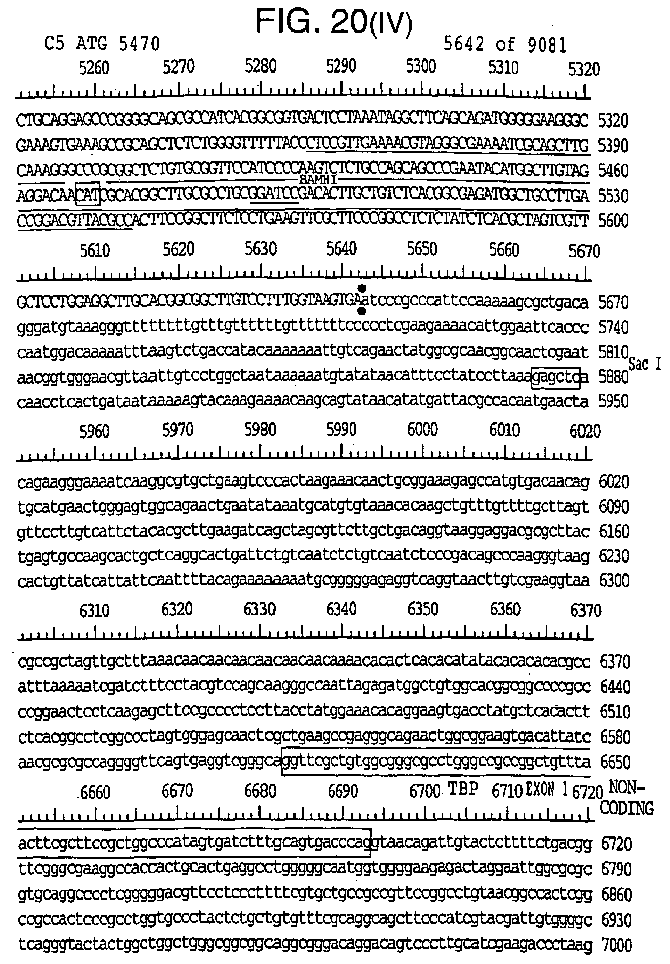

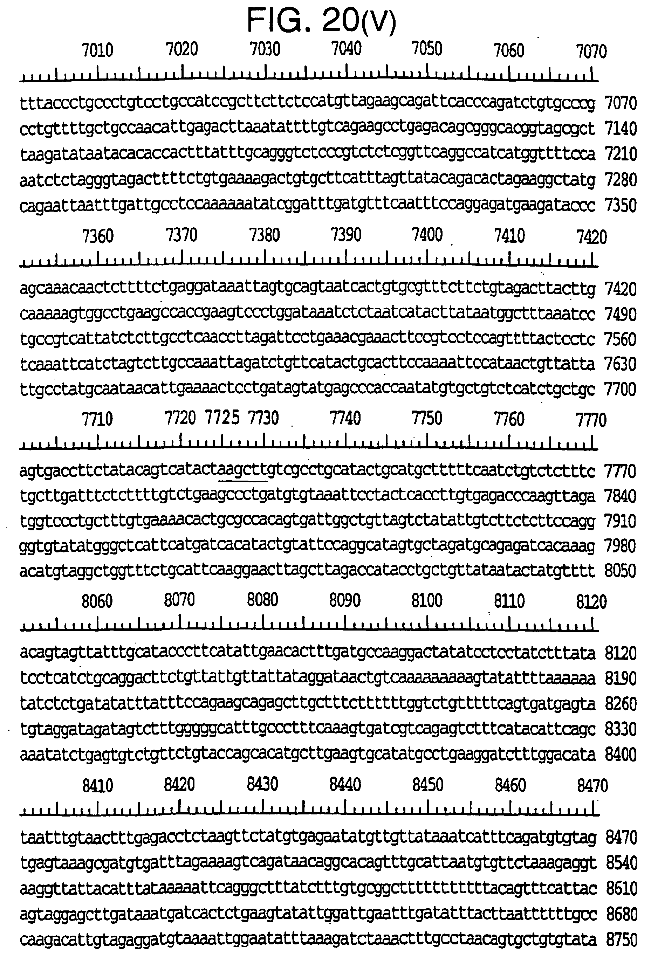

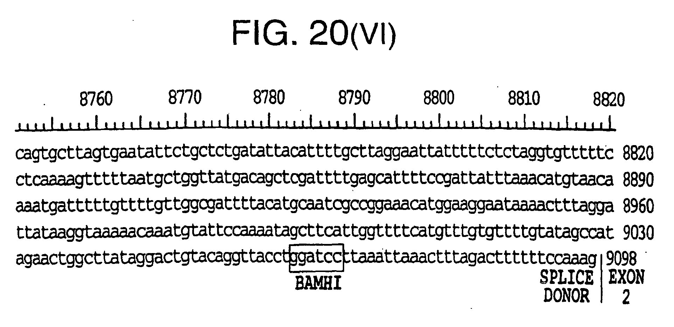

It is further preferred that the UCOE has the nucleotide sequence of Figure 20 or Figure 21, or a functional fragment or homologue thereof.

The term "functional homologues or fragments" as used herein means homologues or fragments, which open chromatin or maintain chromatin in an open state and facilitate reproducible expression of an operably-linked gene. Preferably, the homologues are species homologues corresponding to the identified UCOEs or are homologues associated with other ubiquitously expressed genes. Sequence comparisons can be made between UCOEs in order to identify conserved sequence motifs enabling the identification or synthesis of other UCOEs. Suitable software packages for performing such sequence comparisons are well known to those skilled in the art. A preferred software package for performing sequence comparisons is PCGENE (Intelligenetics, Inc. USA). Functional fragments can be easily identified by methodically generating fragments of known UCOEs and testing for function. The identification of conserved sequence motifs will also assist in the identification of functional fragments, as fragments comprising the conserved sequence motifs will be likely to be functional. Functional homologues also encompass modified UCOEs wherein elements of the UCOE have been replaced by similar elements, such as replacing one or more promoters of a UCOE with different heterologous promoters. As indicated above, the heterologous promoter may be any promoter including tissue specific promoters such as tumour-specific promoters and ubiquitous promoters. Preferably the heterologous promoter is a strong and/or substantially ubiquitous promoter and most preferably is the CMV promoter.

Further, there is provided a method for identifying a UCOE which facilitates reproducible expression of an operably-linked gene in cells of at least two different tissue types, comprising:
1. testing a candidate UCOE by transfecting cells of at least two different tissue types with a vector containing the candidate UCOE operably-linked to a marker gene; and
2. determining if reproducible expression of the marker gene is obtained in the cells of two or more different tissue types.

Preferably, the method for identifying a UCOE comprises the additional step of selecting candidate UCOEs that are associated with one or more of: a ubiquitously expressed gene, a dual or bi-directional promoter and an extended methylation-free CpG-island.

Preferably, reproducible expression of the marker gene is determined in cells containing a single copy of the UCOE linked to the marker gene.

Further disclosed herein is a method wherein the candidate UCOE is tested by generating a non-human transgenic animal containing cells comprising a vector containing the candidate UCOE operably-linked to a marker gene and determining if reproducible expression of the marker gene is obtained in the cells of two or more different tissue types. Preferably, the non-human transgenic animal is a F1, or greater, generation non-human transgenic animal. Preferably the non-human transgenic animal is a rodent, more preferably a mouse.

Provided herein is a UCOE derivable from a nucleic acid sequence associated with or adjacent to a ubiquitously expressed gene. Preferably, the nucleic acid sequence comprises an extended methylation-free, CpGr-island. It is further preferred that the nucleic acid sequence comprises at least one transcription factor binding site. Preferably, the nucleic acid sequence comprises dual or bi-directional promoters that are divergently transcribed. Preferably, the nucleic acid sequence comprises dual promoters that are divergently transcribed. Preferably, the nucleic acid sequence comprises dual or bidirectional promoters that are divergently transcribed and which are associated with ubiquitously expressed genes. Preferably, the nucleic acid sequence comprises dual promoters that are divergently transcribed and which are associated with ubiquitously expressed genes.

Also provided is the use of the polynucleotide as disclosed herein, or a fragment thereof, in an assay for identifying other UCOEs. Preferably, a fragment of the polynucleotide is used which encompasses a conserved sequence or structural motif. Methods for performing such an assay are well known to those skilled in the art.

Also provided is a vector comprising the polynucleotide as disclosed herein .The vector preferably comprises an expressible gene operably-linked to the polynucleotide. The expressible gene comprises the necessary elements enabling gene expression such as suitable promoters, enhancers, splice acceptor sequences, internal ribosome entry site sequences (IRES) and transcription stop sites. Suitable elements for enabling gene expression are well known to those skilled in the art. The suitable elements for enabling gene expression can be the natural endogenous elements associated with the gene or may be heterologous elements used in order to obtain a different level or tissue distribution of gene expression compared to the endogenous gene. Preferably, the vector comprises a promoter operably associated with the expressible gene and the polynucleotide. The promoter may be a natural endogenous promoter of the expressible gene or may be a heterologous promoter. The heterologous promoter may be any promoter including tissue specific promoters such as tumour-specific promoters and ubiquitous promoters.

Preferably the heterologous promoter is a strong and/or a substantially ubiquitous promoter and most preferably is the CMV promoter.

The vector may be any vector capable of transferring DNA to a cell. Preferably, the vector is an integrating vector or an episomal vector.

Preferred integrating vectors include recombinant retroviral vectors. A recombinant retroviral vector will include DNA of at least a portion of a retroviral genome which portion is capable of infecting the target cells. The term "infection" is used to mean the process by which a virus transfers genetic material to its host or target cell. Preferably, the retrovirus used in the construction of a vector is also rendered replication-defective to remove the effect of viral replication of the target cells. In such cases, the replication-defective viral genome can be packaged by a helper virus in accordance with conventional techniques. Generally, any retrovirus meeting the above criteria of infectiousness and capability of functional gene transfer can be employed.

Suitable retroviral vectors include but are not limited to pLJ, pZip, pWe and pEM, well known to those of skill in the art. Suitable packaging virus lines for replication-defective retroviruses include, for example, ΨCrip, ΨCre, Ψ2 and ΨAm.

Other useful vectors include adenovirus, adeno-associated virus, SV40 virus, vaccinia virus, HSV and pox virusvectors. A preferred vector is the adenovirus. Adenovirus vectors are well known to those skilled in the art and have been used to deliver genes to numerous cell types, including airway epithelium, skeletal muscle, liver, brain and skin (Hitt, MM, Addison CL and Graham, FL (1997) Human adenovirus vectors for gene transfer into mammalian cells. Advances in Pharmacology 40: 137-206; and Anderson WF (1998) Human gene therapy. Nature 392 (6679 Suppl): 25-30).

A further preferred vector is the adeno-associated (AAV) vector. AAV vectors are well known to those skilled in the art and have been used to stably transduce human T-lymphocytes, fibroblasts, nasal polyp, skeletal muscle, brain, erythroid and heamopoietic stem cells for gene therapy applications (Philip et al., 1994, Mol. Cell, Biol., 14, 2411-2418; Russell et al., 1994, PNAS USA, 91, 8915-8919; Flotte et al., 1993, PNAS USA, 90, 10613-10617; Walsh et al., 1994, PNAS USA, 89, 7257-7261; Miller et al., 1994, PNAS USA, 91, 10183-10187; Emerson, 1996, Blood, 87, 3082-3088). International Patent Application WO 91/18088 describes specific AAV based vectors.

Preferred episomal vectors include transient non-replicating episomal vectors and self-replicating episomal vectors with functions derived from viral origins of replication such as those from EBV, human papovavirus (BK) and BPV-1. Such integrating and episomal vectors are well known to those skilled in the art and are fully described in the body of literature well known to those skilled in the art. In particular, suitable episomal vectors are described in WO98/07876.

Mammalian artificial chromosomes are also preferred vectors The use of mammalian artificial chromosomes is discussed by Calos (1996, TIG, 12, 463-466).

Preferably the vector is a plasmid. It is further preferred that the plasmid is a non-replicating, non-integrating plasmid.

The term "plasmid" as used herein refers to any nucleic acid encoding an expressible gene and includes linear or circular nucleic acids and double or single stranded nucleic acids. The nucleic acid can be DNA or RNA and may comprise modified nucleotides or ribonucleotides, and may be chemically modified by such means as methylation or the inclusion of protecting groups or cap- or tail structures.

A non-replicating, non-integrating plasmid is a nucleic acid which when transfected into a host cell does not replicate and does not specifically integrate into the host cell's genome (i.e. does not integrate at high frequencies and does not integrate at specific sites).

Replicating plasmids can be identified using standard assays including the standard replication assay of Ustav et al., EMBO J., 10, 449-457, 1991.

Preferably, a non-replicating, non-integrating plasmid is a plasmid that cannot be stably maintained in cells, independently of genomic DNA replication, and which does not persist in progeny cells for three or more cell divisions without a significant loss in copy number of the plasmid in the cells, i.e., with a loss of greater than an average of about 50% of the plasmid molecules in progeny cells between a given cell division. Generally, in self-replicating vectors, the self-replicating function is provided by using a viral origin of replication and providing one or more viral replication factors that are required for replication mediated by that particular viral origin. Self-replicating vectors are described in WO 98/07876. The term "transiently transfecting, non-integrating plasmid" herein means the same as the term "non-replicating, non-integrating plasmid" as defined above.

Preferably the plasmid is a naked nucleic acid. As used herein, the term "naked" refers to a nucleic acid molecule that is free of direct physical associations with proteins, lipids, carbohydrates or proteoglycans, whether covalently or through hydrogen bonding. The term does not refer to the presence or absence of modified nucleotides or ribonucleotides, or chemical modification of the all or a portion of a nucleic acid molecule by such means as methylation or the inclusion of protecting groups or cap- or tail structures.

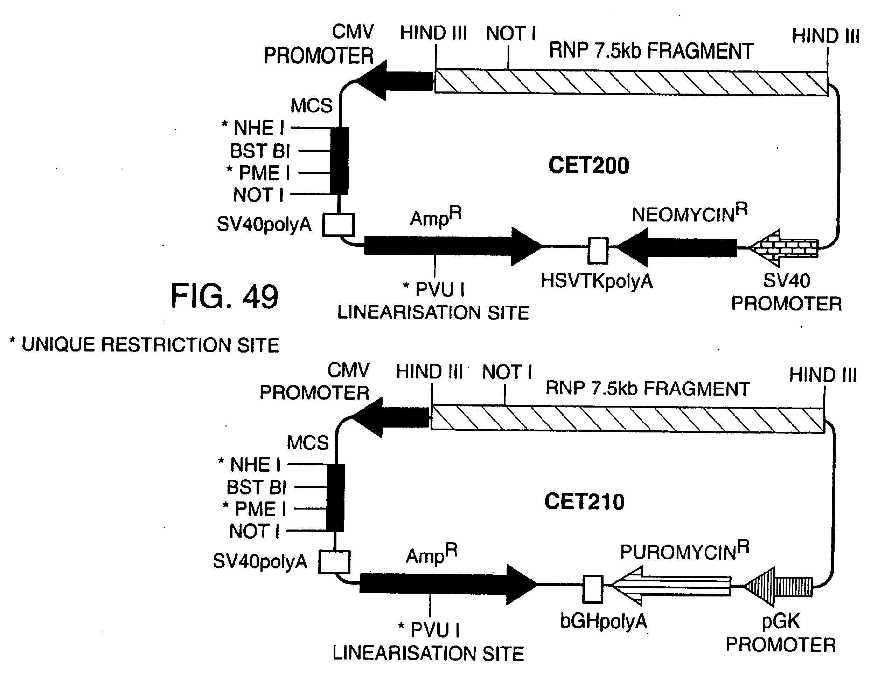

Preferably, the vector comprises the sequence of Figure 20 between nucleotides 1 and 7627 (encompassing both the hnRNP A2 and HP1H-γ promoters), the CMV promoter, a multiple cloning site, a polyadenylation sequence and genes encoding selectable markers under suitable control elements. Preferably the vector is the CET200 or the CET210 vector schematically shown in Figure 49.

Also provided is a host cell transfected with the vector as disclosed herein . The host cell may be any cell such as yeast cells, insect cells, bacterial cells and mammalian cells. Preferably the host cell is a mammalian cell and may be derived from mammalian cell lines such as the CHO cell line, the 293 cell line and NS0 cells.

Preferably, the operably-linked gene is a therapeutic nucleic acid sequence. Therapeutically useful nucleic acid sequences, which may be used include sequences encoding receptors, enzymes, ligands, regulatory factors, hormones, antibodies or antibody fragments and structural proteins. Therapeutic nucleic acid sequences also include sequences encoding nuclear proteins, cytoplasmic proteins, mitochondrial proteins, secreted proteins, membrane-associated proteins, serum proteins, viral antigens, bacterial antigens, protozoal antigens and parasitic antigens. Nucleic acid sequences useful also include sequences encoding proteins, peptides, lipoproteins, glycoproteins, phosphoproteins and nucleic acid (e.g., RNAs or antisense nucleic acids). Proteins or polypeptides which can be encoded by the therapeutic nucleic acid sequence include hormones, growth factors, enzymes, clotting factors, apolipoproteins, receptors, erythropoietin, therapeutic antibodies or fragments thereof, drugs, oncogenes, tumor antigens, tumor suppressors, viral antigens, parasitic antigens and bacterial antigens. Specific examples of these compounds include proinsulin, growth hormone, androgen receptors, insulin-like growth factor I, insulin-like growth factor II, insulin-like growth factor binding proteins, epidermal growth factor, transforming growth factor-α, transforming growth factor-β, platelet-derived growth factor, angiogenesis factors (acidic fibroblast growth factor, basic fibroblast growth factor, vascular endothelial growth factor and angiogenin), matrix proteins (Type IV collagen, Type VII collagen, laminin), phenylalanine hydroxylase, tyrosine hydroxylase, oncoproteins (for example, those encoded byras, fos, myc, erb, src, neu, sis, jun), HPV E6 or E7 oncoproteins, p53 protein, Rb protein, cytokine receptors, IL-1, IL-6, IL-8, , and proteins from viral, bacterial and parasitic organisms which can be used to induce an immunological response, and other proteins of useful significance in the body. The choice of gene, to be incorporated, is only limited by the availability of the nucleic acid sequence encoding it. One skilled in the art will readily recognise that as more proteins and polypeptides become identified they can be integrated into the polynucleotide disclosed herein and expressed.

When the polynucleotide disclosed herein is comprised in a plasmid, it is preferred that the plasmid be used in monogenic gene therapy such as in the treatment of Duchenne muscular dystrophy and in DNA vaccination and immunisation methods.

The polynucleotide disclosed herein may be used to express genes that are already expressed in a host cell (i.e., a native or homologous gene), for example, to increase the dosage of the gene product. It should be noted, however, that expression of a homologous gene might result in deregulated expression, which may not be subject to control by the UCOE due to its over-expression in the cell.

The polynucleotide disclosed herein may be inserted into the genome of a cell in a position operably associated with an endogenous (native) gene and thereby lead to increased expression of the endogenous gene. Methods for inserting elements into the genome at specific sites are well known to those skilled in the art and are described in US-A-5,578,461 and US-A-5,641,670. Alternatively, the polynucteotide herein disclosed in its endogenous (native) position on the genome may have a gene inserted in an operably associated position so that expression of the gene occurs. Again, methods for inserting genes into the genome at specific sites are well known to those skilled in the art and are described in US-A-5,578,461 and US-A-5,641,670.

Also provided is the use of the polynucleotide disclosed herein to increase the expression of an endogenous gene comprising inserting the polynucleotide into the genome of a cell in a position operably associated with the endogenous gene thereby increasing the level of expression of the gene.

Numerous techniques are known and are useful for delivering the vectors described herein to cells, including the use of nucleic acid condensing agents, electroporation, complexation with asbestos, polybrene, DEAE cellulose, Dextran, liposomes, cationic liposomes, lipopolyamines, polyornithine, particle bombardment and direct microinjection (reviewed by Kucherlapati and Skoultchi, Crit. Rev. Biochem. 16:349-379 (1984); Keown et al., Methods Enzymol. 185:527 (1990)).

A vector may be delivered to a host cell non-specifically or specifically (i.e., to a designated subset of host cells) via a viral or non-viral means of delivery. Preferred delivery methods of viral origin include viral particle-producing packaging cell lines as transfection recipients for the vector herein disclosed into which viral packaging signals have been engineered, such as those of adenovirus, herpes viruses and papovaviruses. Preferred non-viral based gene delivery means and methods may also be used and include direct naked nucleic acid injection, nucleic acid condensing peptides and non-peptides, cationic liposomes and encapsulation in liposomes.

The direct delivery of vector into tissue has been described and some short term gene expression has been achieved. Direct delivery of vector into muscle (Wolff et al., Science, 247, 1465-1468, 1990) thyroid (Sykes et al., Human Gene Ther., 5, 837-844, 1994) melanoma (Vile et al., Cancer Res., 53, 962-967, 1993), skin (Hengge et al., Nature Genet, 10, 161-166, 1995), liver (Hickman et al., Human Gene Therapy, 5, 1477-1483, 1994) and after exposure of airway epithelium (Meyer et al., Gene Therapy, 2, 450-460, 1995) is clearly described in the prior art.

Various peptides derived from the amino acid sequences of viral envelope proteins have been used in gene transfer when co-administered with polylysine DNA complexes (Plank et al., J. Biol. Chem. 269:12918-12924 (1994));. Trubetskoy et al., Bioconjugate Chem. 3:323-327 (1992); WO 91/17773; WO 92/19287; and Mack et al., Am. J. Med. Sci. 307:138-143 (1994)) suggest that co-condensation of polylysine conjugates with cationic lipids can lead to improvement in gene transfer efficiency. International Patent Application WO 95/02698 discloses the use of viral components to attempt to increase the efficiency of cationic lipid gene transfer.

Useful nucleic acid condensing agents include spermine, spermine derivatives, histones, cationic peptides, cationic non-peptides such as polyethyleneimine (PEI) and polylysine. Spermine derivatives refers to analogues and derivatives of spermine and include compounds as set forth in International Patent Application. WO 93/18759 (published September 30,1993).

Disulphide bonds have been used to link the peptidic components of a delivery vehicle (Cotten et al., Meth. Enzymol. 217:618-644 (1992)); see also, Trubetskoy *et al.* (*supra*).

Delivery vehicles for delivery of DNA constructs to cells are known in the art and include DNA/poly-cation complexes which are specific for a cell surface receptor, as described in, for example, Wu and Wu, J. Biol. Chem. 263:14621 (1988); Wilson et al., J. Biol. Chem. 267:963-967 (1992); and U.S. Patent No. 5,166,320).

Delivery of a vector is contemplated using nucleic acid condensing peptides. Nucleic acid condensing peptides, which are particularly useful for condensing the vector and delivering the vector to a cell, are described in WO 96/41606. Functional groups may be bound to peptides useful for delivery of a vector as described in WO 96/41606. These functional groups may include a ligand that targets a specific cell-type such as a monoclonal antibody, insulin, transferrin, asialoglycoprotein, or a sugar. The ligand thus may target cells in a non-specific manner or in a specific manner that is restricted with respect to cell type.

The functional groups also may comprise a lipid, such as palmitoyl, oleyl, or stearoyl; a neutral hydrophilic polymer such as polyethylene glycol (PEG), or polyvinylpyrrolidine (PVP); a fusogenic peptide such as the HA peptide of influenza virus; or a recombinase or an integrase. The functional group also may comprise an intracellular trafficking protein such as a nuclear localisation sequence (NLS) and endosome escape signal or a signal directing a protein directly to the cytoplasm.

Also provided is the polynucleotide, vector or host cell disclosed herein for use in therapy.

Preferably, the polynucleotide, vector or host cell is used in gene therapy.

Also provided is the use of the polynucleotide, vector or host cell as herein disclosed in the manufacture of a composition for use in gene therapy.

Also provided is a method of treatment, comprising administering to a patient in need of such treatment an effective dose of the polynucleotide, vector or host cell as disclosed herein. Preferably, the patient is suffering from a disease treatable by gene therapy.

Also provided is a pharmaceutical composition comprising the polynucleotide, vector or host cell as disclosed herein in combination with a pharmaceutically acceptable recipient.

Also provided is use of a polynucleotide, vector or host cell as herein disclosed in a cell culture system in order to obtain the desired gene product. Suitable cell culture systems are well known to those skilled in the art and are fully described in the body of literature known to those skilled in the art.

Also provided is the use of the polynucleotide as herein disclosed in producing transgenic plant genetics. The generation of transgenic plants which have increased yield, resistance, etc. are well known to those skilled in the art. Also provided is a transgenic plant containing cells which contain the polynucleotide as herein disclosed

Also provided is a transgenic non-human animal containing cells, which contain the polynucleotide herein disclosed.

The pharmaceutical compositions disclosed herein may comprise the polynucleotide, vector or host cell as herein described if desired, in admixture with a pharmaceutically acceptable carrier or diluent, for therapy to treat a disease or provide the cells of a particular tissue with an advantageous protein or function.

The polynucleotide, vector or host cell as herein disclosed or the pharmaceutical composition may be administered via a route which includes systemic intramuscular, intravenous, aerosol, oral (solid or liquid form), topical, ocular, as a suppository, intraperitoneal and/or intrathecal and local direct injection.

The exact dosage regime will, of course, need to be determined by individual clinicians for individual patients and this, in turn, will be controlled by the exact nature of the protein expressed by the gene of interest and the type of tissue that is being targeted for treatment.

The dosage also will depend upon the disease indication and the route of administration. Advantageously, the duration of treatment will generally be continuous or until the cells die. The number of doses will depend upon the disease, and efficacy data from clinical trials.

The amount of polynucleotide or vector DNA delivered for effective gene therapy will preferably be in the range of between about 50 ng -1000 µg of vector DNA/kg body weight; and more preferably in the range of between about 1-100 µg vector DNA/kg.

Although it is preferred to administer the polynucleotide, vector or host cell to a mammal for *in vivo* cell uptake, an *ex vivo* approach may be utilised whereby cells are removed from an animal, transduced with the polynucleotide or vector, and then re-implanted into the animal. The liver, for example, can be accessed by an *ex vivo* approach by removing hepatocytes from an animal, transducing the hepatocytes *in vitro* and re-implanting the transduced hepatocytes into the animal (e.g., as described for rabbits by Chowdhury et a/., Science 254:1802-1805, 1991, or in humans by Wilson, Hum. Gene Tlrer. 3:179-222, 1992). Such methods also may be effective for delivery to various populations of cells in the circulatory or lymphatic systems, such as erythrocytes, T cells, B cells and haematopoietic stem cells.

Also provided is a mammalian model for determining the tissue-specificity and/or efficacy of gene therapy using the polynucleotide, vector or host cell as herein disclosed . The mammalian model comprises a non-human transgenic animal whose cells contain the vector as herein disclosed. Methods of making transgenic mice (Gordon et al., Proc. Natl. Acad. Sci. USA 77:7380 (1980); Harbers et al., Nature 293:540 (1981); Wagner et al., Proc. Natl. Acad. Sci. USA 78:5016 (1981); and Wagner et al., Proc. Natl. Acad. Sci. USA 78:6376 (1981), sheep, pigs, chickens (see Hammer et al., Nature 315:680 (1985)), etc., are well-known in the art and are contemplated for use . Such animals permit testing prior to clinical trials in humans.

Non-human transgenic animals containing the polynucleotide as herein disclosed also may be used for long-term production of a protein of interest.

Also provided herein is use of the polynucleotide as herein disclosed in functional genomics applications. Functional genomics relates principally to the sequencing of genes specifically expressed in particular cell types or disease states and now provides thousands of novel gene sequences of potential interest for drug discovery or gene therapy purposes. The major problem in using this information for the development of novel therapies lies in how to determine the functions of these genes. UCOEs can be used in a number of functional genomic applications in order to determine the function of gene sequences. The functional genomic applications herein disclosed include, but are not limted to:
(1) Using the polynucleotide herein disclosed to achieve sustained expression of anti-sense versions of the gene sequences or ribozyme knockdown libaries, thereby determining the effects of inactivating the gene on cell phenotype.
(2) Using the polynucleotide herein disclosed to prepare expression libraries for the gene sequences, such that delivery into cells will result in reliable, reproducible, sustained expression of the gene sequences. The resulting cells, expressing the gene sequences can be used in a variety of approaches to function determination and drug discovery. For example, raising antibodies to the gene product for neutralisation of its activity; rapid purification of the protein product of the gene itself for use in structural, functional or drug screening studies ; or in cell-based drug screening.
(3) Using the polynucleotide herein disclosed in approaches involving mouse embryonic stem (ES) cells and transgenic mice. One of the most powerful functional genomics approaches involves random insertion into genes in mouse ES cells of constructs which only allow drug selection following insertion into expressed genes, and which can readily be rescued for sequencing (G.Hicks et al., Nature Genetics, 16, 338-334). Transgenic mice with knockout mutations in genes with novel sequences can then readily be made to probe their function. At present this technology works well for the 10% of mouse genes which are well expressed in mouse ES cells. Incorporation of UCOEs into the integrating constructs will enable this technique to be extended to identify all genes expressed in mice.

The following examples, with reference to the figures, are offered by way of illustration. The preparation, testing and analysis of several representative polynucleotides are described in detail below. One of skill in the art may adapt these procedures for preparation and testing of other polynucleotides.

The figures show:

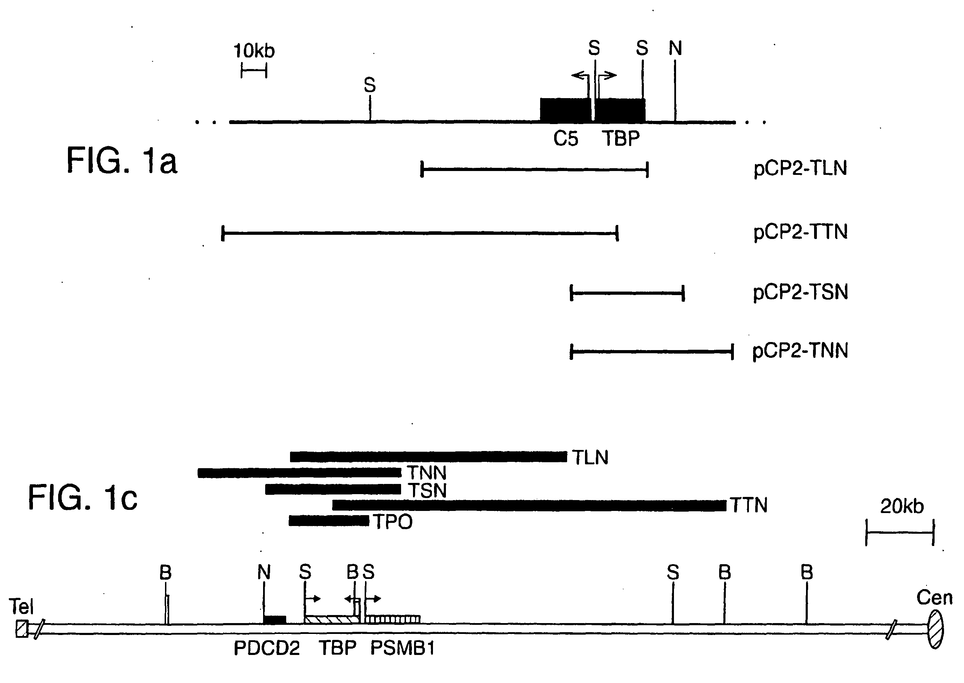

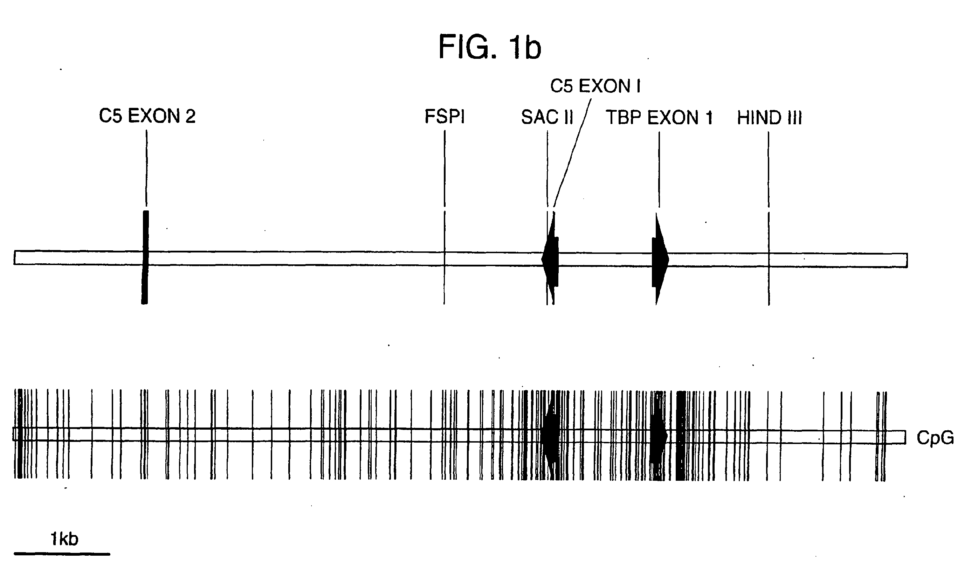

Figure 1 shows the human TBP gene locus.
   A: Schematic representation of the pCYPAC-2 clones containing the human TBP gene used in this study. The positions of *Not*I and *Sac*II restriction sites that may indicate the positions of unidentified genes are marked.
   B: Illustration of the CpG-island spanning the 5' TBP/C5 regions. The density of CpG di-nucleotide residues implies that the methylation-free island is 3.4kb in length and extends between the *Fsp*I site within intron I of C5, and the *Hind*III site within the first intron of TBP.
   C: Is a further schematic representation of the clones from the TBP/C5 region. The arrangement of the genes has been reversed from that given in Figure 1A. Please note, the C5 gene is also referred to as the PSMB1 gene. A 257 kb contiguous region from the telomere of chromosome 6q with positions of the 3 closely linked genes and relevant restriction sites is shown (B, *Bss*HII; N, *Not*I; S, *Sac*II). PAC clones with their designated names are indicated. The subclone pBL3-TPO-puro is also shown. The distance between the *Not*I site within the first exon of PDCD2 and the beginning of the telomeric repeat is approximately 150 kb.

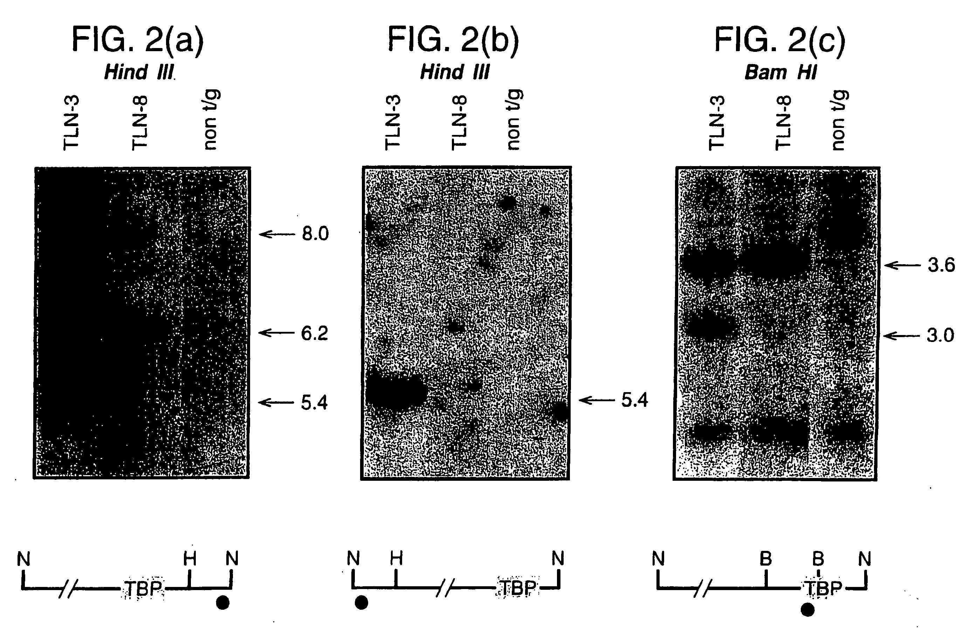

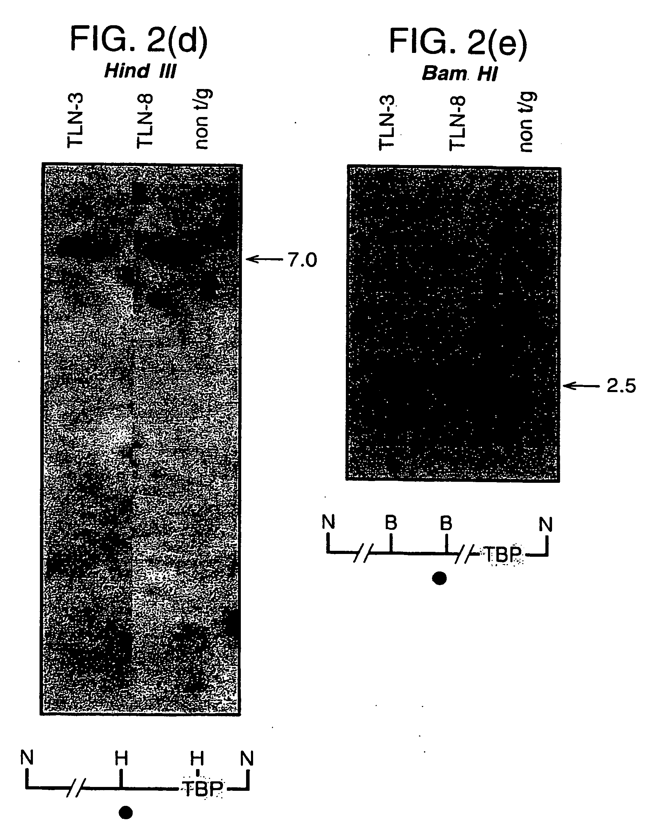

Figure 2 shows end-fragment analysis of TLN:3 and TLN:8 transgenic mice. Southern blot analysis of transgenic mouse tail biopsy DNA samples were probed with small DNA fragments located at (a) the 3' end of the transgene, (b) the 5' end, (c) the promoter, (d) -7.7 kb from TBP mRNA CAP site, (e) -12kb from TBP mRNA CAP site. The results for TLN:3 (a,b) show that there is only one hybridising band with both end-probes, which does not match the predicted size for any head-to-head, head-to-tail, or tail-to-tail concatamer. Thus it would appear that there is only one transgene copy in this line. However, panel (c) shows that with a promoter probe, two bands are seen indicating that there must also be a second, deleted copy of the transgene present in this line. TLN:8 analysis in (a) shows a transgene concatamer band at 6kb and an end fragment band at 7.8kb. As the concatamer band is twice the intensity of the end fragment, this indicates a copy number of three for this line. The lack of hybridisation in (b) suggests a deletion at the 5' end of all three copies has occurred and work is in progress to map this. Panels (d) and (e) indicate that the transgenes appear to be intact up to 12kbp 5' to the TBP gene.

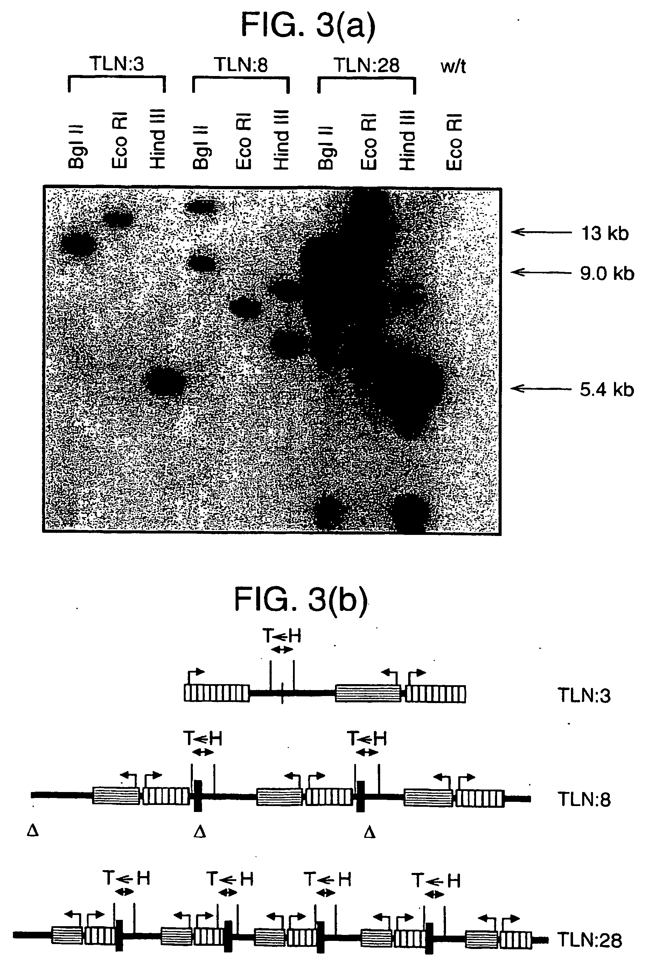

Figure 3A shows the analysis of TLN:28 mice. Southern blots of TLN:28 DNA were hybridised to a probe located at the very 3' end of the transgene locus. Multiple bands were seen to hybridise to this probe, suggesting multiple integration events. However, an intense concatamer band is seen in the position expected for a head to tail integration event. Comparison of the signal intensities between this and the end-fragments suggested a copy number of approximately 4 in this line.
Figure 3B shows a summary of transgene organisation in TLN mouse lines. TLN:3: contains two copies of the transgene in a head to tail arrangement. A deletion has occurred at both the 5' and 3' ends of this array. The 5' deletion extends into the 5' flanking region of TBP, completely deleting the C5 gene in this copy. At the 3' end, the deletion extends into the 3'UTR of TBP, leaving the C5 gene intact. This animal, therefore, possesses a single copy of the C5 gene and a single functional copy of the TBP gene. Tan:8: contains a heat to tail arrangement of three copies. Each copy would seem to possess a deletion at the very 5' region, although the extent of this deletion is not known at present, it does not extend to the C5 gene as human C5 mRNA is detected in this line. TLN:28: contain 5 copies in a head to tail configuration, but there are also a number of additional fragments seen, indicating that this array may be more complex.

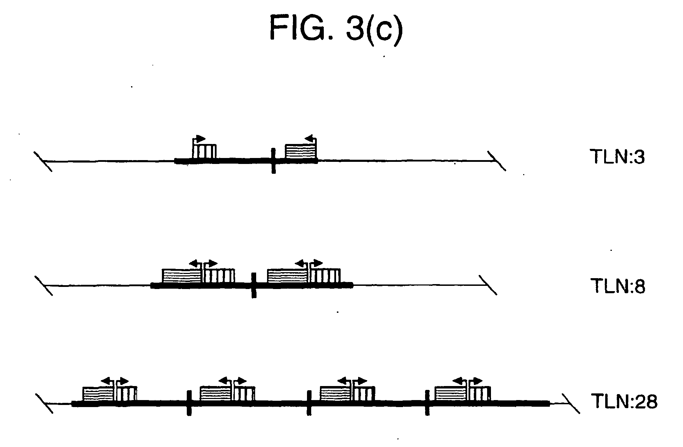

Figure 3C shows an updated summary of the transgene organisation in the TLN mouse lines. The figure shows the predicted organisations of the TLN transgene arrays in each of the mouse lines. Only functional genes are shown and only one of the 3 possible arrangements of the TLN:3 mice is indicated.

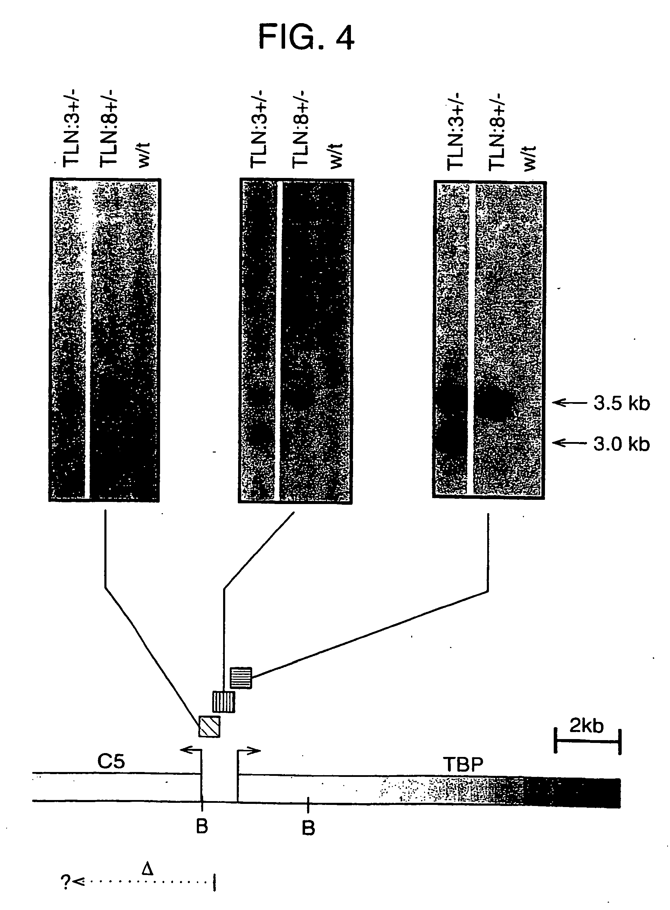

Figure 4 shows analysis of the deletion in TLN:3 mice. A series of probes were hybridised to Southern blots of TLN:3 DNA. Only the furthest 5' probe gave a single band, indicating that the deleted copy did not contain this sequence. The deletion maps to a region upstream of the major TBP mRNA CAP sites, Ets factor binding site and DNase I hypersensitive site. It is currently unknown if the entire 5' region is deleted in this copy or a small internal deletion has occurred.

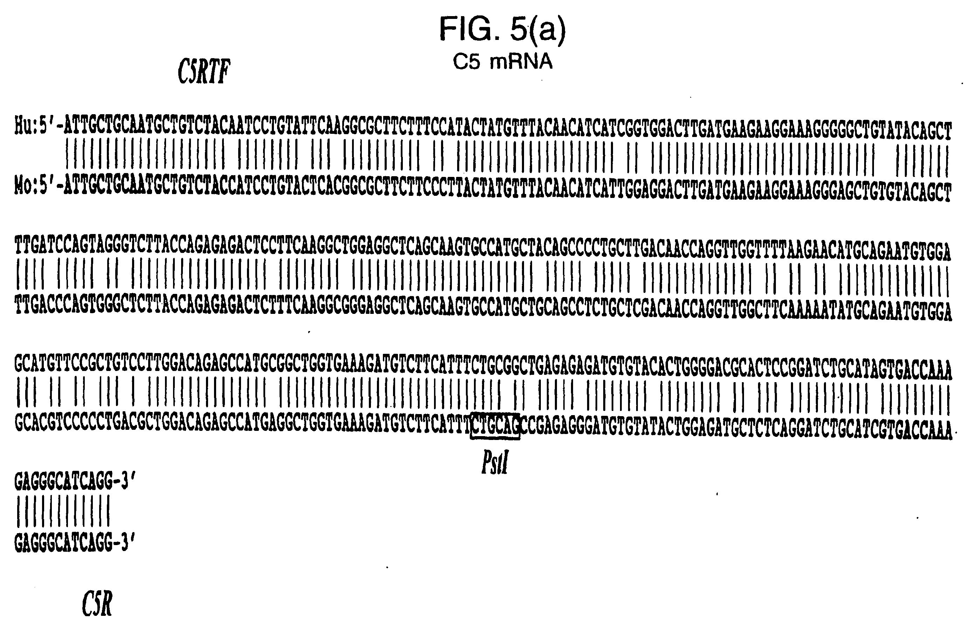

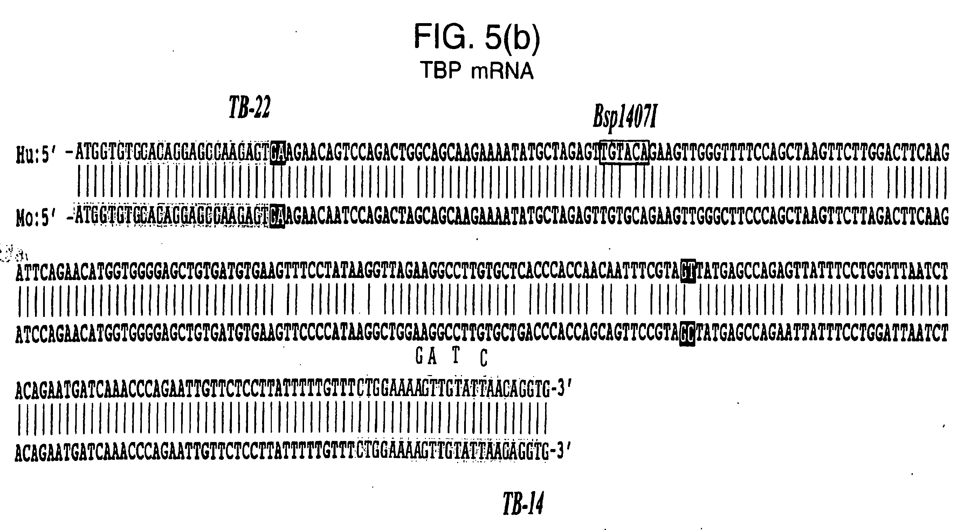

Figure 5 shows the comparison of TBP and C5 mRNA sequences from human and mouse. (a)The human C5 mRNA sequence from nt. 358 to 708 (Genbank accession no. D00761) exhibits significant homology to the mouse sequence (indicated by a vertical bar) from nt. 355 to 705 (Genbank accession no. X80686). RT-PCR amplification of both human and mouse mRNAs produces a mixture of 350bp DNA molecules from both species. The primer locations (highlighted, 5' primer C5RTF, 3' primer C5R) are positioned so as to span a number of exons, eliminating error from PCR amplification from contaminating genomic DNA. Although the intron/exon structure of either the human or mouse gene is limited, the distance between the primers is such that they are positioned in different exons. Mouse and human PCR products can be distinguished by incubation with *Pst*I that will only cut the mouse sequence. Radiolabelling of the C5RTF primer gives a product of 173nt when resolved on a denaturing polyacrylamide gel. (b)Similar analysis for human TBP mRNA sequence from nt. 901 in exon 5 to nt. 1185 in exon 7 (Genbanh accession no. M55654) and mouse TBP mRNA from positions 655 to 939 (Genbank accession no. D01034). The last nucleotide from an exon and the first nucleotide from the next exon are shown in red. The primers used (highlighted) were 5' TB-22 and 3' TB-14. The size of the amplified product from both species with the primers shown (boxed) is 284 bp. The *Bsp1407I* site 63 nt from the 5' end of the PCR products allows human and mouse transcripts to be distinguished. The size of the human specific product on a polyacrylamide gel with radiolabelled TB-14 is 221nt.

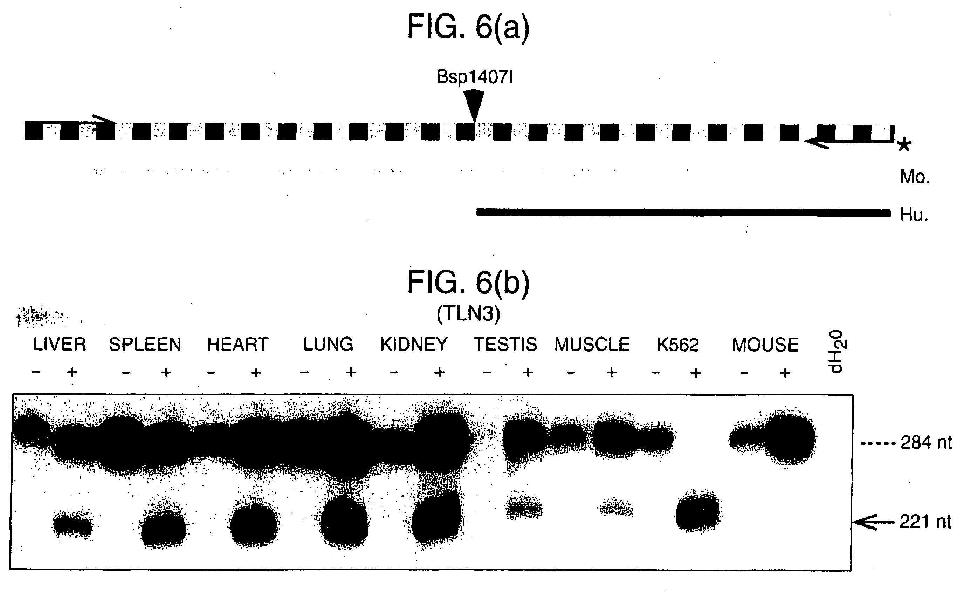

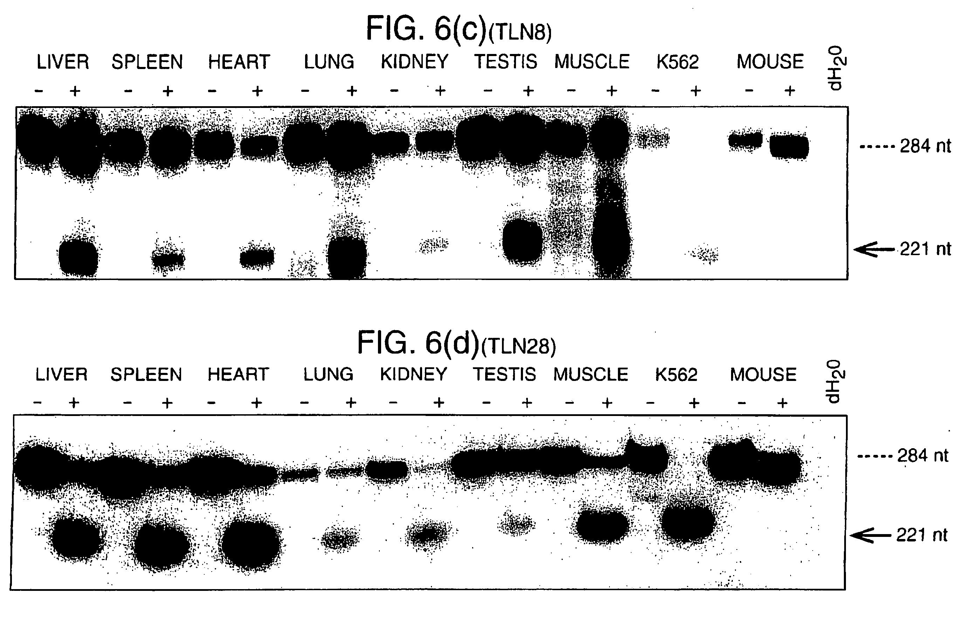

Figure 6 shows expression analysis of human TBP expression in the TLN transgenic mice.
   Total RNA (1µg) from various mouse tissues was used in a reverse transcription reaction using Avian Myeloblastosis Virus reverse transcriptase. As a control, human RNA from K562 cells and non-transgenic mouse RNA were also used. (a)Location of the recognition site for the human specific restriction endonucleases within the TB22/14 RT-PCR products. (b)Analysis of TLN:3 expression in various tissues. As can be seen, the level of human expression is physiological in all tissues. (c) Similar analysis for TLN:8. (d)Analysis of TLN:28 indicates levels of human TBP mRNA are again expressed at comparable levels to the endogenous gene.

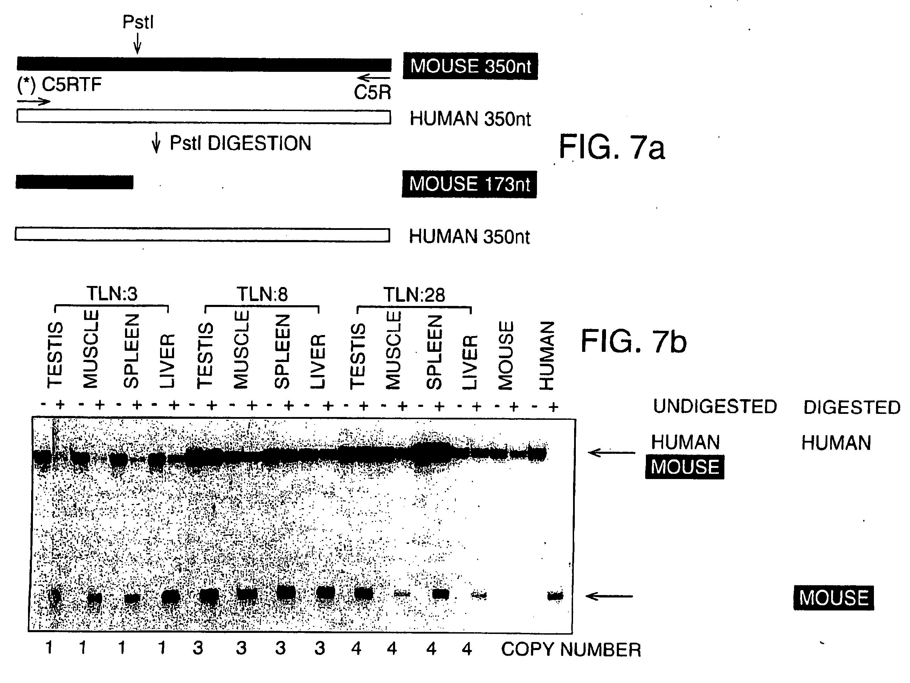

Figure 7 shows expression analysis of human C5 expression in the TLN transgenic mice.
   Analysis was performed as in figure 6. The upper panel (a) shows the location of the recognition site for the mouse specific restriction endonucleases within the C5RTF/C5R RT-PCR products. (b) Analysis of C5 expression in various tissues of TLN transgenics can be seen, the level of human expression is physiological in all tissues tested.
Figure 8 shows a summary of quantification of (a) human TBP gene expression and (b) human C5 gene expression inTLN transgenic mice.
Figure 9 shows a schematic representation of the pWE-TSN cosmid.
Figure 10 shows transgene copy number determination of pWE-TSN L-cell clones.
   Mouse L-cells were transfected with the pWE-TSN cosmid, DNA isolated and used to generate Southern blots. Blots were probed with a DNA fragment from the two copy murine vav locus and a probe located -7kb from the TBP gene. Copy numbers were determined from the ratio of the three copy TLN:8 control and are given underneath each lane. Copy numbers ranged from 1 to 60.

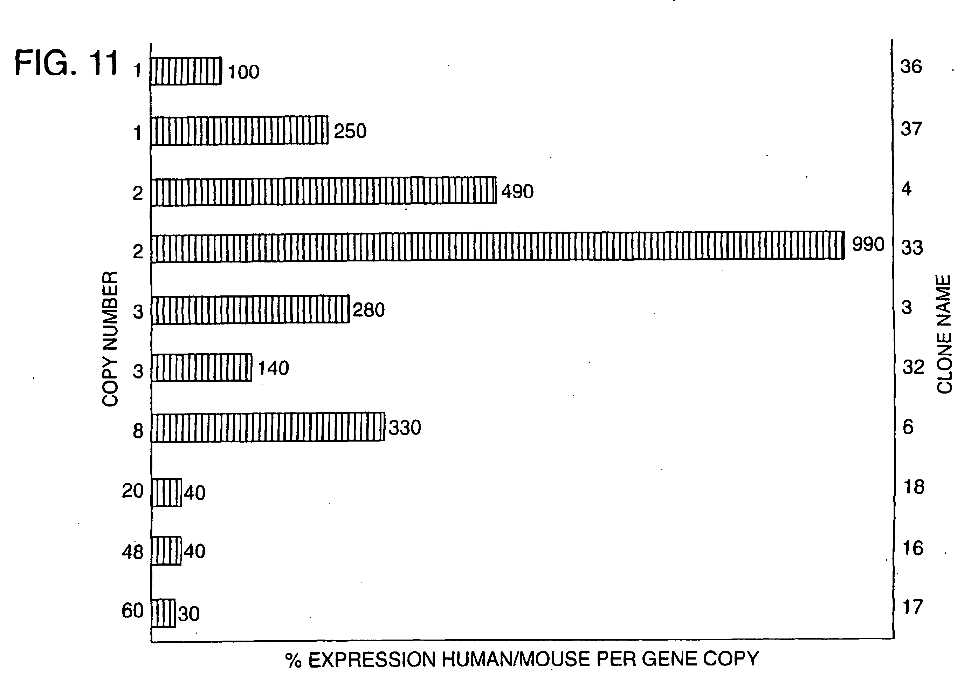

Figure 11 shows a summary of expression of pWE-TSN cosmid clones in mouse L-cells.

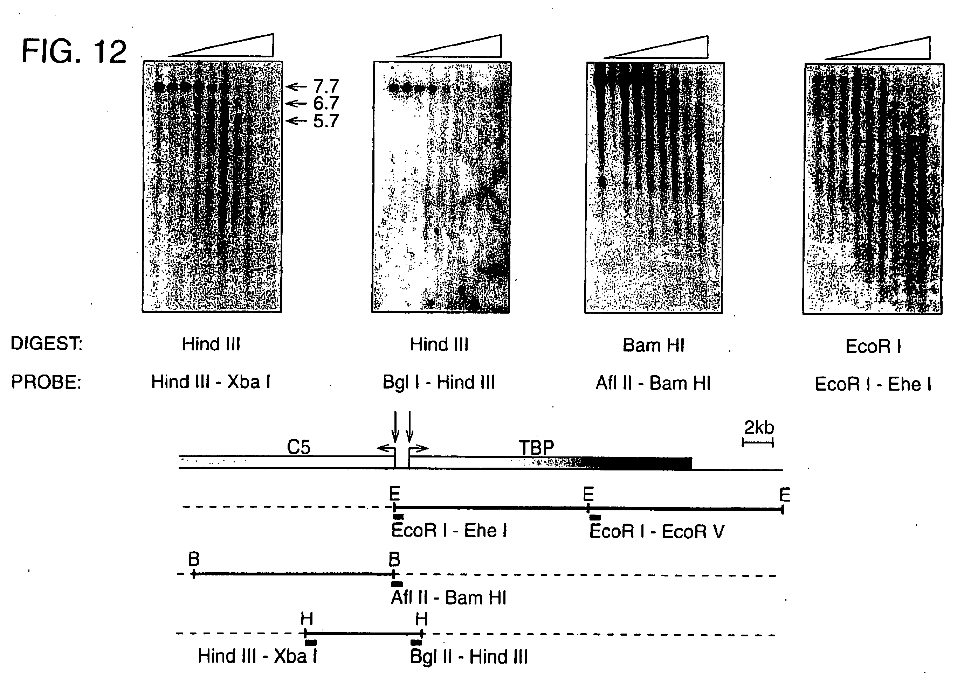

Figure 12 shows DNase I hypersensitive site analysis of the human TBP locus. Probes located over a 40kb region surrounding the TBP gene were used to probe Southern blots of K562 nuclei digested with increasing concentrations of DNase I. Only two hypersensitive sites were found, at the promoters of the PSMB1 and the TBP gene. Increased DNase I concentration is shown from left to right in all cases.

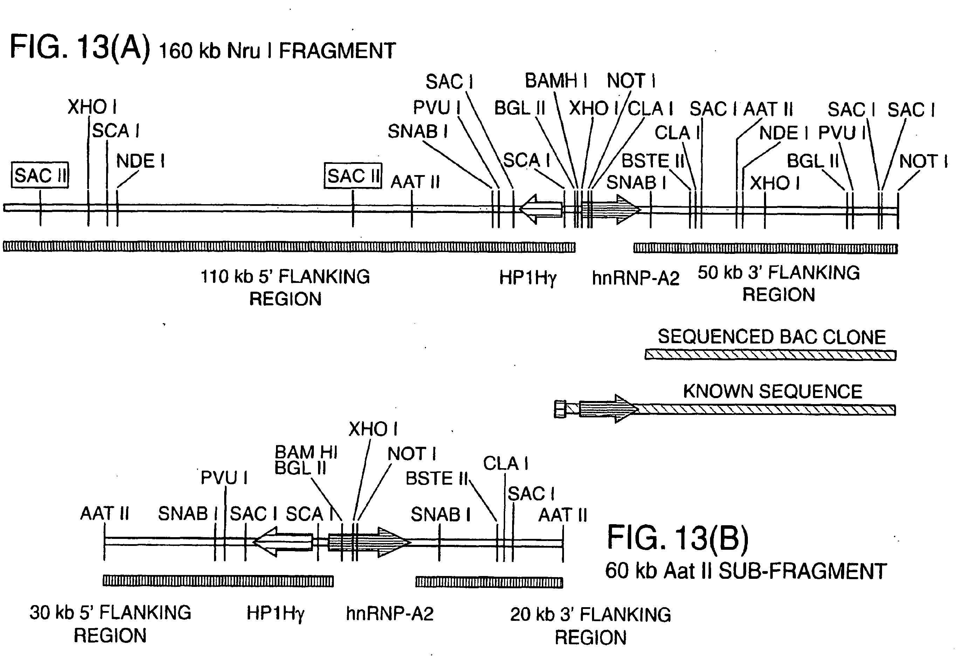

Figure 13A shows a schematic representation of the human hnRNP A2 gene locus showing the large 160kb pCYPAC-derived clone MA160. The reverse arrow denotes the HP1H-γ gene. The two SacII sites, which may represent the presence of methylation-free islands are boxed.
Figure 13B shows the 60kb *Aat*II sub-fragment derived from MA160. Both of these have been used for generation of transgenic mice.

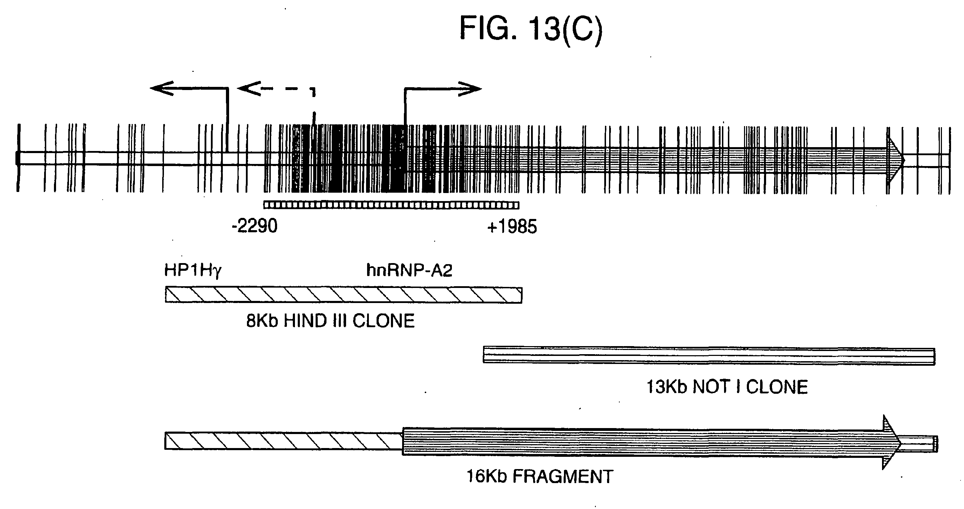

Figure 13 Chows the extent of the CpG-island (red bar) spanning the 5' end of the hnRNP A2 gene. The CpG residues are denoted as vertical lines. The numbers are in relation to the transcriptional start site (+1) of the hnRNP A2 gene (solid arrow). The broken arrow denotes the position of the divergently transcribed HP1H-γ gene. The 16kb sub-fragment that contains the intact hnRNP A2 gene is also shown.

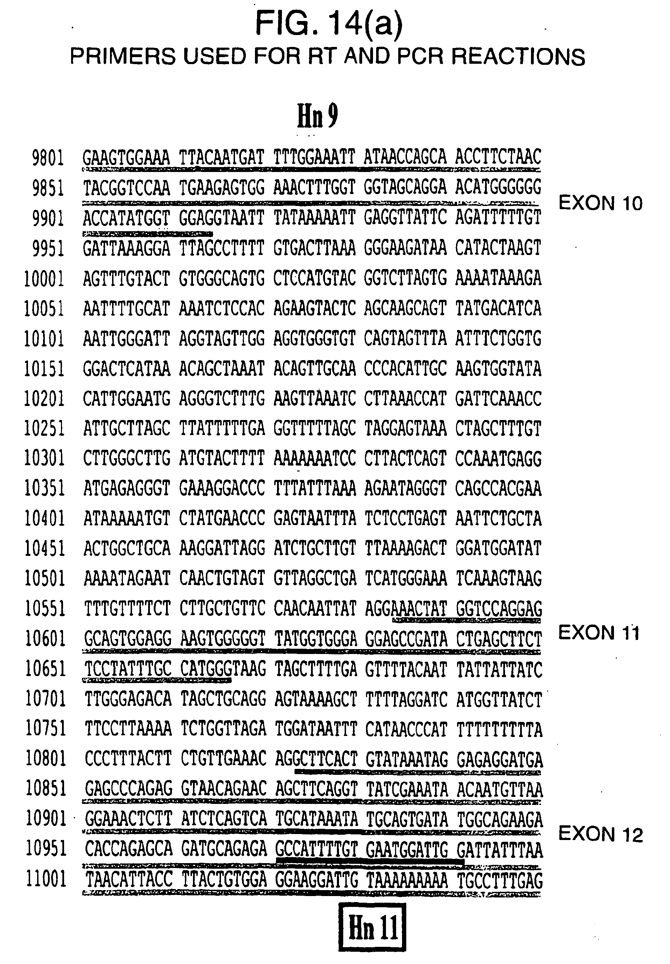

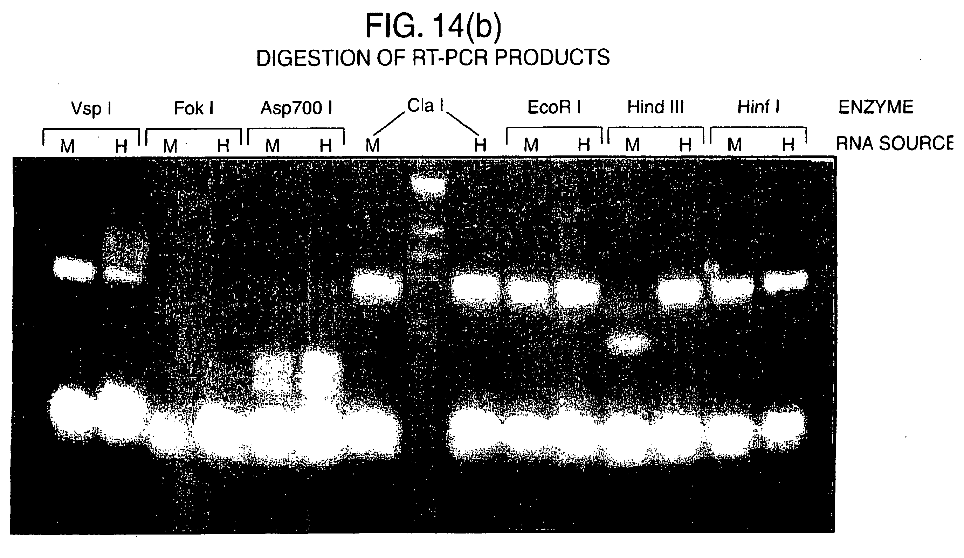

Figure 14 shows quantification of human and mouse hnRNP A2 gene expression. Human (K562) and mouse RNA was reverse transcribed with a primer to exon 12 of the hnRNP2 gene. Samples were subsequently amplified by PCR with primers Hn9 and Hn11 spanning exons 10 to 12. The product produced was then digested with random enzymes to find a cut site unique to each species. The mouse product can be seen to contain a *Hind*III that is not present in the human product.

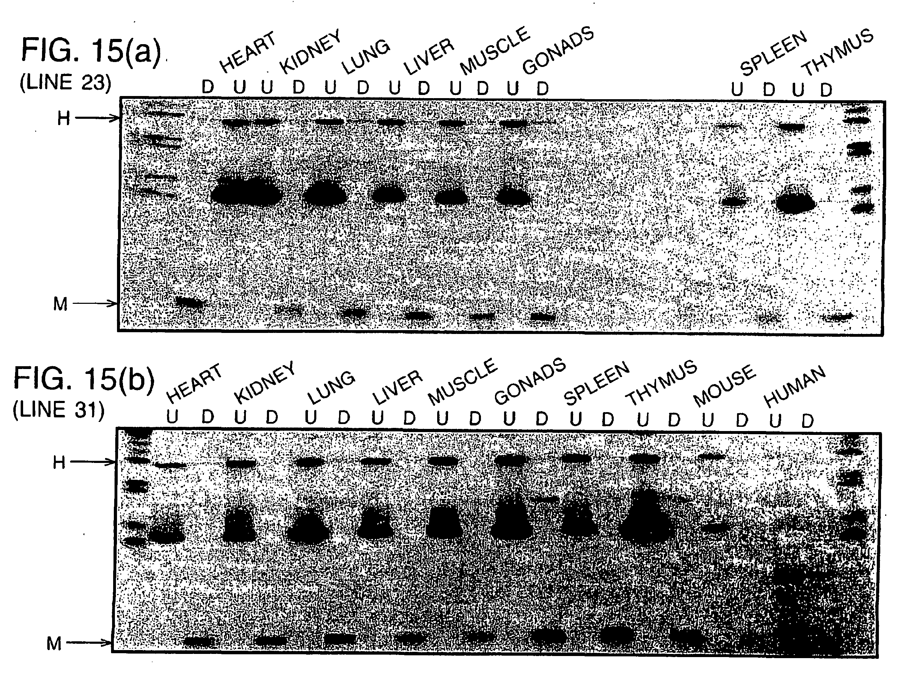

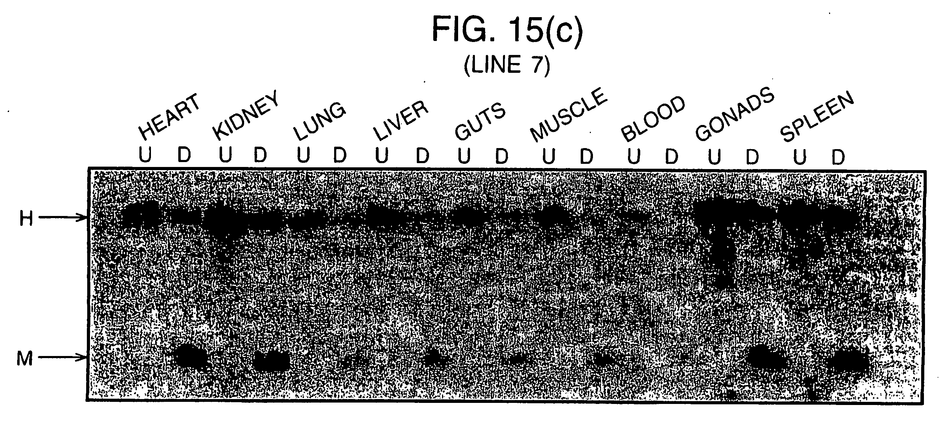

Figure 15 shows the analysis of human hnRNP A2 expression in transgenic mice microinjected with the Aa60 fragment (Figure 13B). Total RNA from various tissues was analysed as described in Figure 15. After RT-PCR, samples were either untreated (-) or digested with *Hind*III (+) and then separated on a polyacrylamide gel to resolve the human (H) and mouse (M) products. Intensity of the bands was measured by Phosphorlmager analysis.

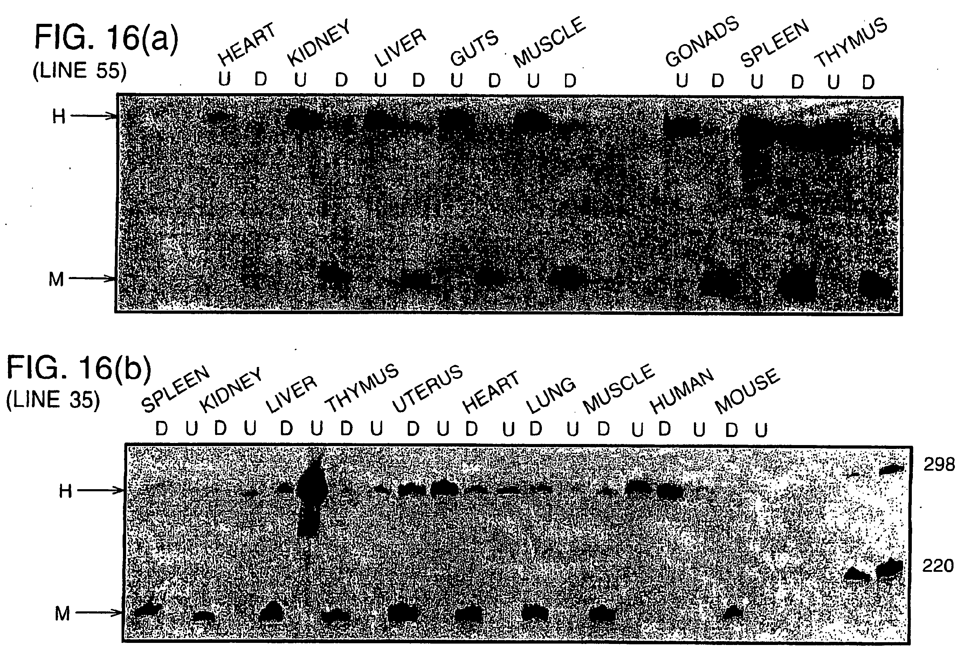

Figure 16 shows the analysis of human hnRNP A2 expression by transgenic mice microinjected with the 160kb *Nru*I fragment (Figure 13A). A transgenic mouse was dissected and total RNA extracted from tissues. The RNA was reverse transcribed by Hn1 and then amplified by PCR using primers Hn9 and Hn1 of which Hn9 was radioactively end-labelled with ³²P. Samples were either untreated (-) or digested with *Hind*III (+) and then separated on a 5% polyacrylamide gel in the presence of 8M urea as denaturant to resolve the human (H) and mouse (M) products. Intensity of the bands was measured by Phosphorlmager analysis.

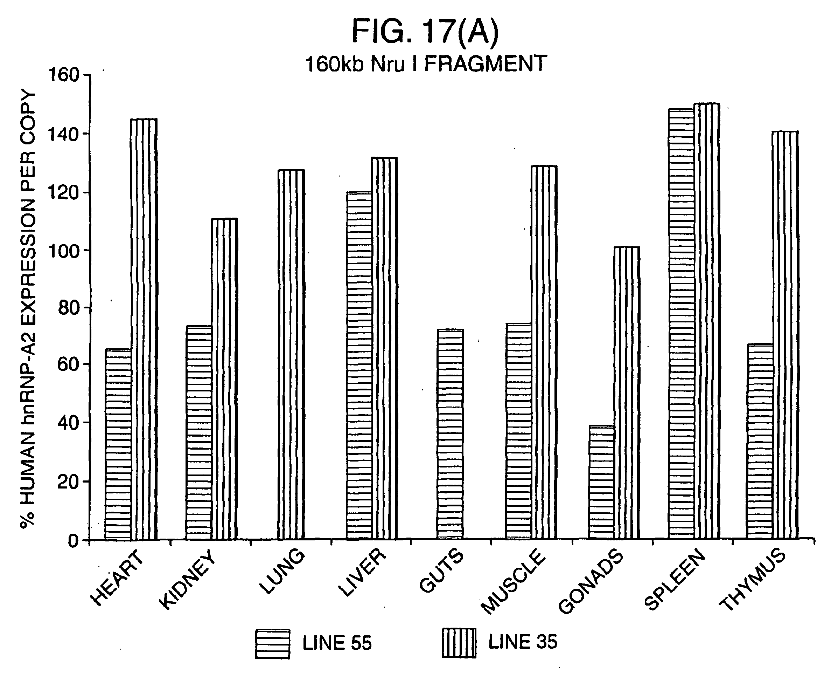

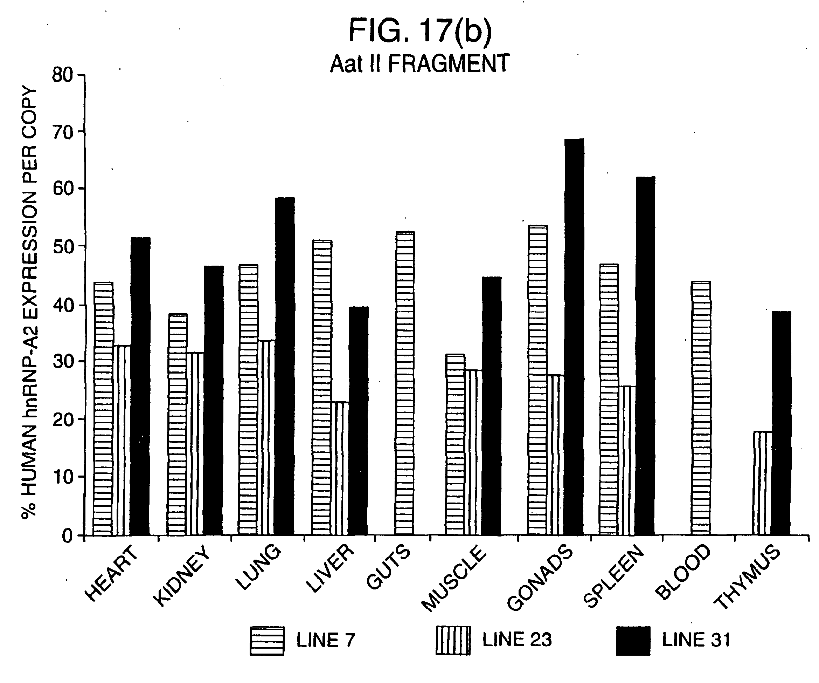

Figure 17 shows the quantification of hnRNP A2 transgene expression. The RT-PCR analysis of human hnRNP A2 transgene expression in various mouse tissues was quantified by PhosphorImager. Levels are depicted as a percentage of murine hnRNP A2 expression on a transgene copy number basis. A: Mice harbouring MA160 (see Figure 15). B: Mice harbouring Aa60 (see Figure 16).

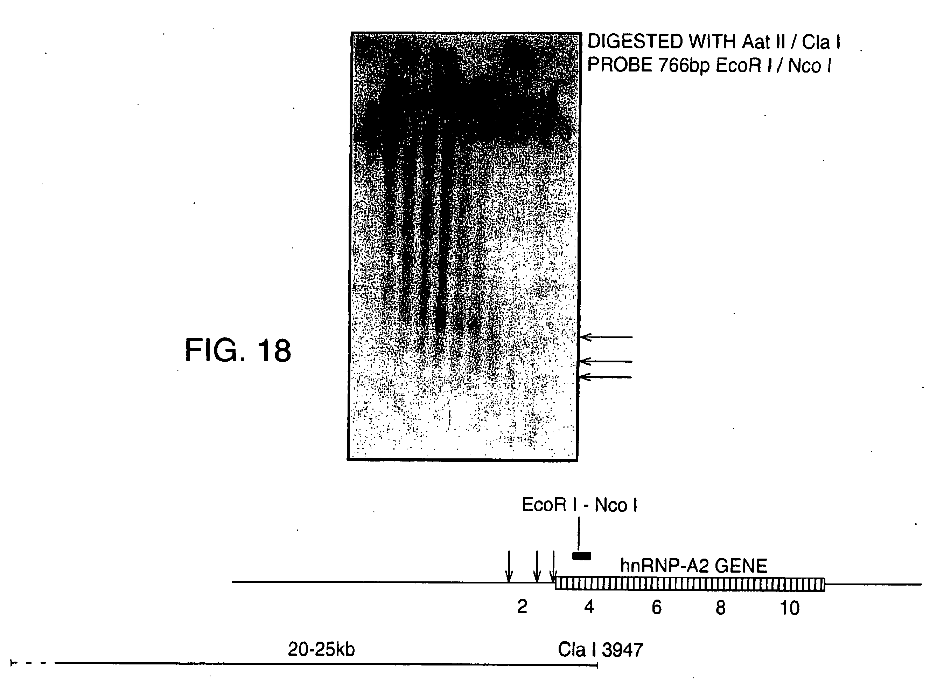

Figure 18 shows DNase I hypersensitive site mapping of the human hnRNP A2 gene locus. Nuclei from K562 cells were digested with increasing concentrations of DNase I. DNA from these nuclei was subsequently digested with a combination of *Aat*II and *Nco*I restriction endonucleases and Southern blotted. The blot was then probed with a 766bp *Eco*RI/*Nco*I fragment from exon II of the hnRNP A2 gene. Three hypersensitive sites were identified corresponding to positions -1.1, -0.7 and -0.1kb 5' of the hnRNP A2 transcriptional start site.

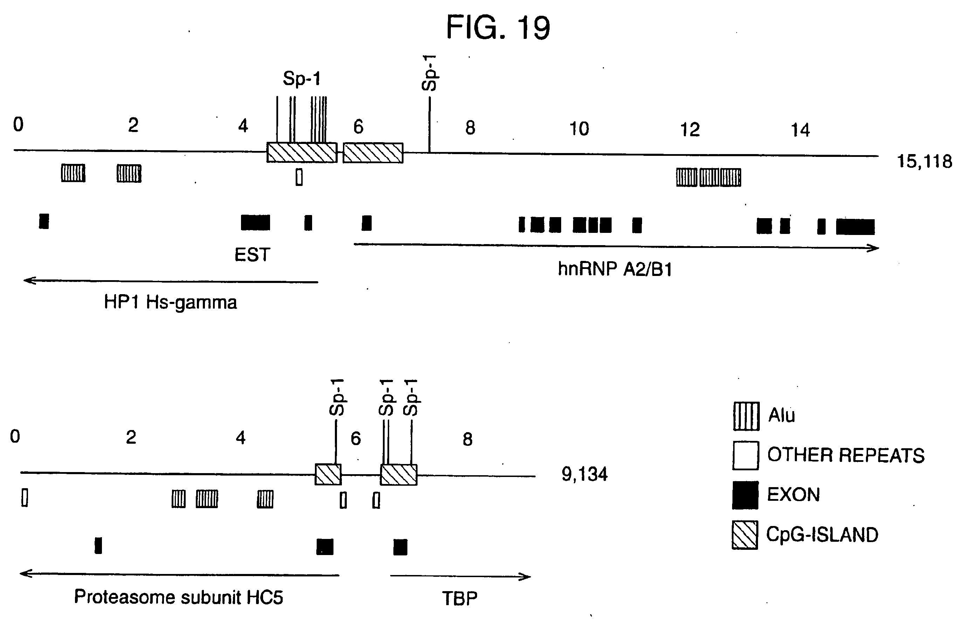

Figure 19 shows the bioinformatic analysis and sequence comparisons between the hnRNP A2 and the TBP loci.
Figure 20 shows the nucleotide sequence of a genomic clone of the TBP locus beginning at the 5' *HindIII* site (nucleotides 1 to 9098).

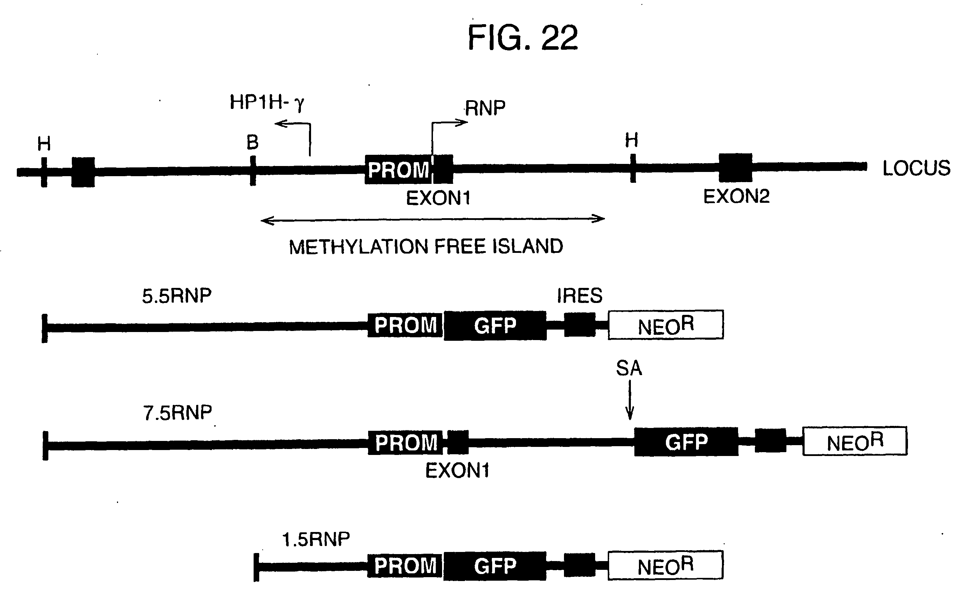

Figure 21 shows the nucleotide sequence of a genomic clone of the hnRNP locus beginning at the 5' *Hind*III site shown in Figure 22 (nucleotides 1 to 15071).
Figure 22 shows the expression vectors containing sub-fragments located in the dual promoter region between RNP and HP1H-γ which were designed using both GFP and a Neo^{R} reporter genes. The vectors are: a control vector with the RNP promoter (RNP) driving GFP/Neo expression; a vector comprising the 5.5kb fragment upstream of the RNP promoter region and the RNP promoter (5.5RNP); vectors constructed using a splice acceptor strategy wherein the splice acceptor/branch concensus sequences (derived from exon 2 of the RNP gene) were cloned in front of the GFP gene, resulting in exon I/part of intron 1 upstream of GFP (7.5RNP, carrying approximately 7.5kb of the RNP gene preceeding the GFP gene; and a vector comprising the 1.5kb fragment upstream of the RNP promoter region and the RNP promoter (1.RNP).

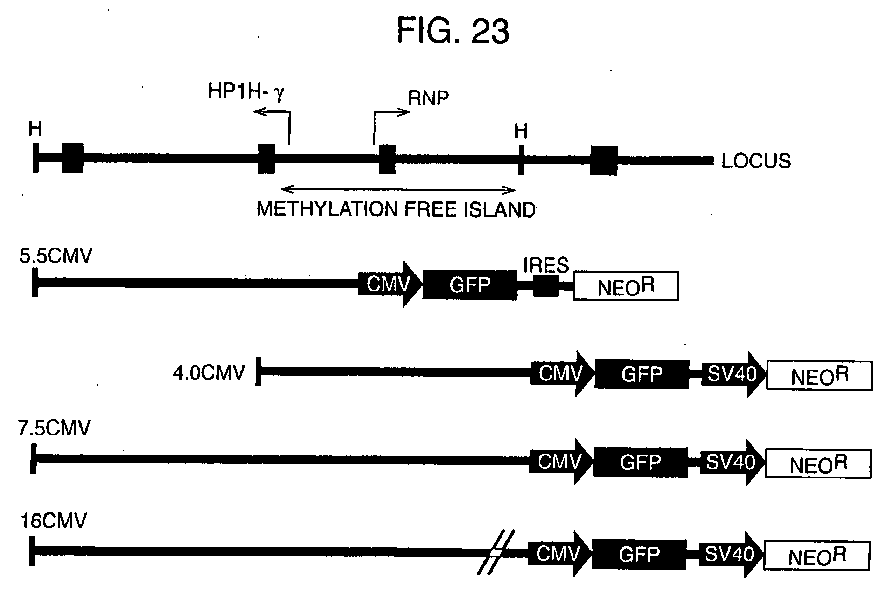

Figure 23 shows expression vectors containing sub-fragments located in the dual promoter region between RNP and HP1H-γ which were designed using both GFP and a Neo^{R} reporter genes. The vectors comprise the heterologous CMV promoter. The vectors are: control vectors with the CMV promoter driving GFP/Neo expression with (a) internal ribosome entry site sequences (CMV-EGFP-IRES) and (b) with without internal ribosome entry site sequences and an SV40 promoter upstream of the Neo^{R} reporter gene (CMV-EGFP); a vector comprising the 5.5kb fragment upstream of the RNP promoter region and the CMV promoter driving GFP/Neo expression with internal ribosome entry site sequences (5.5CMV); a vector comprising 4.0kb sequence encompassing the RNP and the HPlH-γ promoters and the CMV promoter driving GFP-Neo expression with an SV40 promoter upstream of the Neo^{R} reporter gene (4.OCMV); and a vector comprising 7.5kb sequences of the RNP gene including exon 1 and part of intron 1, and the CMV promoter driving GFP-Neo expression with an SV40 promoter upstream of the Neo^{R} reporter gene (7.5CMV).

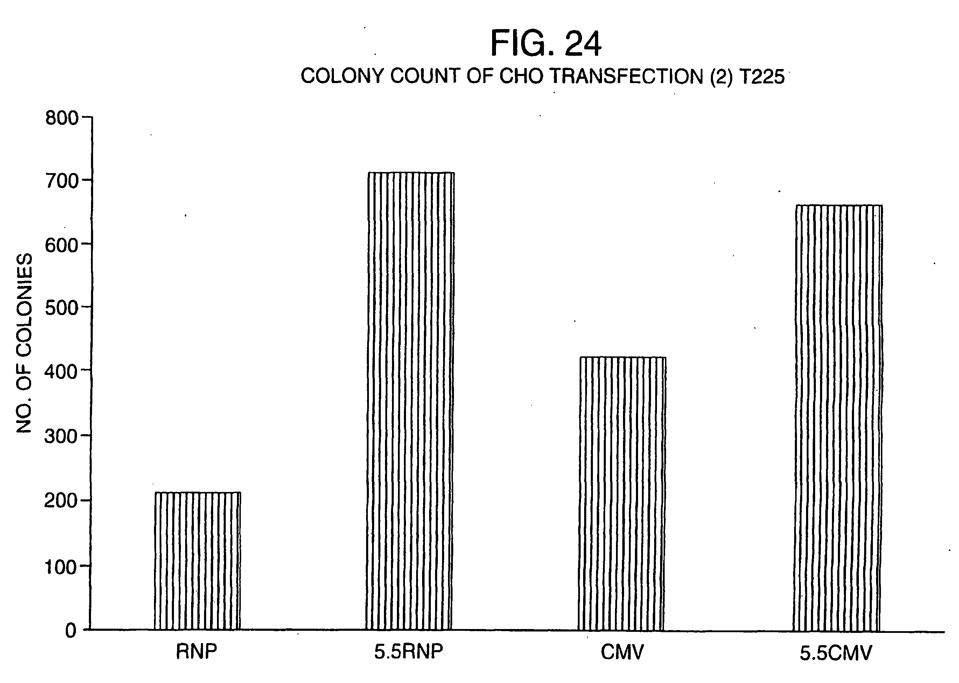

Figure 24 shows the number of G418^{R} colonies produced by transfecting the RNP- and CMV-constructs into CHO cells.

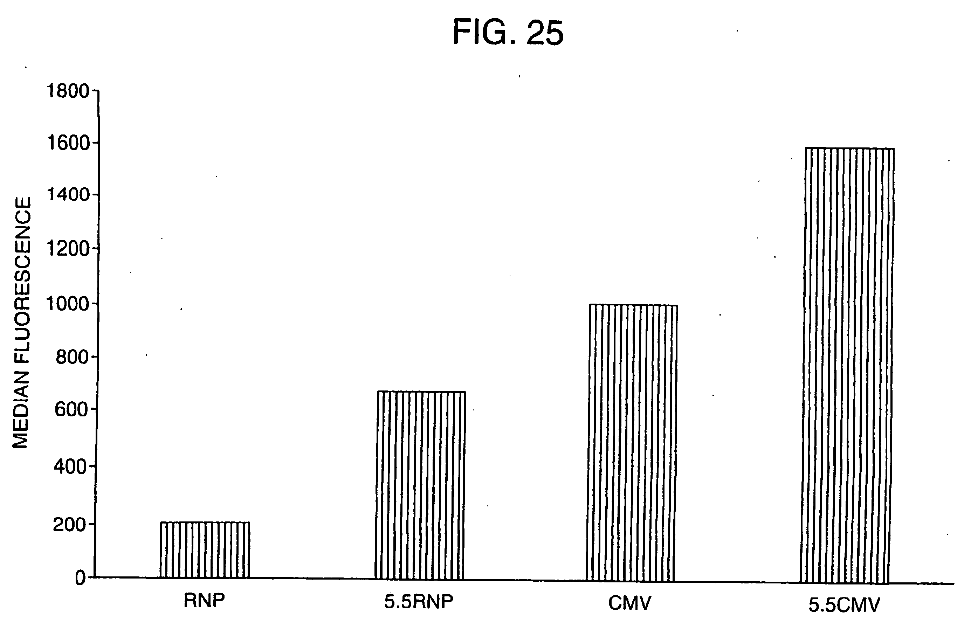

Figure 25 shows the comparison of GFP expression in G418-selected CHO clones transfected with RNP- and CMV-constructs with and without upstream elements.

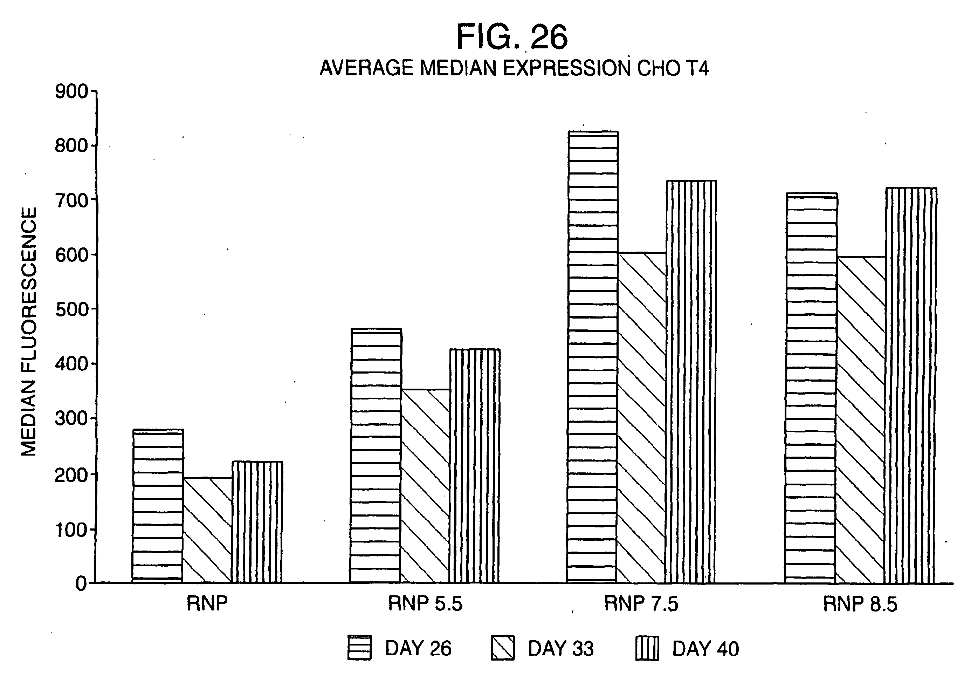

Figure 26 shows the average median GFP fluorescence levels in G418-selected CHO clones transfected with RNP- constructs with and without upstream elements over a period of 40 days.

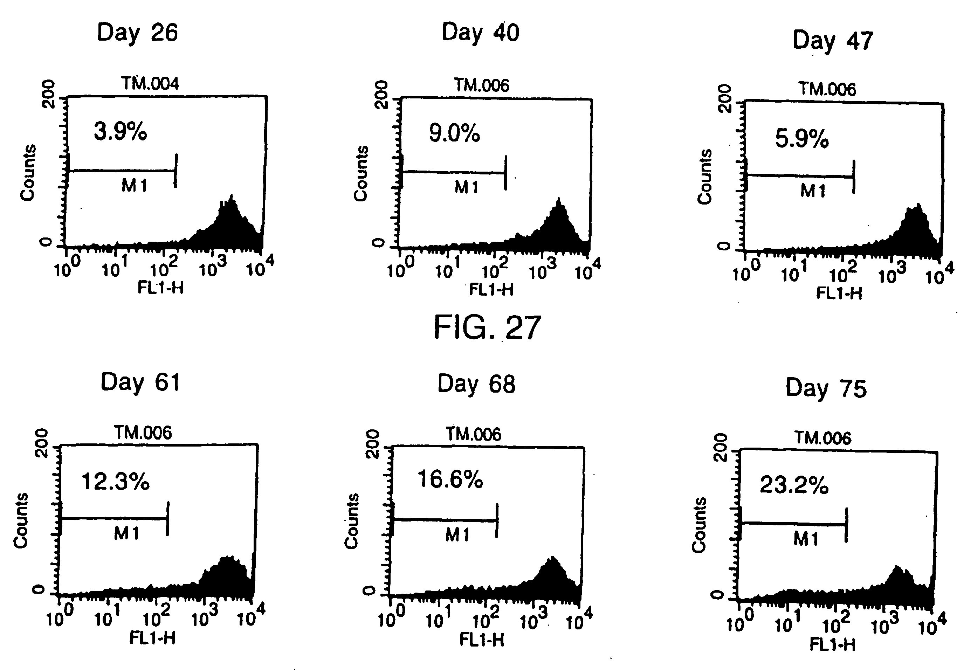

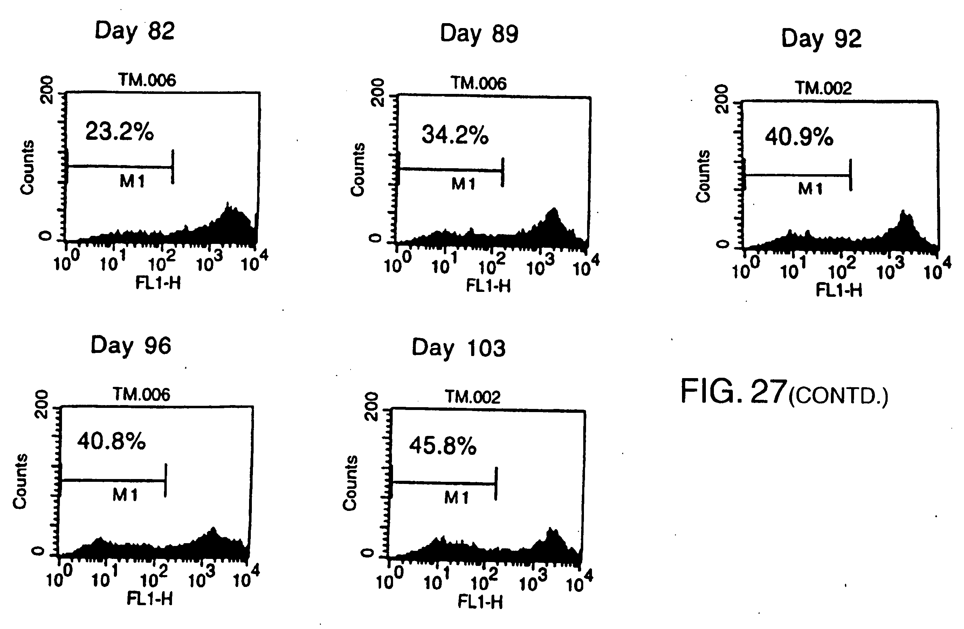

Figure 27 shows FACS profiles of GFP expression of CMV-GFP pools cultured in the absence of G418 followed over a period of 103 days.

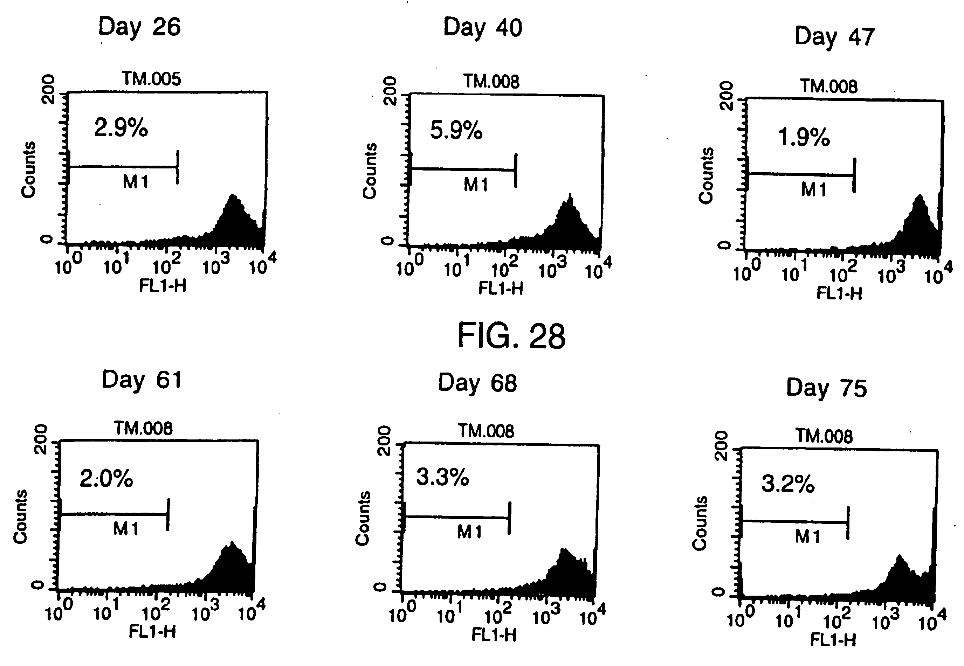

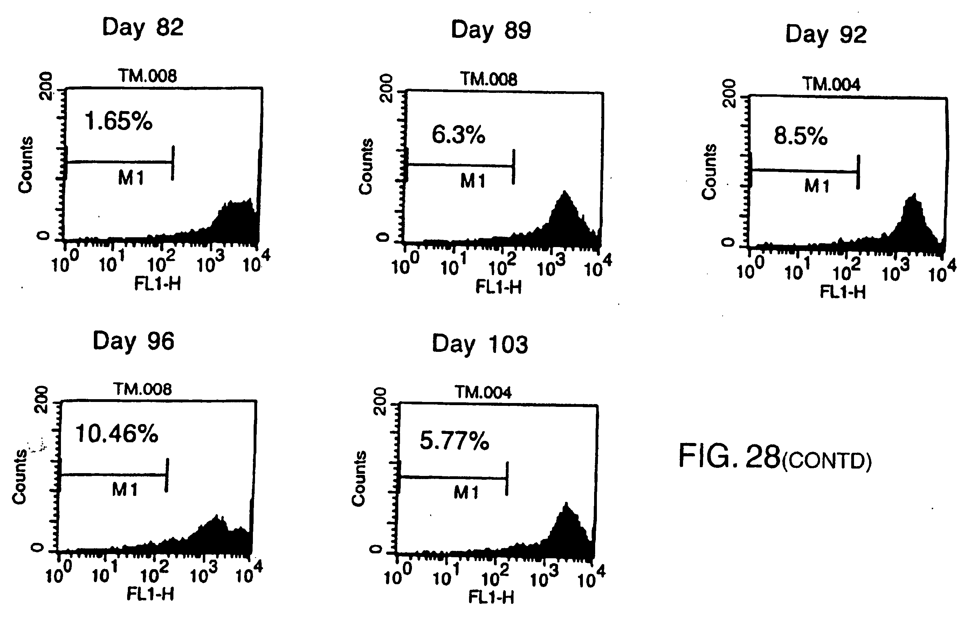

Figure 28 shows FACS profiles of GFP expression of 5.5CMV-GFP pools cultured in the absence of G418 followed over a period of 103 days.

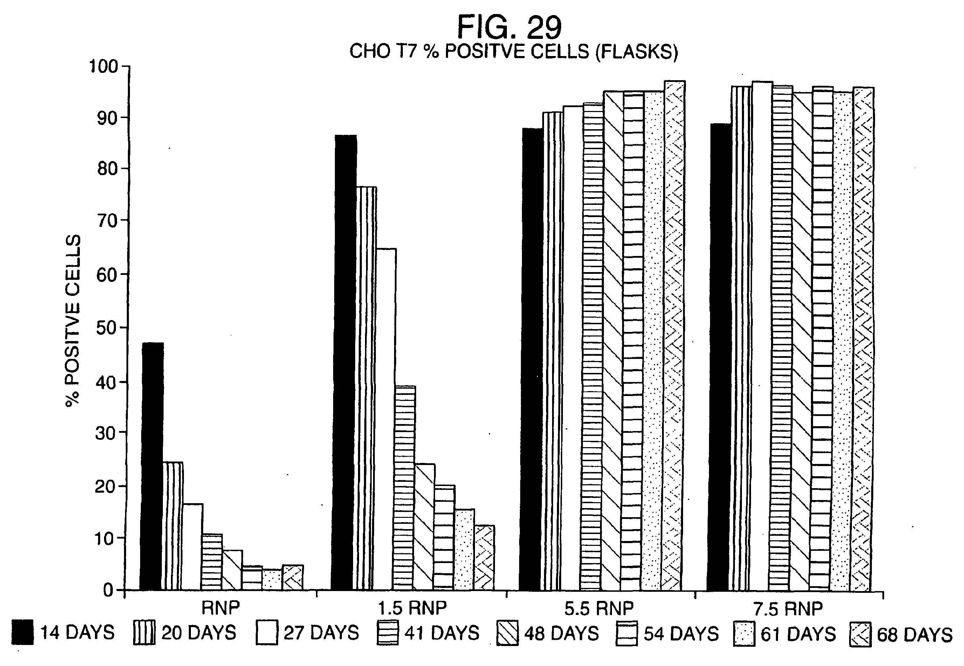

Figure 29 shows the percentage of transfected cells expressing GFP reducing over a 68 day time course.

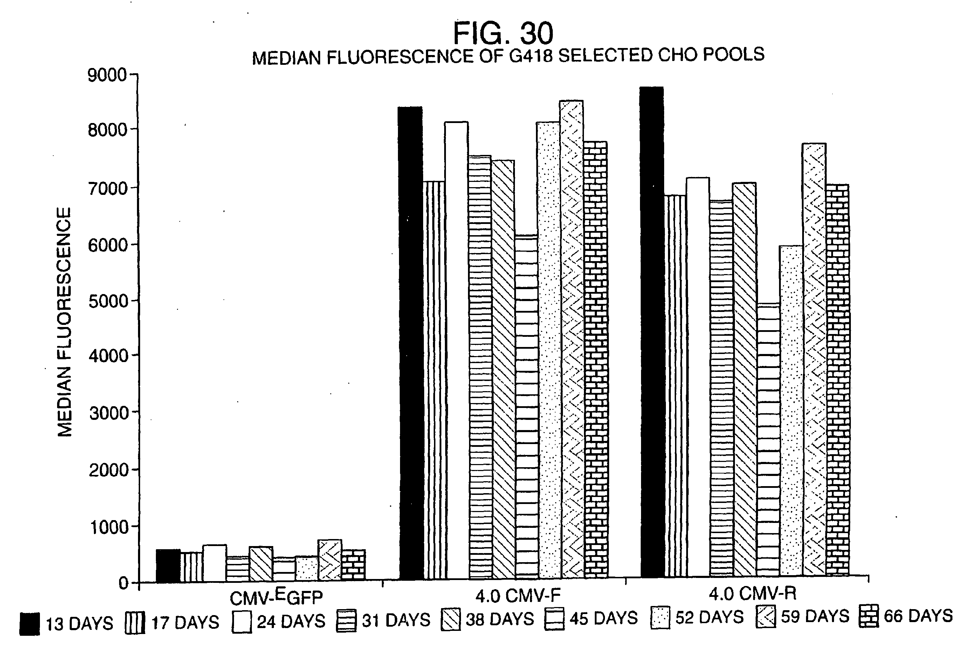

Figure 30 shows the median fluorescence of G418 selected cells transfected with CMV-contructs over a 66 day time course.

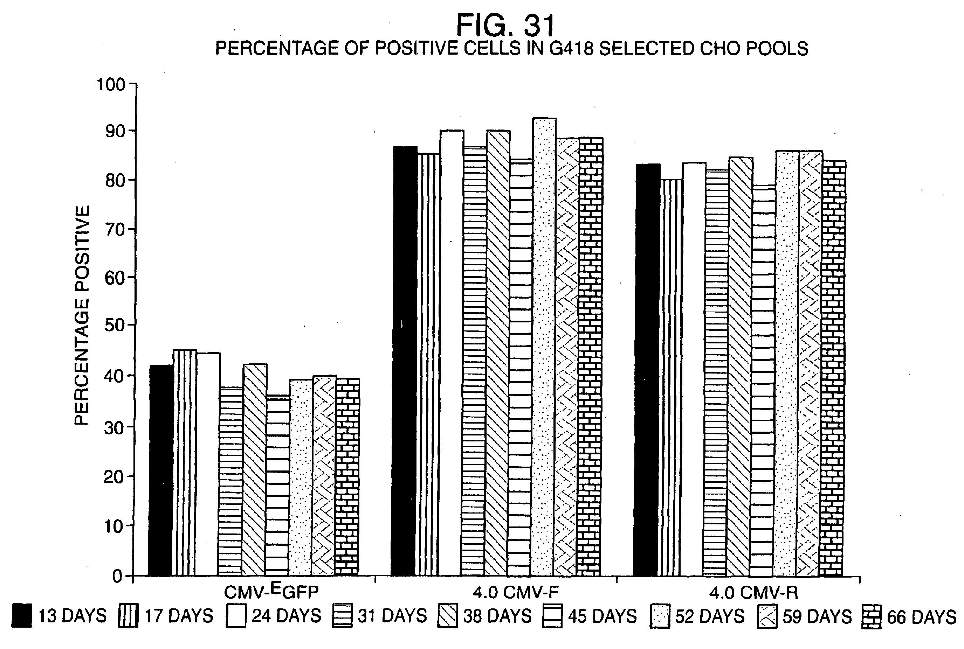

Figure 31 shows the percentage of positive G418 selected cells transfected with CMV-constructs over a 66 day time course.

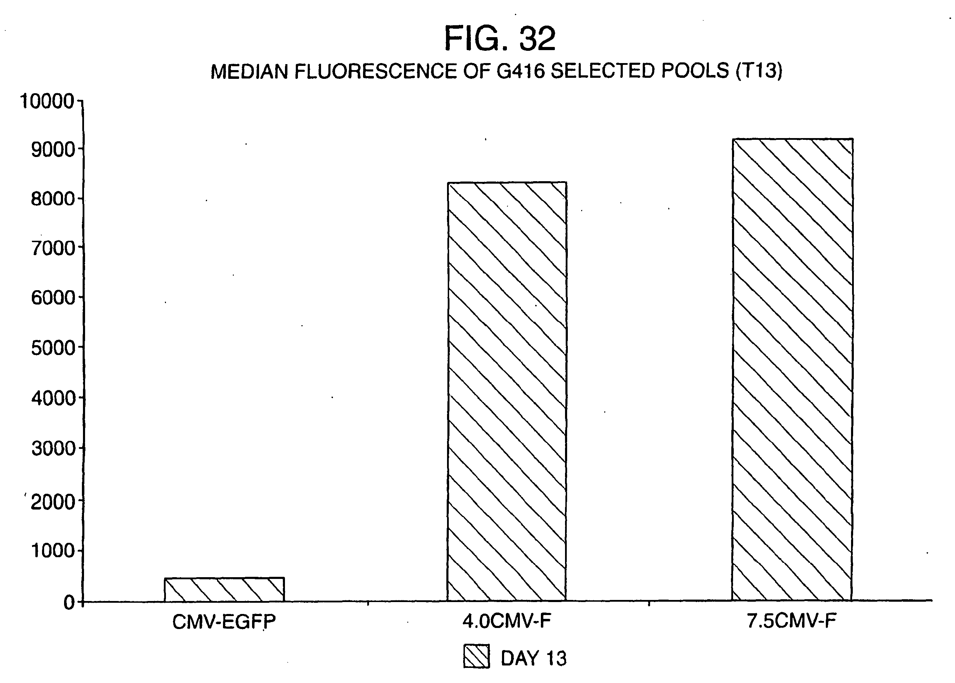

Figure 32 shows the median fluorescence of G418 selected cells transfected with CMV-constructs on day 13 after transfection.

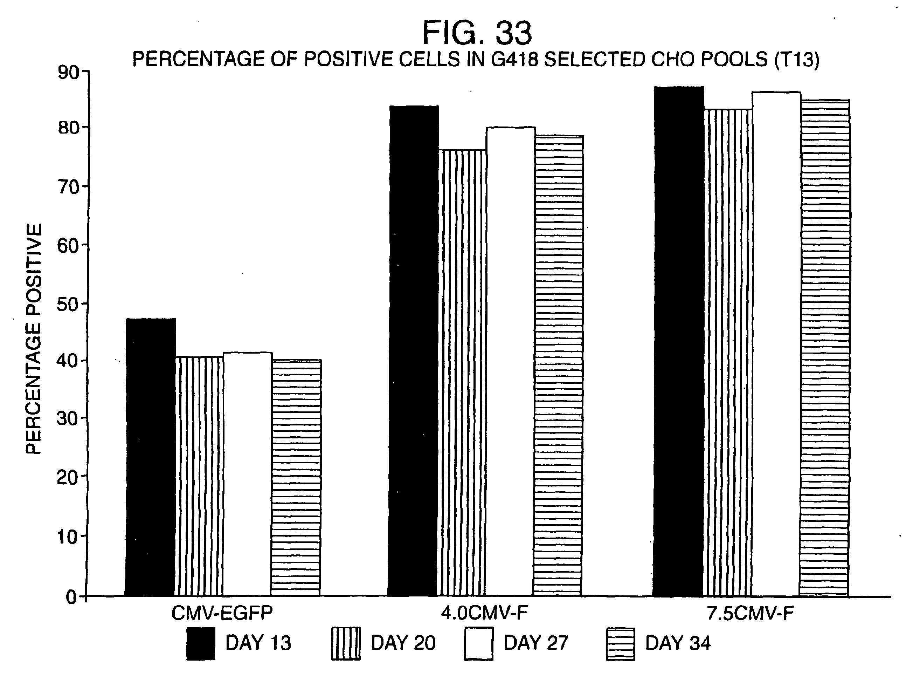

Figure 33 shows the percentage of positive G418 selected cells transfected with CMV-constructs over a 27 day time course.

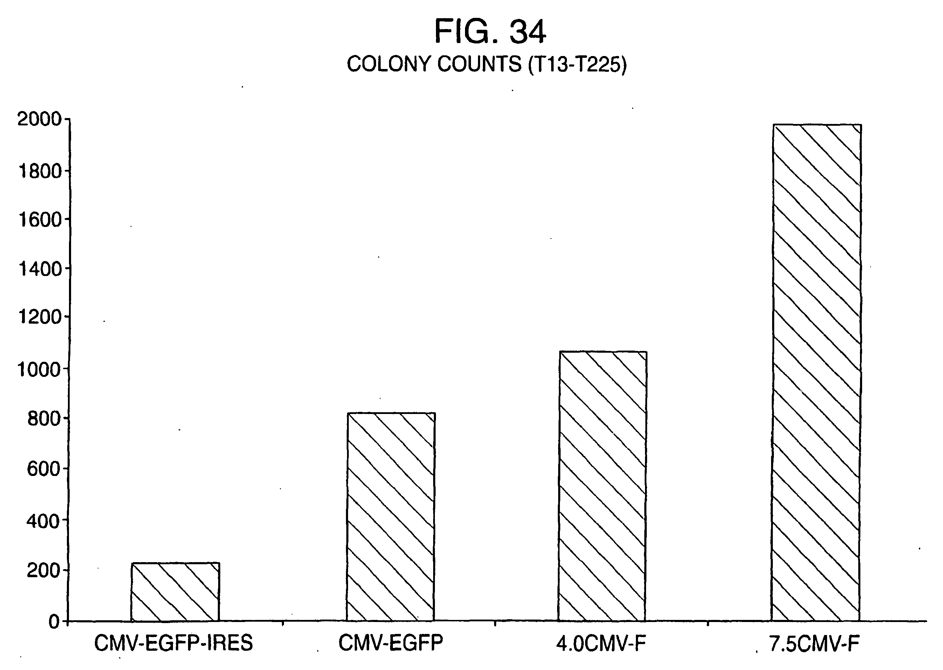

Figure 34 shows the colony numbers after transfection of CHO cells with various CMV-constructs.

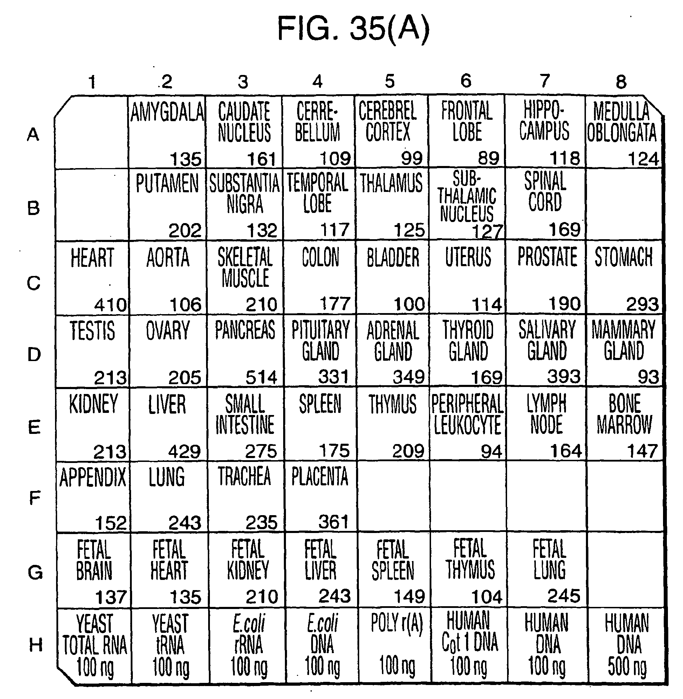

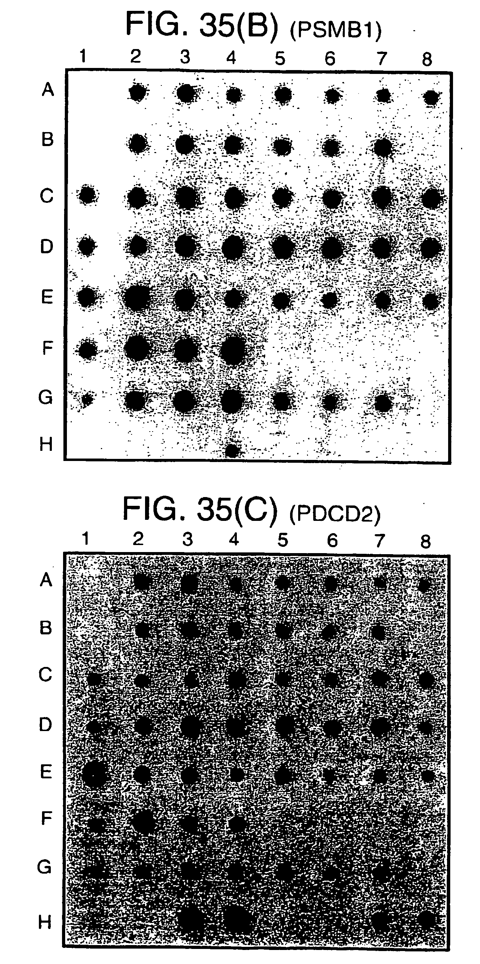

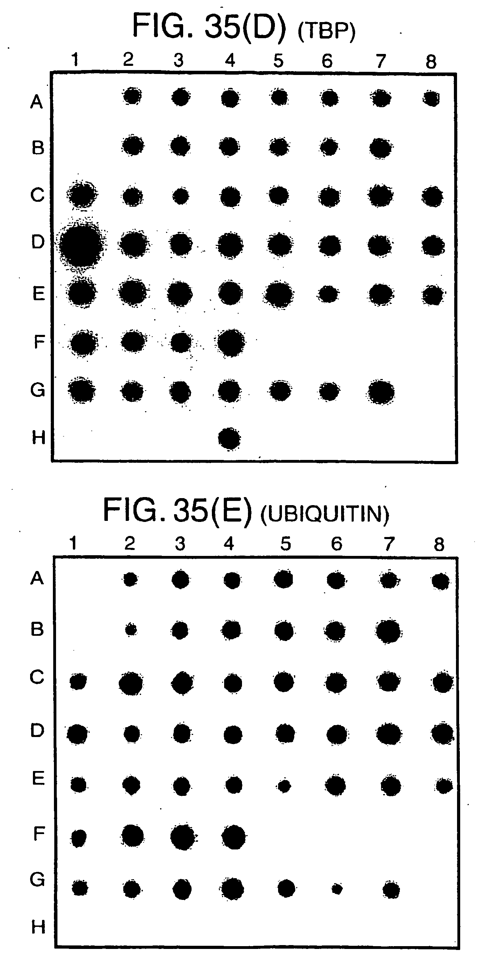

Figure 35 shows the dot blot analysis of human PSMB 1, PDCD2 and TBP mRNAs. The tissue distribution of mRNAs from genes within the TBP cluster using a human multiple tissue mRNA dot-blot: each segment is loaded with a given amount of poly(A)⁺ RNA (A, shown in ng below each tissue). The dot-blot was hybridised with (B) PSMB1 cDNA, (C) a 4.7 kb genomic fragment (MA445) containing a partial PDCD2 gene and (D) TBP cDNA. A ubiquitin control probe (E) demonstrated the normalisation process had been successful and that the RNA was intact.

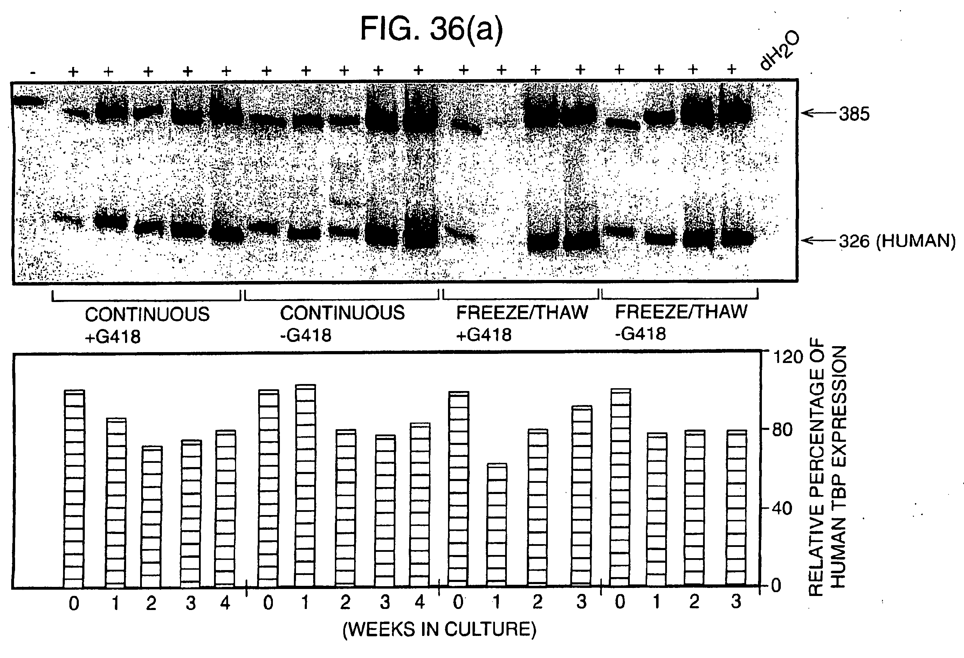

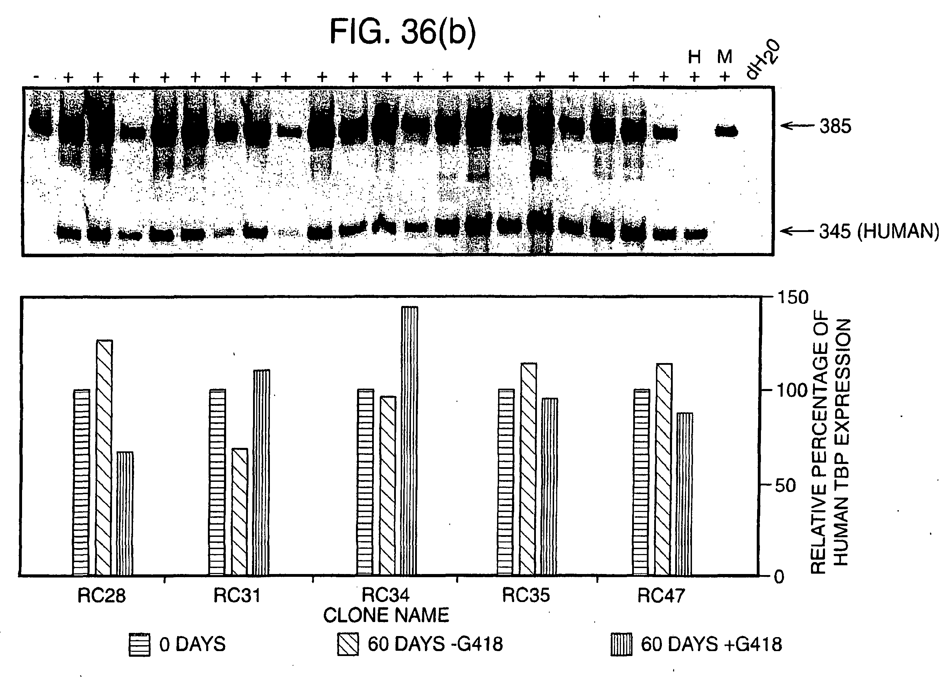

Figure 36 shows the effect of long-term culturing on pWE-TSN clones. At number of pWE-TSN mouse L-cell clones were grown continuously for 60 generations. For freeze/thaw, clones were stored in liquid nitrogen for at least 2 days, defrosted and cultured for 1 weeks before RNA was harvested and the cells frozen for the next cycle. Experiments were performed with and without G418 present in the medium. TBP expression was assayed by using TB14 oligonucleotides and a human-specific restriction endonuclease (as indicted by +) as described herein. All samples were analysed without the enzyme and were identical. A representative (-) sample is also shown.

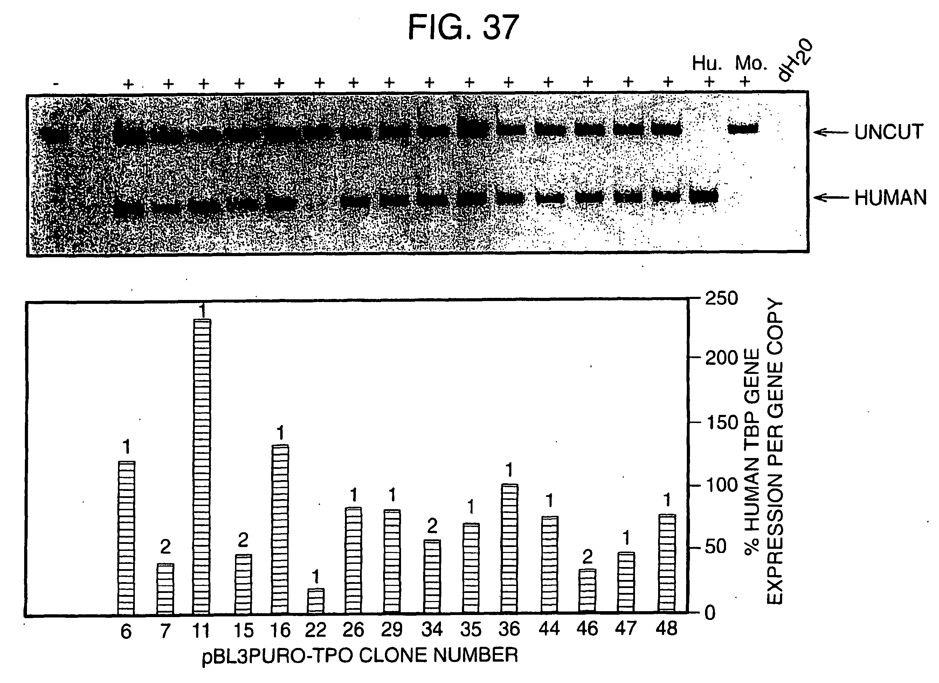

Figure 37 shows analysis of TBP gene expression in pBL3-TPO-puro clones. The analysis for TBP gene expression was performed using the TB14 primers with total RNA isolated from mouse L-cells transfected with the pBL3-TPO-puro construct as described herein. A (+) above a lane indicates that the PCR product has been digested with a human specific enzyme, (-) indicates no digestion (control). Human (K562) and mouse (non-transgenic lung) RNA controls are also shown as well as a no-RNA control (dH₂O). Arrows indicate the positions of the uncut (human and mouse or mouse) and human specific products. Expression values are corrected for copy number such that 100% expression means that a single copy of the transgene is expressing at the same level as one of the two endogenous mouse genes. All copy numbers varied from 1-2 and are indicated above each bar.

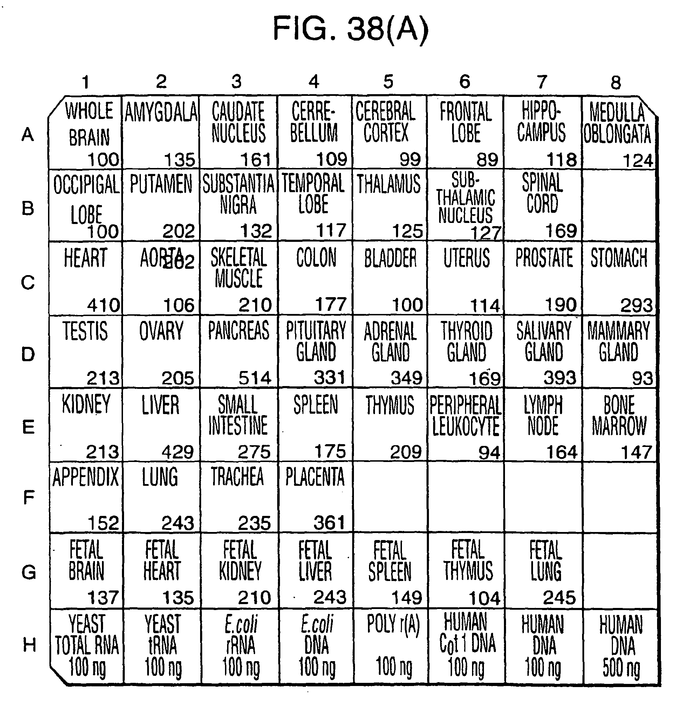

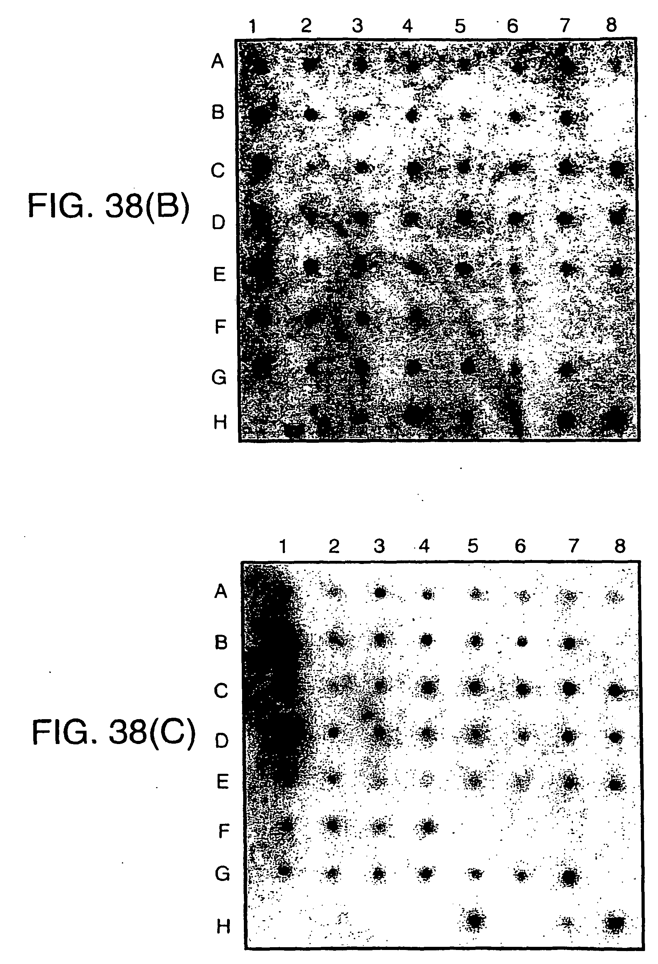

Figure 38 shows dot blot analysis of (B) human HPlγ mRNA expression and (C) human hnRNP A2 mRNA. Tissue distribution of HPlγ mRNA and hnRNP A2 mRNA from within the hnRNP A2 cluster using a human multiple-tissue mRNA dot-blot: each segment is loaded with a given amount of poly(A)⁺ RNA (A, shown in ng below each tissue). The blot was hybridised with (B) a 717nt PCR fragment from the HPlγ cDNA sequence and with (C) a 1237nt PCR probe generated by using PCR primers 5' GCTGAAGCGACTGAGTCCATG 3' and 5' CCAATCCATTGACAAAATGGGC 3' for the expression of hnRNP A2.

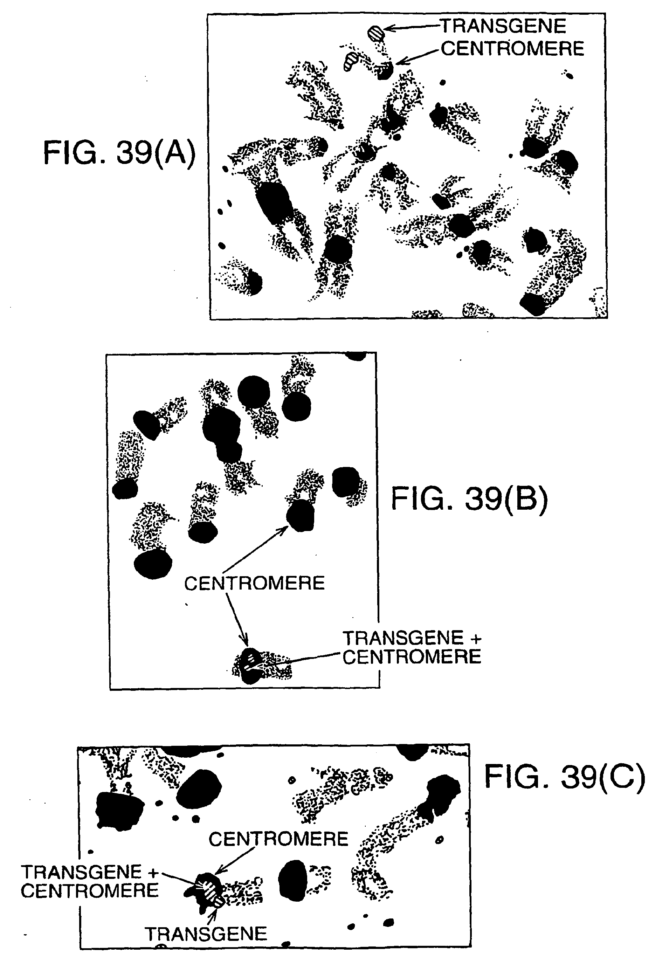

Figure 39 shows the results of the FISH analysis of TBP transgene integrated into mouse Ltk cells demonstrating integration onto centromeric heterochromatin . (A) shows a non-centromeric integration, (B) and (C) show two separate centromeric integrations.

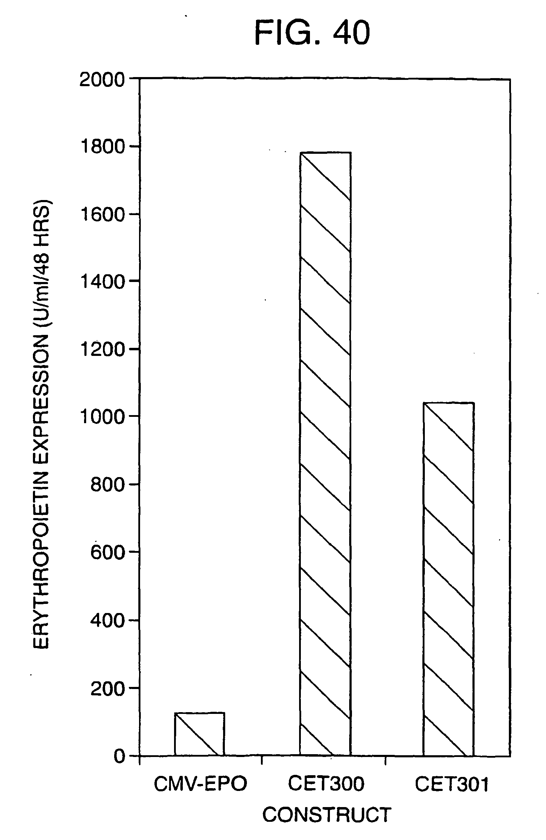

Figure 40 shows erythropoietin (EPO) expression in CHO cell pools stably transfected with CET300 and CET301 constructs comprising the 7.5kb sub-fragment located in the dual promoter regions between RNP and HPlH-y, the CMV promoter and the gene encoding EPO.

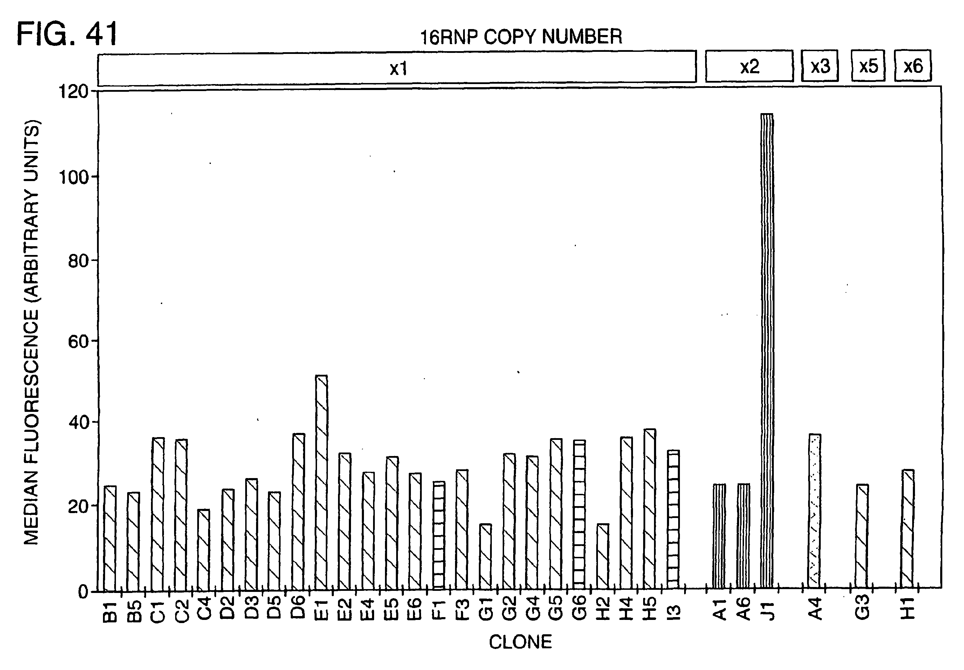

Figure 41 shows fluorescent EGFP expression of mouse Ltk cell clones transfected with 16RNP-EGFP and its relationship to copy number. Clones F1, G6 and 13 have 16RNP-EGFP colocalised with the murine centromeric heterochromatin.

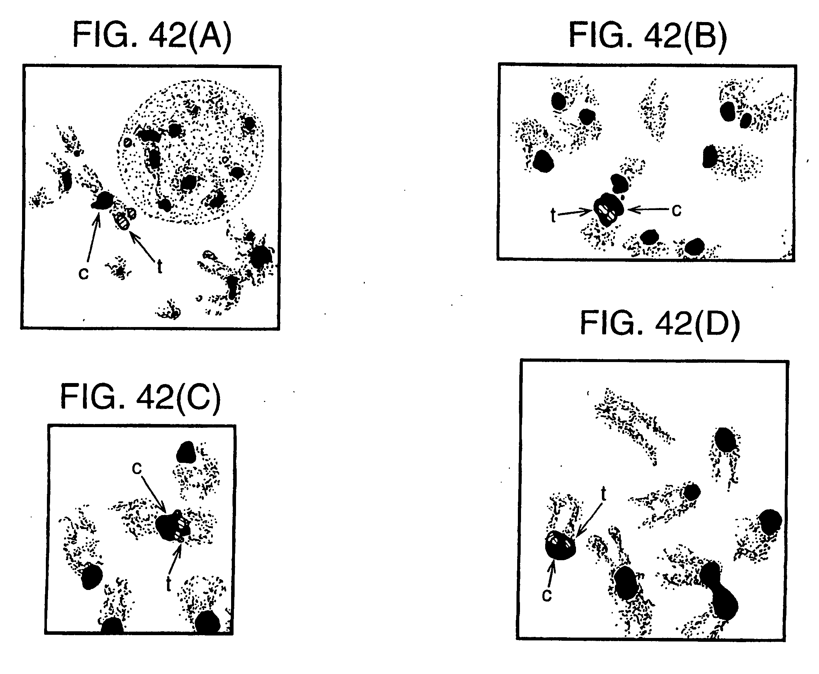

Figure 42 shows the FISH analysis of mouse Ltk cells transfected with 16RNP-EGFP. (A) shows clone H4 having a non-centromeric integration. (B, C, & D) show clones G6, F1 and 13 having centromeric integration, respectively. t is the 16RNP-EGFP and c is the mouse centromere.

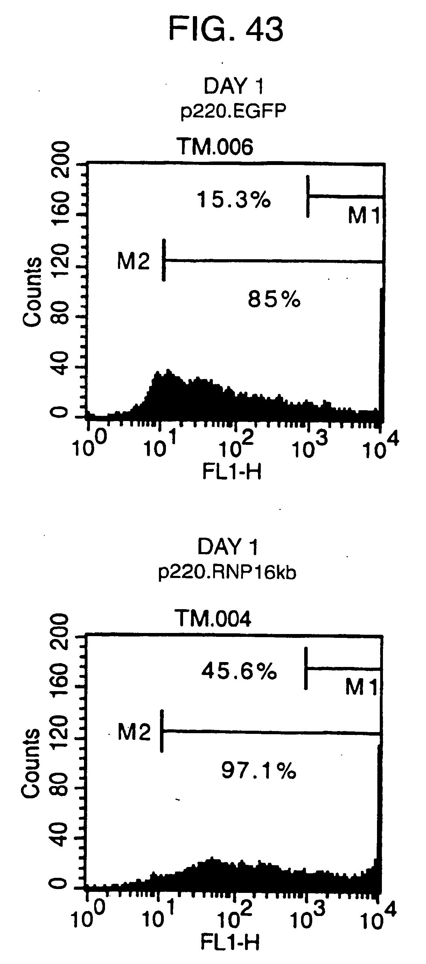

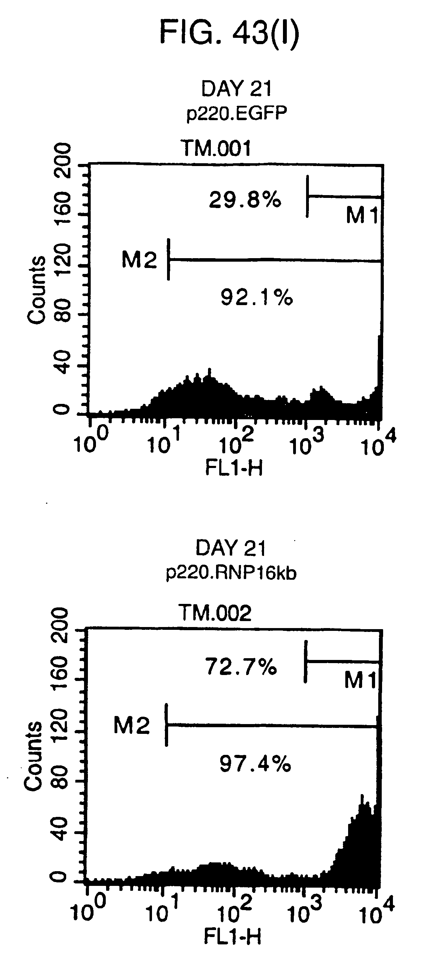

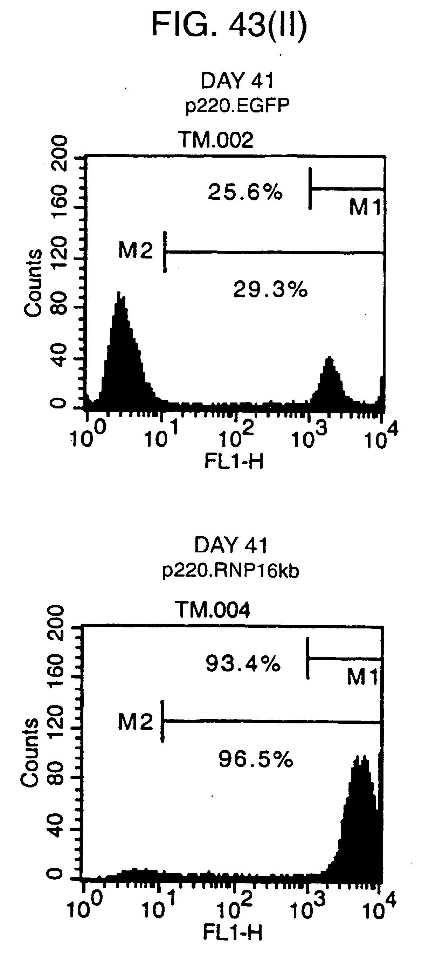

Figure 43 shows FACS profiles of EGFP expression of Hela cells transfected with EBV comprising 16RNP cultured in the presence of Hygromycin B over a period of 41 days.

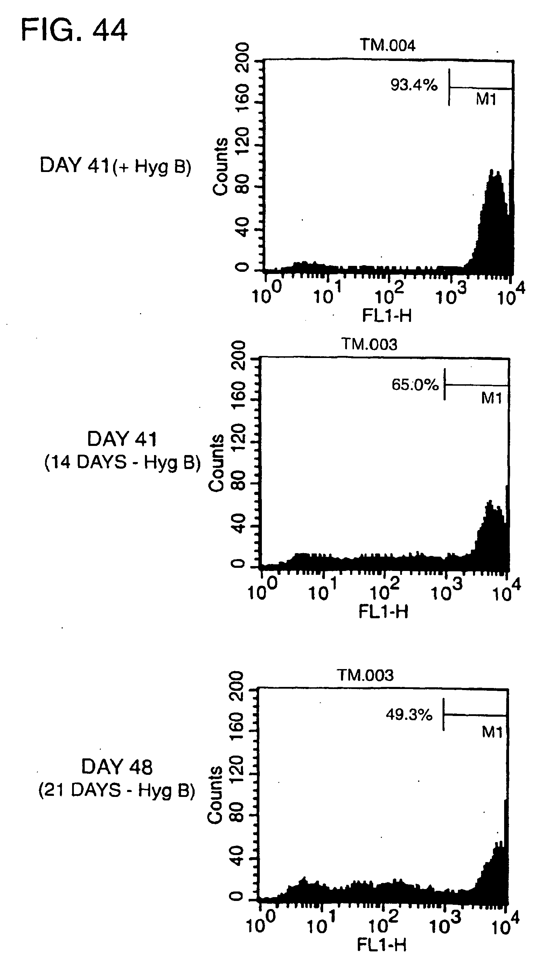

Figure 44 shows FACS profiles of EGFP expression of Hela cells transfected with EBV comprising 16RNP cultured in the presence of Hygromycin B throughout and when Hygromycin B is removed from day 27.

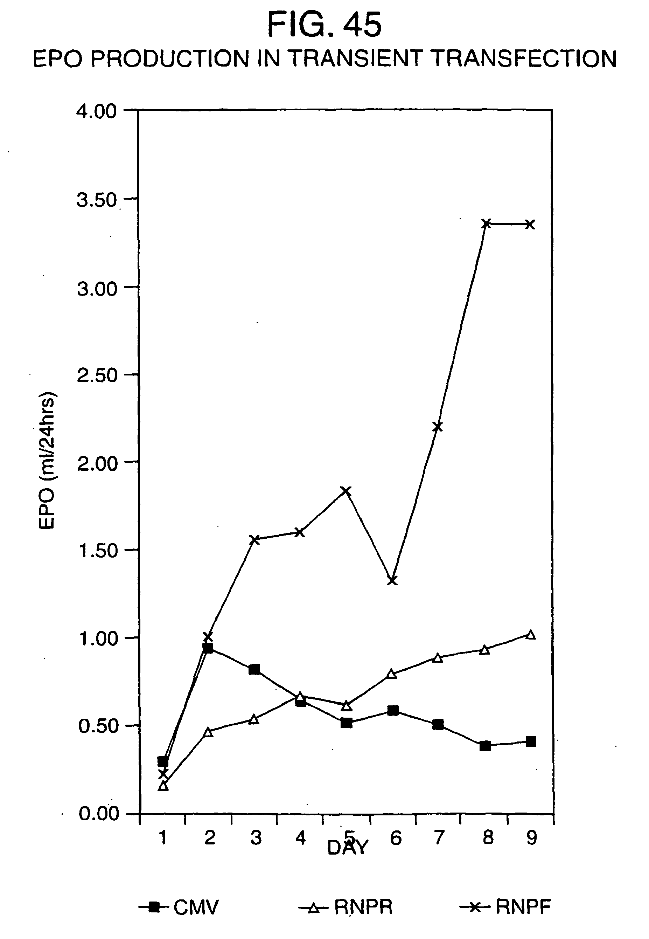

Figure 45 shows EPO production in cells transiently transfected with CET300, CET301 and CMV-EPO.

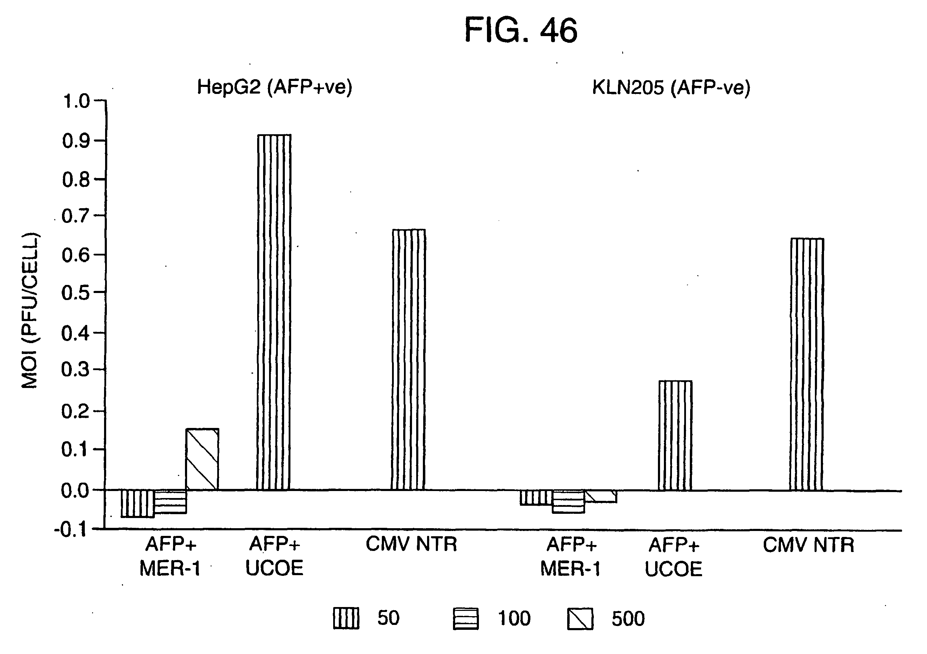

Figure 46 shows results of ELISA detecting NTR expression for various AFP constructs in HepG2 (AFP+ve) and KLN205 (AFP-ve) cells.

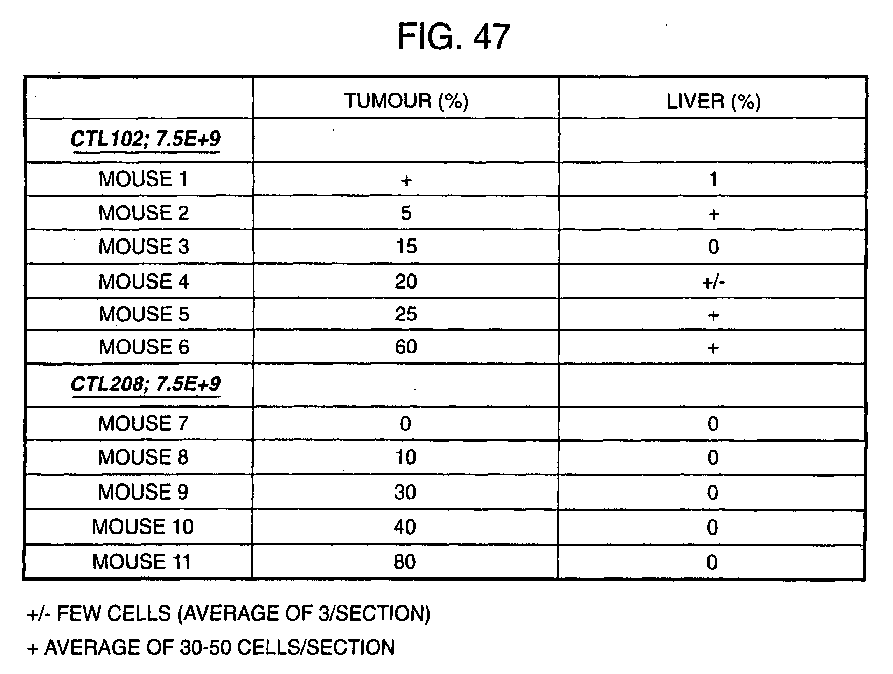

Figure 47 shows NTR expression in HepG2 tumours and host mouse livers following intratumoural injection with CTL102/CTL208.

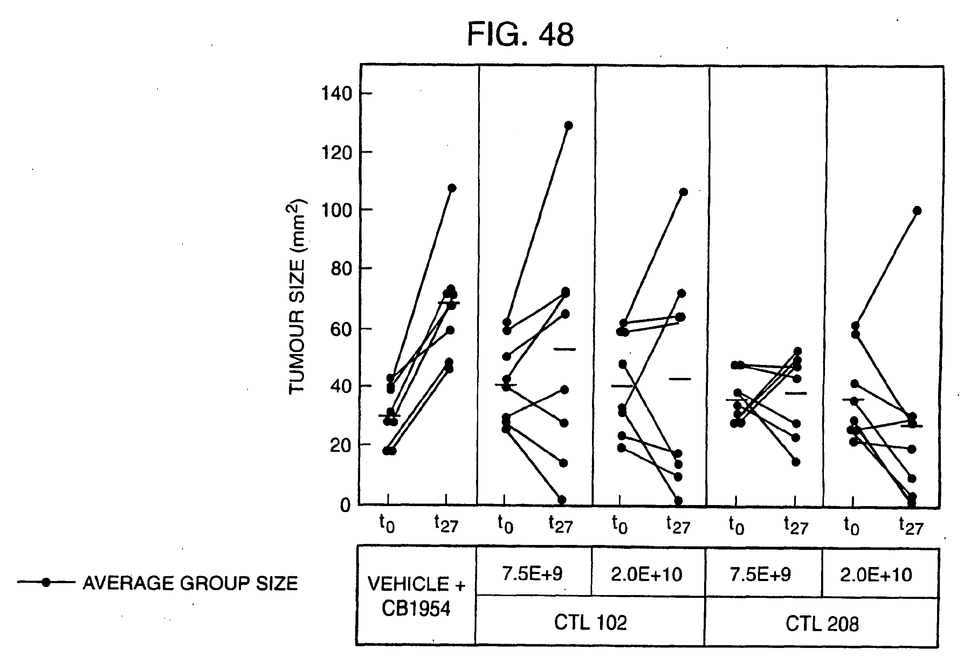

Figure 48 shows growth inhibition of HepG2 tumours following intratumoural injection with CTL 102/CTL208 and CB 1954 adminstration.
Figure 49 shows schematically the structure of vectors CET200 and CET210.

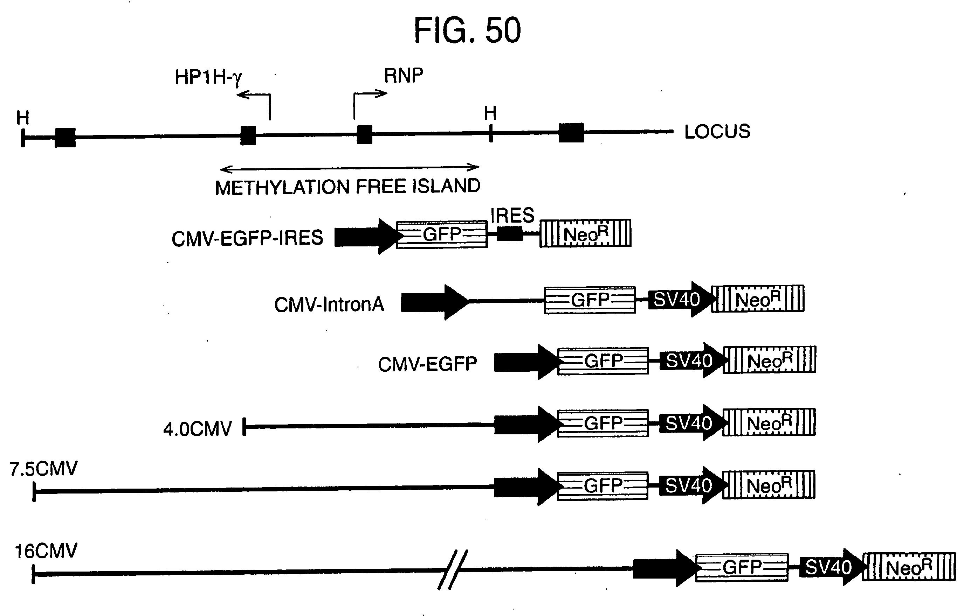

Figure 50 shows the constructs generated and fragments used in comparison to the hnRNP A2 endogenous genomic locus.

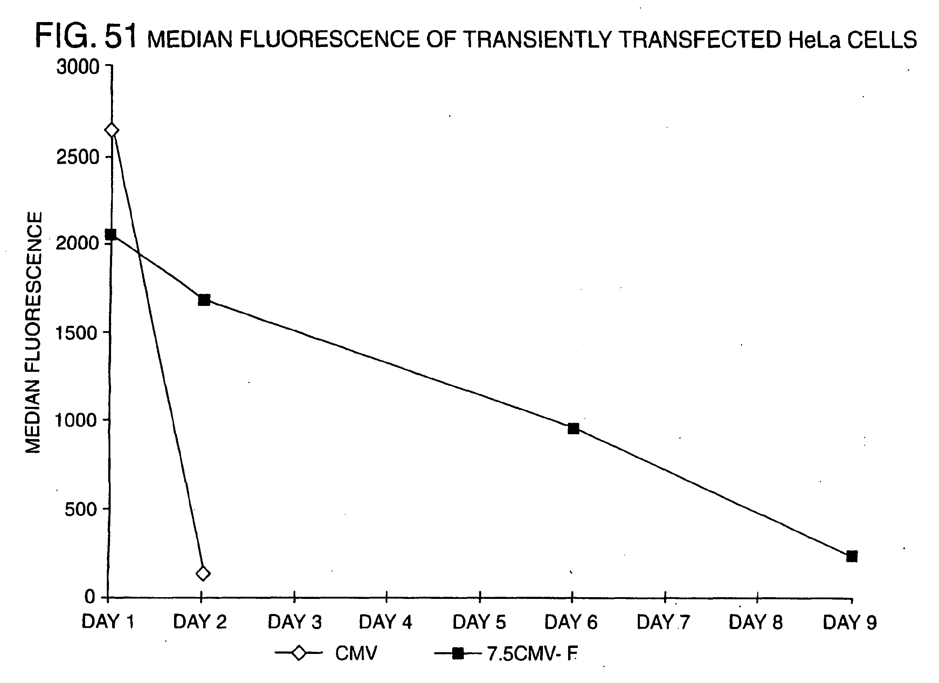

Figure 51 shows a graph of the FACs analysis with median fluorescence of HeLa populations transiently transfected with non-replicating plasmid.

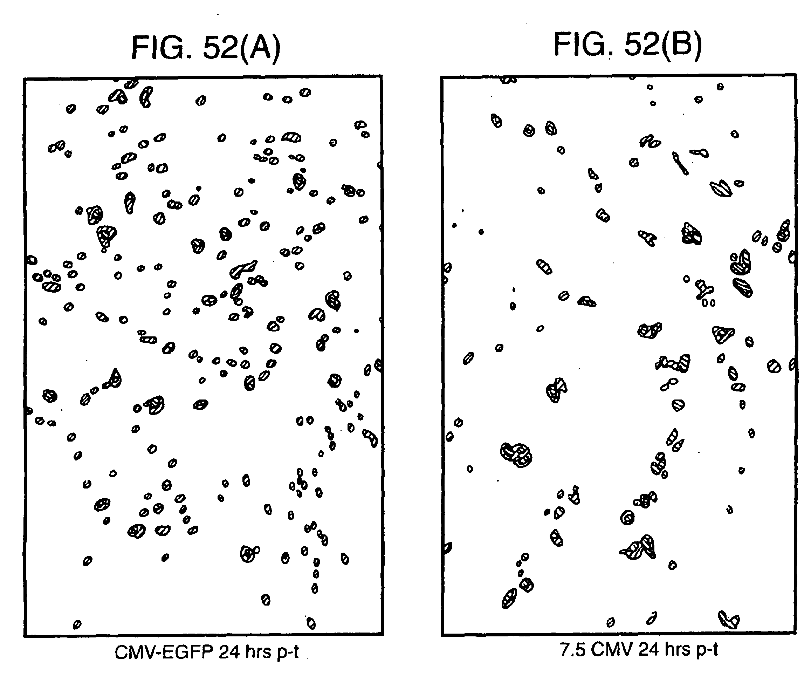

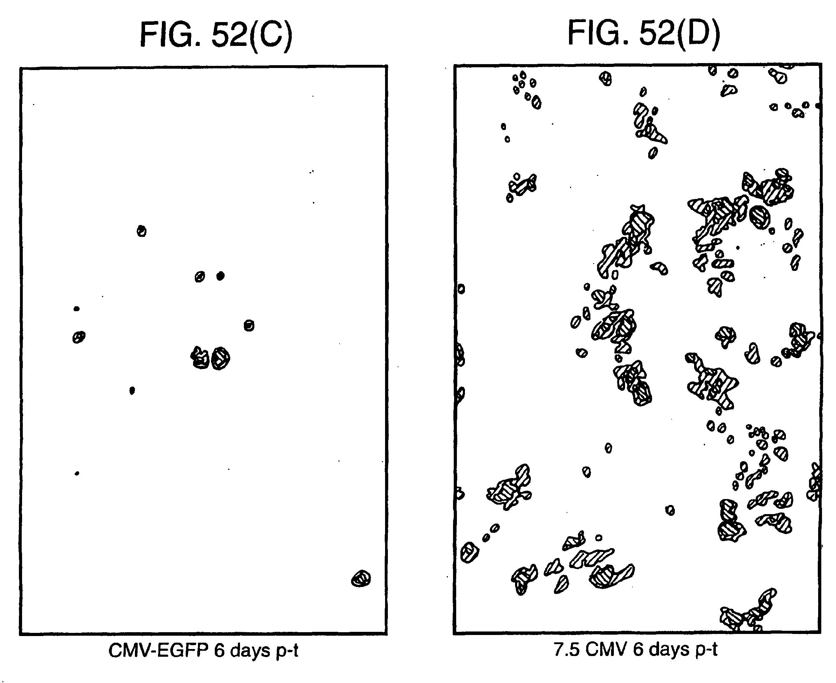

Figure 52 shows representative low magnification field of views of HeLa cell populations transiently transfected with non-replicating plasmid.

### EXAMPLES

### Materials and Methods

### Library screening

Genomic clones spanning the human TBP and hnRNPA2 loci were isolated from a P1-derived artificial chromosome (pCYPAC-2) library (CING-1; Ioannou *et al.,* 1994). Screening was by polymerase chain reaction (PCR) of bacterial lysates.

### Primers for TBP

Primers were designed using the partial genomic sequence described by Chalut *et al.* (1995) and were as follows:
TB3 [5'ATGTGACAACAGTGCATGAACTGGGAGTGG3'] (-605) and TB4 [5'CACTTCCTGTGTTTCCATAGGTAAGGAGGG3'] (-119) hybridise to the 5' untranslated region (5'UTR) of the TBP gene and give rise to a 486bp PCR product from the human gene only (see results). The numbers in parenthesis are with respect to the mRNA CAP site defined by *Peterson et al.,* (1990).
TB5 (5'GGTGGTGTTGTGAGAAGATGGATGTTGAGG3'] (1343) and TB6 [5'GCAATACTGGAGAGGTGGAATGTGTCTGGC3'] (1785) amplify a region from the 3'UTR and produce a 415bp product from both human and mouse DNA due to significant sequence homology in this region. The numbers in parenthesis are with respect to the cDNA sequence defined by Peterson *et al.,* (1990).

### Primers for hnRNP A2

Primers for hnRNP A2 were designed from the genomic sequence described by Biamonti *et al*., (1994).

Hn1 [5' ATTTCAAACTGCGCGACGTTTCTCACCGC3'] (-309) and
Hn2 [5' CATTGATTTCAAACCCGTTACCTCC3'] (199) in the 5' UTR to give a PCR product of 508bp. Hn3 [5'GGAAACTTTGGTGGTAGCAGGAACATGG3'] (7568) AND Hn4 [5' ATCCATCCAGTCTTTTAAACAAGCAG 3'] (8176) amplify a region in the penultimate exon (number 10) to give a PCR product of 607bp. The numbers in parentheses are with respect to the transcription start point defined by Biamonti *et al.* (1994).

### PCR protocol

PCR was carried out using 1 µl pooled clone material in a reaction containing 25mM each dATP, dGTP, dCTP, dTTP, 1 X reaction buffer (50mM Tris-HCI [pH 9.1], 16mM (NH₄)₂SO₄, 3.5mM MgCl₂, 150µg/ml bovine serum albumin), 2.5 units Taq Supreme polymerase (Fermentas) and 1 µM each primer in a total reaction volume of 25µl. Cycling conditions were: 4 cycles of 94°C for 1 minute, 62°C for 1 minute, 72°C for 1 minute, followed by 30 cycles of 94°C for 1 minute, 58°C for 1 minute, 72°C for 1 minute. Positively identified clones were grown in T-Broth (12g tryptone, 24g yeast extract (both Difco), 23.1g KH₂PO₄, 125.4g K₂HPO₄, 0.4% glycerol per 1 litre distilled water; Tartof and Hobbs, 1987) containing 30 µg/ml kanamycin. Permanent stocks of the bacteria were prepared by freezing individual suspensions in 1X storage buffer (3.6 mM K₂HPO₄ 1.3 mM KH₂PO₄, 2.0 mM sodium citrate, 1 mM MgSO₄ 4.4% glycerol) at -80°C.

### CYPAC-2 DNA isolation

Plasmid DNA was isolated using a modified alkaline lysis method (Bimboim and Dolly, 1979), as follows. Baffled 2 litre glass flasks containing .1 litre T-broth were inoculated with a single bacterial colony and incubated at 37°C for 16 hours with constant agitation. Bacteria were harvested by centrifugation in a Beckman J6 centrifuge at 4200 rpm (5020xg, similarly for all subsequent steps) for 10 minutes. Pellets were vortexed, resuspended in 15mM Tris-HCl [pH 8.0], 10mM EDTA, 10 µg/ml RNaseA (200 ml) and incubated at room temperature for 15 minutes. Lysis solution (0.2M NaOH, 1% SDS; 200ml) was added with gentle mixing for 2 minutes, followed by the addition of 200ml neutralisation solution (3M potassium acetate [pH 5.5]) with gentle mixing for a further 5 minutes. Bacterial debris was allowed to precipitate for 1 hour at 4°C and then removed by centrifugation for 15 minutes and filtration of the supernatant through sterile gauze. Isopropanol (400ml; 40% final concentration) was added to precipitate the plasmid DNA at room temperature for 1 hour. After centrifugation for 15 minutes and washing of the pellet in 70% ethanol, the DNA was re-suspended in a 4 ml solution of 1X TNE (50mM Tris-HCl [pH 7.5], 5mM EDTA, 100mM NaCl), 0.1% SDS and 0.5mg/ml Proteinase-K (Cambio) to remove residual proteins. Following incubation at 55°C for I hour and subsequent phenol:chloroform (1:1 v/v) extraction, the DNA was precipitated with 1 volume of 100% ethanol or isopropanol and spooled into 2ml TE buffer (10mM Tris-HCl [pH8.0], 1mM EDTA). Yields of 50µg/ml were routinely obtained.

### Restriction enzyme mapping

Restriction enzyme mapping was carried out by hybridising oligonucleotides derived from both pCYPAC-2 and TBP gene sequences to Southern blots (Southern, 1975) of restriction enzyme digested cloned DNA as described above. Oligonucleotides which hybridise to pCYPAC-2 sequences just proximal to the *Bam*HI site into which genomic fragments are cloned were used, the sequences of which were:
EY2: [5'-TGCGGCCGCTAATACGACTCACTATAGG-3']
189: [5'-GGCCAGGCGGCCGCCAGGCCTACCCACTAGTCAATTCGGGA-3']

Excision of any genomic insert from pCYPAC-2 with *Not*I means that the released fragment will retain a small amount of plasmid sequence on each side. On the EY2 side this will be 30 bp with the majority of the EY2 sequence within the excised fragment. Hybridisation of this oligonucleotide to *Not*I digested pCYPAC-2 clones should therefore, highlight the released genomic band on Southern blot analysis. At the 189 side, the excised fragment will contain 39 bp of plasmid sequence and the majority of the 189 oligonucleotide sequence is 3' to the *Not*I site, within pCYPAC-2. Therefore, this oligonucleotide will hybridise to the vector on *Not*I digests of pCYPAC-2 clones. Approximately 100ng plasmid DNA was subjected to restriction endonuclease digestion using manufacturers recommended conditions (Fermentas), and subsequently electrophoresed on 0.7% agarose gels in 0.5 X TAE buffer (20mM Tris-Acetate [pH 8.0], 1mM EDTA, 0.5µg/ml ethidium bromide) or on pulsed field gels. Pulsed Field Gel Electrophoresis (PFGE) was carried out on a CHEF-DRII system (Biorad) on 1% PFGE agarose (FMC) / 0.5X TAE gels at 6V/cm for 14 hours with switch times from 1 second to 30 seconds. Identical conditions were used for all PFGE analysis throughout this study. Gels were stained in 1 µg/ml ethidium bromide solution before being photographed under ultraviolet light.

In preparation for Southern blot analysis, the DNA was depurinated by first exposing the agarose gels to 254nm ultraviolet light (180,000µJ/cm² in a UVP crosslinker, UVP) and then subsequently denaturing by soaking in 0.5M NaOH, 1.5M NaCl for 40 minutes with a change of solution after 20 minutes. The DNA was transferred to HYBOND-N nylon membrane (Amersham) by capillary action in a fresh volume of denaturation solution for 16 hours. Crosslinking of the nucleic acids to the nylon was achieved by exposure to 254nm ultraviolet light at 120,000µJ/cm². Membranes were neutralised in 0.5M Tris-HCl [pH 7.5], 1.5M NaCl for 20 minutes and rinsed in 2X SSC before use. (1X SSC is 150mM NaCl, 15mM sodium citrate, [pH7.0]).

Oligonucleotide probes were 5'end labelled with T4 polynucleotide kinase and ³²P-γATP to enable detection of specific fragments on Southern blots. Each experiment employed 100ng of oligonucleotide labelled in a reaction containing 2µl ³²P-γATP (>4000 Ci/mmol; 10 mCi/ml, Amersham) and 10 units T4 polynucleotide kinase (Fermentas) in the manufacturers specified buffer. After incubation at 37°C for 2 hours, unincorporated nucleotides were removed by chromatography on Sephadex G50 columns (Pharmacia) equilibrated with water. End-labelled probes were typically labelled to a specific activity >1 x 10⁸ dpm_{/}µg.

Hybridisation was carried with membranes sandwiched between nylon meshes inside glass bottles (Hybaid) containing 25ml pre-warmed hybridisation mix (1mM EDTA [pH8.0], 0.25M Na₂HPO₄ [pH 7.2], 7% SDS; Church and Gilbert, 1984) and 100µg/ml denatured sheared salmon testis DNA. After pre-hybridisation at 65°C for 1 hour, the solution was decanted and replaced with an identical solution containing the labelled probe. Optimal hybridisation temperature was determined experimentally and found to be 20°C below the Tₘ for the oligonucleotide in TE buffer, calculated as Tₘ = 59.9 + 41 [%GC] - [675 / primer length]). After 16 hours hybridisation membranes were removed and washed with three, 2 minutes washes of 6 X SSC, 0.1% SDS followed by exposure to x-ray film (BioMAX, Kodak).

### DNA constructs

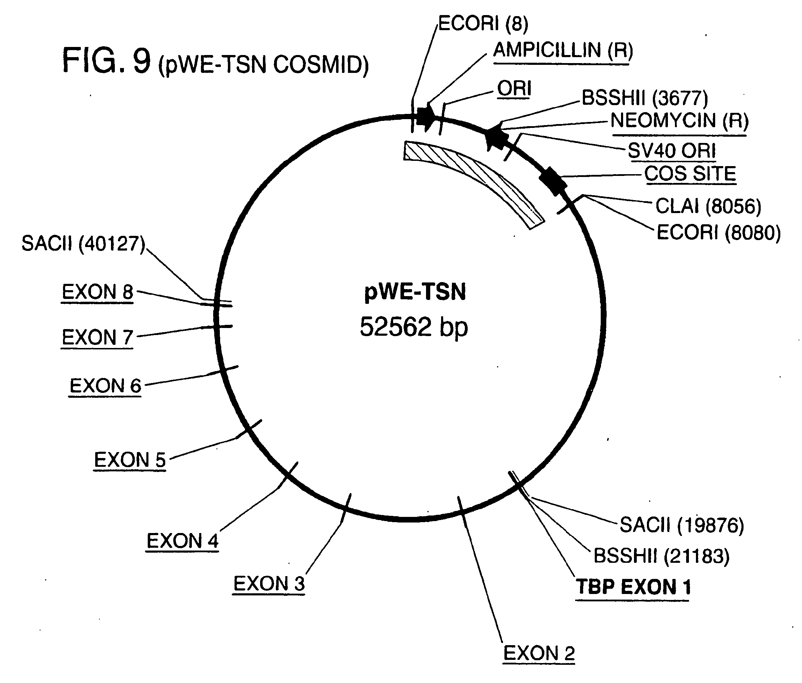

A 44kb genomic DNA region spanning the TBP gene with 12kb of both 5' and 3' flanking sequences, was derived from the pCP2-TNN pCYPAC-2 clone (see Figure 9) as a *Not*I fragment. This was cloned into the cosmid vector pWE15 (Clontech) to generate pWE-TSN (Figure 9). The vector exchange was necessary as the pCYPAC-2 plasmid does not contain a selectable marker for eukaryotic cell transfection studies. Digestion of pCP2-TNN with *NotI* liberates a 44kb fragment extending from the 5' end of the genomic insert to the *Not*I site present in the genomic sequence located 12 kb downstream of the last exon of TBP (see Figure 9). In addition, fragments containing the remaining 20kb of 3' flanking sequence in this clone and the pCYPAC-2 vector are produced. The ligation reaction was performed using approximately 1 µg of *Not*I digested pCP2-TNN and 200ng similarly cut pWE15 in a 10µl reaction using conditions as described above. After heat inactivation of the T4 DNA ligase, the complete ligation mix was packaged into infectious lambda 'phage particles with Gigapack Gold III (Stratagene). Recombinant bacteriophage were stored in SM buffer (500µl of 50mM Tris-HCl, 100mM NaCl, 8mM MgSO₄, 0.01 % (w/v) gelatine, 2% chloroform). Infection was carried out as follows: 5ml of an overnight culture of *E. coli* DH5α was centrifuged (3000 x g, 5 minutes) and the bacteria resuspended in 2.5ml of 10mM MHCl₂. Equal volumes of packaged material and *E. coli* were mixed and incubated at 25°C for 15 minutes after which time 200µl L-broth was added and the mixture incubated at 37°C for a further 45 minutes. The suspension was plated on LB-ampicillin agar plates and single colonies analysed as mini preparations the following day. Large amounts of pWE-TSN were prepared from 1 litre cultures as for pCYPAC-2 clones.

### pCYPAC-2 DNA sub-cloning methods

The following procedure was used in order to sub-clone small (less than 10kb) restriction enzyme fragments derived from pCYPAC-2 clones. DNA was restriction enzyme digested and electrophoresed on 0.6% low melting point agarose gels (FMC) with all ultraviolet photography carried out at a wavelength of 365 nm to minimise nicking of ethidium bromide stained DNA (Hartman, 1991). The gel area containing fragments of the desired range of sizes was excised from the gel, melted at 68°C for 10 minutes and allowed to equilibrate to 37°C for a further 5 minutes. The plasmid vector pBluescriptKS(+) (Stratagene) was similarly restriction enzyme digested to give compatible termini with the pCYPAC-2 derived DNA, treated with 10 units calf intestinal phosphatase (Fermentas) for 1 hour to minimise self-ligation and purified by phenol:chloroform (1:1 v/v) extraction followed by ethanol precipitation. Molten gel slices were mixed with 50ng of this vector preparation giving a molar excess of 4:1 fragment to vector molecules. T4 DNA Ligase (10 units; Fermentas) was added along with the specified buffer and the mixture incubated at 16°C for 16 hours after which time the enzyme was heat inactivated (65°C for 20 minutes) to improve transformation efficiency (Michelsen, 1995). Preparation of calcium chloride competent DH5α *E. coli* and subsequent transformation was performed using established procedures (Sambrook *et al.,* 1989). Transformation was achieved by melting and equilibrating the ligation mixture to 37°C before the addition of 100µl competent cells maintaining a final agarose concentration of no more than 0.02%. Bacteria were incubated on ice for 2 hours followed by heat shock at 37°C for 5 minutes and subsequent addition of 1 ml SOC media (20g tryptone; 5g yeast extract; 0.5g NaCl; 20mM glucose, [pH 7.0] per 1 litre distilled water; Sambrook *et al.,* 1989). After a further hour at 37°C, cells were mixed with 50µl selection solution (36mg/ml Xgal, 0.1 M IPTG) and plated on the appropriate LB-antibiotic plates (10g NaCl [pH 7.0], 10g tryptone, 5g yeast extract, 20g agar per litre distilled water) containing 20 µg/ml ampicillin. After incubation at 37°C for 16 hours, bacterial colonies containing recombinant plasmids were identified by their white (as opposed to blue) colour due to disruption of β-galactosidase gene activity. Selected colonies were analysed by restriction digestion of DNA isolated from single colony mini preparations. Using this procedure it was possible to sub-clone fragments of up to 20kb in size into the pBluescriptKS(+) vector.

PCR amplified products were cloned using the following procedure. After a standard PCR reaction using 1 ng of the pCYPAC-2 derived clone DNA as a template in a 50µl volume, 10 units T4 DNA polymerase (Fermentas) were added to the reaction and incubated for 30 minutes at 37°C. After inactivation of the polymerase enzyme (96°C, 20 minutes), 7µl of the PCR product were ligated to 50ng *Eco*RV digested pBluescriptKS(+) vector in a final volume of 10µl. Use of the T4 DNA polymerase, to blunt the ends of the PCR products resulted in a high proportion of recombinant clones (data not shown).

### Generation of pBL3-TPO-puro

pBL3-TPO-puro contains the entire 19 kb TBP gene with approximately 1.2 kb 5' and 4.5 kb 3' flanking sequences and a puromycin resistance gene cassette, sub-cloned into the pBL3 vector. This was achieved by 3 consecutive cloning steps.

Firstly, the 4.5 kb of sequence flanking the 3' end of the human TBP gene in the pCP2-TLN plasmid was sub-cloned from pCP2-TLN as a NotI - SacII fragment. This fragment extends from the SacII site in the 3' UTR of the TBP gene to the OL189-proximal NotI site within the pCYPAC-2 vector. This fragment was cloned into SacII and NotI digested pBL3 and designated MA426. The remaining TBP gene sequences reside on a 19 kb SacII fragment extending from approximately 1.2 kb upstream of the mRNA cap site to the SacII site in the 3'- UTR. This fragment was ligated in to MA426 which was linearised with SacII, and clones screened for the correct orientation.

### DNA sequencing and computer sequence analysis

DNA was prepared using the Flexi-Prep system (Pharmacia) and automated fluorescent sequencing provided as a service from BaseClear (Netherlands). dBEST and nonredundant Genbank databases were queried using previously described search tools (Altschul *et al.,* 1997). All expressed sequence tag clones used in this study were obtained through the I.M.A.G.E. consortium (Lennon *et al.,* 1996). Multiple sequence alignments and prediction of restriction enzyme digestion patterns of known DNA sequences was performed using the program PCGENE (Intelligenetics Inc., USA). Plots of CpG di-nucleotide frequency were produced using VectorNTI software (Informax Inc., USA).

### GENERATION OF NON-HUMAN TRANSGENIC ANIMALS

### Preparation of TBP fragments for microinjection

The 90kb genomic fragment (TLN) encompassing the TBP/PSMB1 gene region was isolated by *Not*I digestion of the pCP2-TLN clone and prepared for microinjection using a modified sodium chloride gradient method (Dillon and Grosveld, 1993). Initially, bacterial lipopolysaccharide (LPS) was removed from a standard pCP2-TLN maxi preparation using an LPS removal kit (Quiagen) according to the manufacturer's instructions. Approximately 50µg of DNA was then digested for I hour with 70 units of *Not*I (Fermentas) and a small aliquot analysed by PFGE to check for complete digestion. A 14ml 5-30% sodium chloride gradient in the presence of 3mM EDTA was prepared in ultra-clear centrifuge tubes (Beckman) using a commercial gradient former (Life Technologies). The digested DNA was layered on the top of the gradient using wide-bore pipette tips to minimise shearing and the gradient centrifuged at 37,000 rpm for 5.5 hours (at 25°C) in a SW41Ti swing-out rotor (Beckman). Fractions of approximately 300µl were removed starting from the bottom of the gradient (highest density) into individual microcentrifuge tubes containing 1ml 80% ethanol followed by incubation at -20°C for 1 hour. DNA precipitates were collected by centrifugation at (14900 × g, 15 minutes). Pellets were washed in 70% ethanol, dissolved in 20µl transgenic microinjection buffer (10mM Tris-HCl [pH 7.4], 0.1mM EDTA) and 5µl aliquots from alternate fractions analysed by gel electrophoresis to asses contamination of vector and chromosomal DNA. Those fractions, which appeared to be free of such contaminants, were pooled and the DNA concentration assessed by absorbance at 260 nm.

The 40kb genomic fragment (TSN) was isolated from pWE-TSN by *Not*I digestion and purification using electro-elution as previously described (Sambrook *et al.,* 1989). After electro-elution, DNA was purified by sequential extraction with TE buffer-saturated phenol, phenol:chloroform (1:1 v/v) and twice with water saturated n-butanol to remove residual ethidium bromide. DNA was precipitated with 2 volumes of 100% ethanol and resuspended in microinjection buffer. Fragment integrity was assessed by PFGE and concentration determined by absorbance at 260nm. The 25kb genomic fragment (TPO) was isolated from pBL3-TPO using an identical procedure except the insert was liberated from the vector by digestion with *Sal*I.

### Preparation of bnRNP A2 fragments for microinjection

The 160kb genomic fragment (MA160) encompassing the hnRNP A2 gene region was isolated and prepared for microinjection by *NruI* digestion of pCP2-HLN (Figure 13A) and sodium chloride gradient ultracentrifugation as described above.

The 60kb genomic fragment (HSN; Figure 13B) was isolated from MA160 by *Aat*II digestion and purification by PFGE as described above. The 60kb band was excised from the gel and cut into slices. Each slice was melted at 65°C and 30µl analysed by PFGE. The fraction showing the purest sample of the 60kb fragment was retained. The melted gel volume was measured, made 1X with Gelase buffer, equilibrated at 42°C for 10 minutes and 1 unit Gelase enzyme (Epicentre Technologies) added per 500µl. Samples were incubated overnight at 42°C and then centrifuged for 30 minutes at 4°C. The supernatant was decanted with a wide bore tip and drop-dialysed against 15ml of transgenic microinjection buffer on a 0.25µm filter in a 10cm Petri dish for 4 hours. The dialysed solution was transferred into a microcentrifuge tube and spun for 30 minutes at 4°C. Fragment integrity was assessed by PFGE and concentration determined by absorbance at 260nm.

### Generation of transgenic mice

Transgenic mice were produced by pronuclear injection of fertilised eggs of C57/B16 mice. Each DNA fragment was injected at a concentration of 1ng/µl in transgenic buffer. This was performed as a service by the UMDS Transgenic Unit (St Thomas's Hospital, London) using standard technology. Transgenic founders were identified using PCR screening of tail biopsy DNA isolated as follows. Approximately 0.5cm tail biopsies from 10-15 day old mice were incubated at 37°C for 16 hours in 500µl tail buffer (50mM Tris-HCl [pH 8.0], 0.1M EDTA, 0.1M NaCl, 1% SDS, 0.5 mg/ml Proteinase-K). The hydrosylate was extracted by gentle inversion with an equal volume phenol:chloroform (1:1 v/v) followed by centrifugation (14900 x g, 15 minutes). The DNA was precipitated from the aqueous phase by the addition of 2 volumes of 100% ethanol and washed in 70% ethanol. DNA was spooled and dissolved in 100µl TE buffer. Typically, 50-200µg DNA was obtained as determined by absorbance measurements at 260nm. The conditions for the PCR reactions were as described for the screening of the pCYPAC-2 library using 100ng tail biopsy DNA as template and the TB3/TB4 primer set. Positive founders were bred by back-crossing to wild-type C57/B16 mice to generate fully transgenic F1 offspring.

### Transgene integrity and copy number

Transgene copy number and integrity was assessed by Southern blot analysis of *Bam*HI*, Bgl*II, *Eco*RI, and *Hind*III digested tail biopsy DNA. Approximately 10µg DNA was digested with 20-30 units of the specific restriction endonuclease and electrophoresed on 0.7% agarose/0.5X TBE (45mM Tris-borate, [pH 8.0], 1mM EDTA,) gels for 16 hours at 1.5V/cm. Staining and transfer of DNA onto nylon membranes was as for plasmid Southern blots except a positively charged matrix (HYBOND N+, Amersham) was used.

DNA probes were prepared by restriction enzyme digestion to remove any cloning vector sequences and purified from low-melting point agarose using the Gene-Clean system (Bio101, USA). Radioactive labelling of 100ng samples of the probes was performed by nick translation using a commercially available kit (Amersham) and 200 pmol each of dCTP, dGTP, dTTP and 3µl α-P³²-dATP (specific activity >3000 Ci/mmol, 10mCi/ml, Amersham). The enzyme solution consisting of 0.5 units DNA polymerase I/10pg DNase I in a standard buffer, was added and the reaction incubated at 15°C for 2.5 hours. Probes were purified by Sephadex G-50 chromatography and boiled for 5 min immediately prior to their use. Typically, specific activities of >1 × 10⁸ cpm/µg were obtained.

Hybridisation was performed as for plasmid Southern blots described above. Membranes were incubated in 15ml pre-hybridisation solution (3X SSC, 0.1% SDS, 5X Denhardt's solution [100X Denhardt's solution is 2% Ficoll (Type 400, Pharmacia), 2% polyvinyl pyrollidone, 2% bovine serum albumin (Fraction V, Sigma) per litre distilled water]), containing 100µg/ml denatured salmon testis DNA at 65°C for 1 hour. The solution was then replaced by 15ml hybridisation solution (as pre-hybridisation solution with the addition of dextran sulphate to 10%) containing 100µg/ml denatured salmon testis DNA and the heat denatured radio-labelled probe. After hybridisation at 65°C for 16 hours membranes were washed three times in 2X SSC/0.1% SDS for 30 minutes each and exposed to PhosphorImager (Molecular Dynamics) screens or x-ray film at -80°C. Those blots which were to be re-analysed, bound probe was removed by soaking in 0.2M NaOH for 20 minutes followed by neutralisation as described above.

The majority of the probes used in this study were derived from regions of the genomic clones where no sequence information was available (e.g. pCP2-TLN end-fragment probes and those derived from the TBP intronic regions). A number of probes hybridised non-specifically to human genomic DNA suggesting the presence of repetitive sequence elements. In order to circumvent this problem, aliquots of probe DNA were individually digested with a number of restriction enzymes, electrophoresed and Southern blotted. Enzymes with short recognition sites (which should occur very frequently within the DNA), were chosen so as to digest the probe into a number of smaller fragments. Radiolabelled human C₀t-1 DNA was used as a probe to indicate those fragments that contained repetitive sequences. Using this procedure, it was possible to obtain fragments >500 bp that did not hybridise to the C₀t-1 probe, for all probes which contained repetitive elements.

### Preparation of cosmid DNA and generation of single copy L-cell clones

pWE-TSN DNA was prepared by alkaline lysis of 1 litre cultures as described above until the isopropanol precipitation stage. After incubation at 25°C for 1 hour, the pellet was resuspended in 300µl TE and then added with continuous mixing to 10ml Sephaglas FP DNA binding matrix (Pharmacia). The solution was constantly inverted for 10 minutes and the martix-bound DNA collected by centrifugation (280 x g, 1 minute). The pellet was washed firstly with WS buffer (20 mM Tris-HCl [pH 7.5], 2mM EDTA, 60% ethanol), collected by centrifugation, washed with 70% ethanol and re-centrifuged. DNA was eluted from the matrix by resuspending the pellet in 2ml TE buffer and incubation at 70°C for 10 minutes with periodic mixing. The solution was centrifuged (1100 x g, 2 minutes) and the DNA containing supernatant split equally into two microfuge tubes. Residual Sephaglass was removed by centrifugation (14950 x g, 15 minutes), the supernatants pooled and DNA precipitated with 2 volumes of ethanol. The spooled DNA was washed once in 70% ethanol and resuspended at 1µg/µl in sterile water. Approximately 75-100µg of pure cosmid DNA was obtained using this procedure, which represents a yield of 60-80% of DNA obtained without Sephaglas purification.

Transfection of adherent mouse L-cells (Earle *et al*., 1943) was performed as follows. Approximately 1 x 10⁷ cells grown in DMEM containing 10% heat inactivated foetal calf serum (PAA laboratories), 2 mM L-glutamine, were mixed with 1 µg pWE-TSN DNA linearised with *Sal*I and incubated on ice for 10 minutes. DNA was introduced into the cells by electroporation (Chu *et al.,* 1987) with settings of 960µF, 250V in a Biorad Gene-Pulser. Transfected cells were selected for and maintained in the same medium including 400µg/ml geneticin sulphate (G418; Life Technologies Inc.). Individual clones were isolated using cloning rings (Freshney, 1994). Thick-walled stainless steel cloning rings (Life Technologies Inc.) were autoclaved in silicon grease and transferred to the tissue culture plate such that the colony was isolated. A solution of trypsin (300µl of 0.25% trypsin [pH 7.6] (Difco), 0.25M Tris-HCl [pH 8.0], 0.4% EDTA [pH 7.6], 0.12M NaCl, 5mM glucose, 2.4mM KH₂PO₄ 0.84mM Na₂HPO₄.12H₂O, 1% phenol red) was added and the plate incubated at 37°C for 5 minutes. Cells were transferred to 24 well plates and clonal cell lines established. Clones were preserved as follows. Approximately 1 x 10⁷ cells were harvested by centrifugation, resuspended in 0.75 ml freezing mix (70% standard growth media but including 20% foetal calf serum and 10% DMSO) and snap frozen on dry ice for 1 hour before transfer to liquid nitrogen storage.

Genomic DNA was prepared from these L-cell clones using standard procedures (Sambrook *et al.,* 1989). Cells in T75 flasks were grown to confluency (approximately 4 x 10⁷), the media removed and the flask washed with PBS (2.68mM KCl, 1.47mM KH₂PO₄, 0.51 mM MgCl₂, 136.89mM NaCl, 8.1mM Na₂HPO₄ [pH 7.3]) and 2ml lysis buffer (10mM Tris-HCl [pH 7.5], 10mM EDTA, 10mM NaCl, 0.5% SDS, 1mg/ml Proteinase-K) added. Cells were dislodged from the culture flask by scraping and transferred to a 15ml centrifuge tube using a wide bore pipette tip. Lysis was allowed to proceed at 68°c for 16 hours after which the solution was extracted once with phenol:chloroform (1:1 v/v) and the DNA precipitated with an equal volume of isopropanol. After washing in 70% ethanol, the DNA was resuspended in 1 ml TE buffer and concentration assessed by absorbance at 260nm.

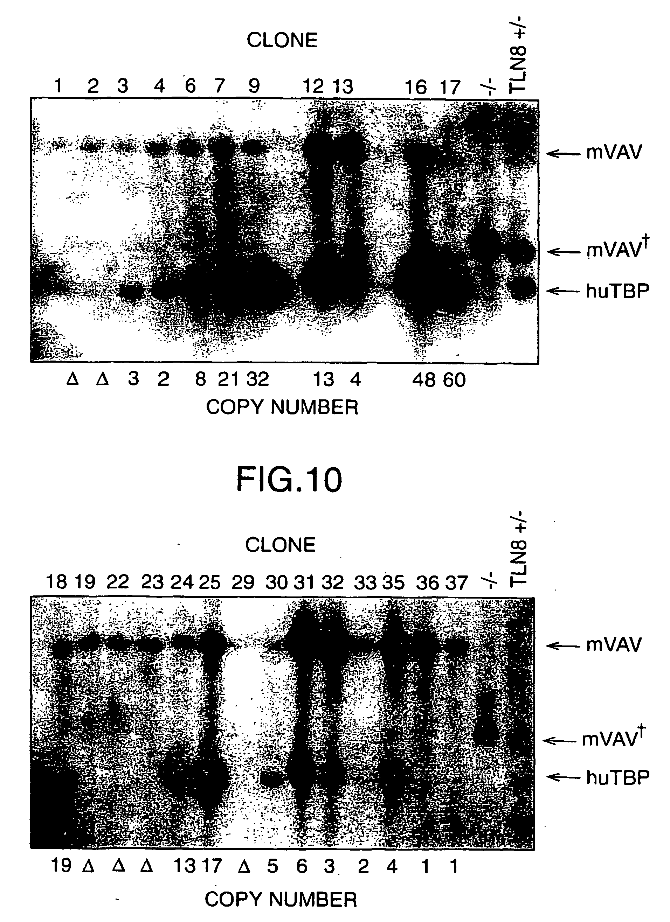

Transfected gene copy numbers were determined by Southern Blot analysis of *Bgl*II digested genomic DNA. Human TBP was detected using a specific probe (1.4HX) located in the C5 gene, 4kb 5' of the TBP transcription initiation region and which detects a 4.2kb fragment (see Figure 10). In addition, blots were simultaneously probed with a 1 kb *Nco*I fragment derived from the endogenous murine *vav* locus (Ogilvy *et al*., 1998) that gives a 5.2kb band and that acts as a single copy reference standard. Human TBP transgene copy-number was ascertained by comparing the ratio of the TBP to vav signal obtained with the 3 copy transgenic mouse line TLN:8 after analysis of blots by PhosphorImager.

Total RNA was prepared from approximately 4 x 10⁷ cells by selective precipitation in 1 ml of 3M LiCl, 6M urea (Auffrey and Rougeon, 1980; see Antoniou, 1991).

### DNase I hypersensitive site analysis

This was performed as previously described (Forrester *et al.,* 1987; Reitmann *et al.,* 1993). Nuclei were prepared from approximately 1 x 10⁹ K562 cells (Lozzio and Lozzio, 1975). Harvested cells were washed in PBS and resuspended in 4ml ice cold RSB (10mM Tris-HCl [pH7.5], 10mM NaCl, 3mM MgCl₂) and placed in a glass dounce homogeniser fitted with a loose pestle. After the addition of 1ml of 0.5% NP40/RSB the cells were homogenised slowly for 10-20 strokes and nuclei recovered by the addition of 50ml RSB and centrifugation at 4°C (640 x g, 5 minutes). The supernatant was discarded and nuclei were resuspended in 1 ml RSB with 1 mM CaCl₂. Immediately, a 100µl aliquot (representing approximately 1 x 10⁸ nuclei) was taken and DNA purified as described below, to control for endogenous nuclease activity during the isolation procedure.

The DNase I digestion was performed as follows. A range of aliquots (0, 0.5, 1, 2, 3, 4, 5, 6, 8, 10 µl) of 0.2mg/ml DNase I (Worthington) was added to individual microfuge tubes containing 100µl of nuclei and incubated at 37°C for 4 minutes. The digestion was stopped by the addition of 100µl 2X stop mix (20mM Tris-HCl [pH 8.0], 10mM EDTA, 600mM NaCl, 1%SDS), 10µl Proteinase-K (10mg/ml concentration) and incubation at 55°C for 60 minutes. DNA was purified by phenol:chloroform (1:1 v/v) extraction and ethanol precipitation. Samples were electrophoresed on 0.7% agarose/0.5X TBE gels and Southern blotted for analysis using ³²P-radiolabelled probes.

### RNA preparation

Adult mice aged 10-40 weeks were sacrificed by cervical dislocation and whole tissues isolated, snap frozen in liquid nitrogen and stored at -80°C until required. Total RNA was prepared by selective precipitation in 3M LiCl, 6M urea (Auffray and Rougeon, 1980). Tissues were transferred to 14ml tubes containing 1ml of the LiCl-urea solution and homogenised for 30 seconds with an Ultra-Turrax T25 (Janke & Kunkel). Samples were then subjected to three, 30-second pulses of sonication (Cole-Parmer Instrument Co., USA), the homogenate transferred to sterile microfuge tubes and RNA allowed to precipitate at 4°C for 16 hours. The RNA was collected by centrifugation (4°C, 14900 x g, 20 minutes) washed in 500 µl LiCl-urea solution and resuspended in 500µl TES (10mM Tris-HCl [pH 7.5], 1mM EDTA, 0.5% SDS). After extraction with phenol:chloroform, samples were made 0.3M with sodium acetate and RNA precipitated by the addition of 1 ml 100% ethanol and storage at -20°C for at least 1 hour. The RNA was collected by centrifugation and resuspended in 20µl sterile water and concentration assessed by absorbance at 260nm.

### COMPETITIVE RT-PCR BASED ASSAY

### Analysis of human TBP expression

A modified competitive RT-PCR approach (Gilliland *et al*., 1990) was used to accurately quantify human TBP and PSMB 1 gene expression in a mouse background. Total RNA (1µg) from transgenic mouse tissues or cell lines was reversed transcribed in a 25µl reaction consisting of 10 units Avian Mycloblastosis Virus (AMV) reverse transcriptase (Promega), 10mM DTT, 2.5mM each dNTP, 25 units ribonuclease inhibitor (Fermentas) with 1 µM reverse primer (TB14 or C5R) in 1X RT buffer (25mM Tris-HCl [pH 8.3], 25mM KCl, 5mM MgCl2, 5mM DTT, 0.25mM spermidine). Synthesis of cDNA was allowed to proceed at 42°C for 1 hour followed by a further hour at 52°C and heat inactivation of the enzyme at 95°C for 5 minutes. PCR reactions contained 1 µl cDNA amplified using the reaction mix described for tail biopsy screening and containing specific primer sets for the sequence in question (as detailed above, one of which was end-labelled using the protocol described above. Primers were purified with two rounds of Sephadex-G25 chromatography (Pharmacia) and an 80% recovery was assumed. PCR conditions were 94°C for 1 minute, 58°C for 1 minute and 72°C for 1 minute with cycle numbers Between 5 and 30.

In order to distinguish between human and mouse PCR products, 2-10µl of each sample was incubated with 5 units of the appropriate restriction enzyme at 37°C for 2 hours. This reaction was carried out in a large (250µl) volume to dilute salts and detergents from the PCR buffer to prevent inhibition of restriction enzyme activity. (Control experiments demonstrated that this was indeed the case). Digested and undigested samples were ethanol precipitated in the presence of 25µg yeast tRNA (Sigma) as co-precipitant, collected by centrifugation and resuspended in 5µl gel loading buffer (5mM Tris-Borate [pH 8.3], 1mM EDTA, 7M Urea, 0.1% xylene cyanol, 0.1% bromophenol blue). Samples were analysed on pre-run, 5% polyacrylamide gels in the presence of 7M Urea (National Diagnositics) as denaturant and 0.5X TBE buffer. After electrophoresis at 40V/cm for 1 hour, the gel was cut to remove residual unincorporated nucleotide running below the xylene cyanol dye front, dried and exposed to x-ray film or PhosphorImager screens.

### Analysis of human hnRNP A2 expression

A similar competitive RT-PCR approach (Gilliland *et al.,* 1990) was used to accurately quantify human HnRNP A2 gene expression in a mouse background. After reverse transcription, cDNA samples were amplified by PCR using primer sets Hn9 and Hn12 [5'-CTCCACCATATGGTCCCC-3'], one of which was end-labelled using the protocol described above. In order to distinguish between human and mouse hnRNP A2 PCR products, 2-10µl of each sample was digested with 5 units *Hind*III at 37°C for 2 hours, purified, resolved on 5% denaturing polyacrylamide gels and results quantified as described above.

### Sequencing and Bioinformatic analyses of clones

HindIII genomic clones of both TBP (nucleotides 1-9098, Figure 20) and hnRNPA2 (nucleotides 1-15071, Figure 21) loci were sequenced by Baseclear, Leiden, NL. Using a primerwalking strategy starting with primers made to known sequence, regions of unknown sequence were generated; TBP nucleotides 1-5642 and hnRNPA2 nucleotides 1-3686.

These sequences were spliced together with previously known sequence data and were then used in bioinformatic analyses.
Direct comparisons were made between TBP and hnRNPA2 sequences using standard Smith-Watenman searching. This showed no obvious regions of homology other than several Alu repeats as shown in Figure 19. Masking these repeats and performing a comparison using the GCG bestfit program resulted in two short regions of homology as follows:

| | | |
|---|---|---|
| RNP 3868-3836: TBP 8971-9003 | length=33 | % identity =75.758 |
| RNP 3425-3459: TBP 9049-9083 | length=35 | % identity=74.286 |

CpG-islands were also identified and are shown in Figure 19. Nucleotide positions are as follows:
RNP 4399-5491, 5 749-6731
TBP 5285-5648, 6390-6966

Sequencing studies were performed as described above so as to provide more sequence data from the region immediately upstream of the RNP and TBP genes.

The sequence data given in Figures 20 and 21 begins at the 5' HindIII site and includes the Baseclear generated sequence and the already published sequence data spliced together. In the case of the TBP sequence the Baseclear sequence is denoted in capitals.

Analysis of these sequences demonstrated the existence of a previously characterised gene, HP1H-γ, or heterochromatin associated protein H-gamma upstream of the RNP gene (Figure 19 and 22). This gene has also been shown to be ubiquitously expressed by human tissue dot blot analysis (data not shown).

Bioinformatic analysis and sequence comparisons showed no obvious sequence homologies between the loci. However, a summary of the data is shown in Figure 19. As can be seen, several putative Sp1 transcription factor binding sites are located in the bidirectional promoter regions of the two loci. The CpG methylation free islands are also indicated. Both loci show a bidirectional stucture containing a cluster of ubiquitously expressed genes.

### Construction of hnRNP A2 EGFP reporter constructs

CMV-EGFP-IRES was constructed by digesting pEGFP-N1 (Clontech) with KpnI and NotI to liberate the EGFP sequence, this was then ligated into pIRESneo (Clontech) that had been partially digested with KpnI and then NotI. This created a vector with the EGFP gene 3' to the CMV promoter and 5' to IRESneo (CMV-EGFP-IRES).

The CMV promoter was exchanged for the RNP promoter to create the construct referred to in Figure 22 as RNP. CMV EGFP-IRES was digested with AgeI, blunted with T4 DNA polymerase (50mM Tris pH7.5, 0.05mM MgCl₂, 0.05mM DTT, 1mM dNTP, 1u T4 DNA polymerase/µg DNA) and then cut with NruI to release the CMV promoter to gibe EGFP-IRES. The RNP promoter was removed from an 8kb hnRNPA2 HindIII clone (8kb Hind BKS) which contained the promoters and first exons of the RNPA2 and HP1H-γ genes. 8kb Hind BKS was cut with BspEI and Tth111I (to release the 630bp promoter) blunted with T4 DNA polymerase, and the isolated RNP promoter ligated into EGFP-IRES.

5.5RNP was constructed by inserting the EGFP-IRES cassette into 8kb Hind BKS such that expression of EGFP was under the control of the RNP promoter. The latter was partially digested with Tth111I, blunted with T4 DNA polymerase and then digested with SalI, this removed all sequences 3' to the RNP promoter. The EGFP-IRES cassette was removed from CMV-EGFP-IRES by digestion with AgeI and blunted prior to digestion with XhoI. This was then ligated into the restricted 8kb Hind BKS.

5.5CMV was constructed by inserting the CMV-EGFP-IRES cassette into 8kb Hind BKS with the subsequent removal of the RNP promoter. 8kb Hind BKS was cut with BspEI, blunted and then digested with SalI removing the RNP promoter and all sequences 3' to the promoter. The CMV-EGFP-IRES cassette was removed from CMV-EGFP-IRES by digestion with NruI and XhoI and ligated into the digested 8kb Hind BKS.

Approximately 4 kb of DNA was removed from 5.5 RNP to leave 1.5 kb 5' to the RNP promoter creating 1.5RNP. This was achieved by digesting 5.5 RNP with BamHI which gave fragments of 4, 2.9 and 5 kb. The 2.9 and 5 kb fragments were then isolated and religated to create 1.5 RNP, when the 2.9kb fragment was inserted in the correct orientation.

The 5.5RNP construct was extended to include hnRNPA2 sequences 3' to the RNP promoter (constructs 7.5RNP and 8.5RNP), this region included the first exon and intron of hnRNPA2. In order to include the EGFP-IRES reporter in these constructs it was necessary to place the hnRNPA2 splice acceptor sequence of exon 2 in frame with the EGFP gene such that the first exon of hnRNPA2 could splice to the EGFP gene and hence EGFP expression could be driven off the RNP promoter. Two constructs were made which included the hnRNP2 splice acceptor, these contained 80bp and approximately 1kb of sequence 5' to the second exon, these sequences were obtained by PCR from MA160 which includes the whole hnRNPA2 genomic sequence. The 80bp sequence was isolated by PCR (20mMTris-HCl pH8.4, 50mM KCl, 1µM Primer, 2mM MgCl₂, 0.2mM dNTP 3.5 µg MA160 DNA, 5U Platinum Taq DNA Polymerase) using primers [5'ACCGGTTCTCTCTGCAAAGGAAAATACC 3'] and [5' GGTACCCTCTGCCAGCAGGTCACCTC 3'], the 1kb fragment was isolated using the primers [5'ACCGGTTCTCTCTGCAAAGGAAAATACC 3'] and [5'GGTACCGAGCATGCGAATGGAGGGAGAGCTCCG 3']. The primers were designed such that the PCR product contained KpnI and AgeI sites at the 5' and 3' ends respectively. PCR products were then cloned into the TA cloning vector pCR3.1 (Invitrogen).

The 80bp and 1kb fragments were isolated from pCR3.1 as KpnI-AgeI fragments and ligated into CMV-EGFP-IRES that had been partially digested with KpnI and then cut with AgeI, this created inframe fusions of the splice acceptor (SA) with the EGFP gene.

7.5RNP was constructed by digesting 8kb Hind BKS with ClaI, blunting with T4 DNA polymerase, then digesting with SalI. The 80bp SA-EGFP-IRES cassette was isolated by a KpnI partial digest followed by blunting with T4 DNA polymerase and XhoI digestion. This was ligated into the ClaI-SalI digested 8kb Hind BKS.

8.5RNP was constructed by an SphI partial digest of 8kb Hind BKS followed by digestion with SalI, the 1kb SA-EGFP-IRES cassette was similarly isolated by an SphI partial digest followed by restriction with XhoI. The cassette was ligated into 8kb Hind BKS to create 8.5 RNP.

4.0CMV was constructed by excising a 4kb fragment from 8kb Hind BKS with BamHI/HindIIIBstEII digestion. The ends of the fragment were then end-filled with Klenow and T4 DNA polymerase.

pEGFP-N1 (Clontech) was linearised with AseI, the ends blunted as above and then treated with calf intestinal phosphatase (CIP). Both fragments were then ligated overnight.

p7.5CMV was constructed by excising the 8.3kb fragment from p8kb Hind BKS with HindIII digestion. The ends of the fragment were then end filled with Klenow and T4 DNA Polymerase. pEGFP-NI (Clontech) was linearised with AseI, the ends were blunted as above and then treated with calf intestinal phosphatase (CIP): Both fragments were then ligated overnight. The resultant clones were screened for both forward and reverse orientations of the 8.3kb UCOE insert.

p16CMV was constructed by excising a 16kbp fragment from MA551 (hnRNPA2 genomic clone containing 5kb 5' and 1.5kbp 3' sequence including the entire coding region (16kb fragment shown in Figure 13C)) by Sal I digestion. The ends of the fragment were then end filled with Klenow and T4 DNA Polymerase. pEGFP-NI (Clontech) was linearised with AseI, the ends were blunted as above and then treated with calf intestinal phosphatase (CIP). Both fragments were then ligated overnight. The resultant clones were screened for both forward and reverse orientations of the 16kb UCOE insert.

### CHO transfection

CHO cells were harvested at 2 x 10⁷ cells/ml in serum free medium. 1 x 10⁷ cells (0.5ml) were used per transfection, along with 1ug (5ul) of linear DNA and 50ug (5ul) of salmon sperm carrier DNA. The DNA and cells were mixed and left on ice for 10 minutes. Cells were electroporated using the BioRad Gene Pulser II^{™} at 975uF/250V and then left on ice for 10 minutes. The mix is then layered onto 10mls of complete medium (HF10) and spun at 1400rpm for 5 minutes. The supernatant is removed and the pellet resuspended in 5mls of HF10. The cells were then plated out at 5 x 10⁴ or 1 x 10⁴ in 10cm dishes and at 2 x 10⁶ cells per T225 flask. After 24 hrs the cells were placed under selection, initially at 300ug/ml G418 and then after 4 days at 600ug/ml G418. 10 days after transfection colonies were stained with methylene blue (2% solution made up in 50% ethanol) and counted. Duplicate plates were maintained in culture either as restricted pools or as single cell clones.

### Analysis of GFP expression in transfected CHO clones

The transfected cells were maintained on G418 selection at 600µg/ml. Cells were stripped off 6-well plates for expression analysis of GFP. Cells were washed with phosphate buffered saline (PBS; Gibco) and incubated in Trypsin/EDTA (Sigma) until they had detached from the surface of the plates. An excess of Nutrient mixture F12 (HAM) medium (Gibco) supplemented with 10% foetal calf serum (FCS; Sigma) was added to the cells and the cells transferred to 5ml polystyrene round-bottom tubes. The cells were then analysed on a Becton-Dickinson FACscan for the detection of GFP expression in comparison to the autofluorescence of the parental cell population. 19 RNP clones, 24 5.5RNP clones, 21 CMV clones and 12 5.5CMV clones were analysed and the average taken of the median fluorescence of all the positive clones.

### Analysis of GFP expression in transfected CHO pools

Colonies of transfected CHO cells, that had undergone selection on G418, were stripped from a T225 tissue culture flask and plated on 10cm petri dishes to give approximately 100 colonies/plate. When the colonies had grown up, the cells were stripped and this limited pool of transfected cells was analysed for GFP expression. GFP expression was monitored on a regular basis, with the pools split 1:10 every 3-4 days. Cells were always split into 24-well plates the day before analysis, so that the cells were approximately 50% confluent on the day of analysis. The cells were then stripped from the 24-well plates and analysed in the same way as the previous section. For the expression time course, a marker region (M1) was set which contained only a minor proportion of the positive population of cells and was used to investigate any loss of GFP expression from the initial level over time.

### FISH analysis of single/low copy number integrants

### FISH analysis using the 40kb TBP cosmid pWE-TSN or the pBL3-TPO-puro.

Mouse Ltk- cells grown in DMEM-10% fetal calf serum were electroporated with the 40kb TBP cosmid pWE-TSN (Figure 9) or the 25kb plasmid pBL3-TPO-puro. The transfectants were selected with either 200 mg /ml G418 (TSN) or 5 mg/ml puromycin (TPO) and single or low copy clones were generated as outlined previously. Logarithmically growing cells from the selected clones were treated with 0.4mg/ml colchicine for 1 h prior to harvest. Cells were then hypotonically swollen in 0.056 M KCl, fixed in 3:1 methanol-acetic acid, and spread on microscope slides to obtain metaphase chromosomes. The slides were pretreated with 100 mg of RNaseA/ml in 2XSSC (1XSSC is 0.15 M NaCl, 0.015 M sodium citrate) for 1 h at 37°C, washed in 2XSSC, and put through an ethanol dehydration series (70, 90, and 100% ethanol). The chromosomes were denatured at 70°C for 5 min in 70% formamide-2XSSC, plunged into ice-cold 70% ethanol, and dehydrated as before. One hundred nanograms of TBP probe (entire TPO plasmid carrying 25 kb of human genomic DNA comprising the TBP gene) and 50 nanograms of mouse gamma-satellite probe (as described by Horz et al., Nucl. Acids Res. 9; 683-696, 1981) were labelled with digoxigenin-11-dUTP and biotin-16-dUTP, respectively, by nick translation (Boehringer) following manufacturer's instructions. Labelled probes were precipitated with 1 mg of cot-1 DNA and 5 mg of herring sperm DNA, resuspended in 50% formamide-2XSSC-1% Tween 20-10% dextran sulfate, denatured at 75°C, the TBP probe preannealed for 30 min at 37°C and pooled and applied to the slides. Hybridization was carried out overnight at 37°C. The slides were washed four times for 3 min each time in 50% fonnamide-2XSSC at 45°C, four times for 3 min each time in 2XSSC at 45°C, and four times for 3 min each time in 0.1xSSC at 60°C. After being washed for 5 min in 4XSSC-0.1% Tween 20, the slides were blocked for 5 min in 4XSSC-5% low-fat skimmed milk. The biotin labelled probe was detected by 30 min incubation at 37°C with each of the following: avidin-conjugated Texas Red (Vector Laboratories Inc, USA) followed by biotinylated anti-avidin (Vector Laboratories Inc, USA) and avidin-conjugated Texas Red (Vector Laboratories Inc, USA). Digoxigenin labelled probe was detected at the same time as biotin detection with each of the following: anti-digoxigenin-fluorescein (FITC, Boehringer) followed by mouse anti-FITC (DAKO) and horse fluorescein-conjugated anti mouse IgG (Vector Laboratories Inc, USA). Between every two incubations, the slides were washed three times for 2 min each time in 4XSSC-0.1% Tween 20. The slides were counterstained with DAPI (4'-6-diamidino-2-phenylindole) and mounted in Vectashield (Vector Laboratories Inc, USA). Images were examined with an oil 100X objective on a fluorescence microscope. The images were capture using a Photometrics cooled charge-couple device camera and Vysis Smartcapture software.

### FISH analysis using the 16RNP-EGFP Construct.

The 16RNP-EGFP vector was constructed by inserting the EGFP-IresNeo expression cassette and some RNP 5' sequences from 8.5RNP into MA551. 8.5 RNP was digested with *Xho*I, blunted with T4 DNA polymerase and then digested with *Pac*I, the resulting fragment was ligated into MA551 that had been cut with *Nhe*I, blunted and then digested with *Pac*I. As with 8.5RNP expression is driven off the RNP promoter resulting in an inframe fusion of exon 1 of RNP with EGFP.

Clones of mouse LTK⁻ cells transfected with 16RNP-EGFP were grown in DMEM-10% fetal calf serum and 200 µg /ml G418. Logaritmically growing cells were treated with 0.4µg/ml colchicine for 1 h prior to harvest. Cells were hypotonically swollen in 0.056 M KCI, fixed in 3:1 methanol-acetic acid, and spread on microscope slides to obtain metaphase chromosomes. The slides were pretreated with 100 µg of RNase A/ml in 2xSSC (lx SSC is 0.15 M NaCl, 0.015 M sodium citrate) for I h at 37°C, washed in 2xSSC, and put through an ethanol dehydration series (70, 90, and 100% ethanol). The chromosomes were denatured at 70°C for 5 min in 70% formamide-2xSSC, plunged into ice-cold 70% ethanol, and dehydrated as before. One hundred nanograms of 16RNP-EGFP and 50 nanograms of mouse gamma-satellite (Horz et al., Nucl.Acids Res. 9, 683-696, 1981) were labelled with digoxigenin-11-dUTP and biotin-16-dUTP, respectively, by nick translation (Boehringer) following manufacturer's instructions. Labelled probes were ethanol precipitated with 5 µg of herring sperm DNA and the RNP probe with 1 µg of cot-1 DNA; resuspended in 50% formamide-2xSSC-1% Tween 20-10% dextran sulfate; denatured at 75°C, the RNP probe preannealed for 30 min at 37°C; pooled and applied to the slides. Hybridization was carried out overnight at 37°C. The slides were washed four times for 3 min each time in 50% formamide-2xSSC at 45°C, four times for 3 min each time in 2xSSC at 45°C, and four times for 3 min each time in 0.1xSSC at 60°C. After being wahed for 5 min in 4xSSC-0.1% Tween 20, the slides were blocked for 5 min in 4xSSC-5% low-fat skimmed milk. The biotin was detected by 30 min incubation at 37°C with each of the following: avidin-conjugated Texas Red (Vector Laboratories) followed by biotynylated anti-avidin (Vector Laboratories) and avidin-conjugated Texas Red (Vector Laboratories). Digoxigenin was detected at the same time as biotin with each of the following: anti-digoxigenin-fluorescein (FITC, Boehringer) followed by mouse anti-FITC (DAKO) and horse fluorescein-conjugated anti mouse IgG (Vector Laboratories). Between every two incubations, the slides were washed three times for 2 min each time in 4xSSC-0.1% Tween 20. The slides were counterstained with DAPI (4'-6-diamidino-2-phenylindole) and mounted in Vectashield (Vector). Images were examined with an oil x100 objective on a Olympus BX40 fluorescence microscope. The images were captured with a Photometrics cooled charge-couple device camera and Vysis Smartcaprture software.

### Copy number determination

Genomic DNA was prepared from cell clones by standard procedures (Sambrook *et al*., 1989). Transfected gene copy number was determined by Soutem blot analysis of *HincII* digested genomic DNA. The transgene was detected as a 2.5 kbp band by hybridization to a 1 kpb fragment from 16RNP-EGFP, comprising the neomycin resistance gene, labelled with [α-³²P] dCTP following manufacturer's instructions (Megaprime DNA labelling system, Amersham). For normalization, blots were simultaneously hybridized with a 1kbp *NcoI* fragment, labelled as above, derived from the murine *vav* locus (Ogilvy *et al.,* 1998) which gave a 1.4 kbp band. As copy number standards, DNA from several pWE-TSN clones was digested with *PstI* and hybridized to the above probes. Hybridization signal quantification was performed with a Cyclone PhorsphorImager (Packard).

### Analysis of GFP expression in transfected Ltk clones

The transfected cells were maintained on G418 selection at 200 µg/ml. Cells at 80-100% confluency were stripped off 6-well plates for expression analysis of GFP. Cells were washed with PBS and incubated in Trypsin/EDTA (Sigma) until they had detached from the surface of the plates. An excess of DMEM (Gibco) supplemented with 10% foetal calf serum (Sigma) was added to the cells and transferred to 5 ml polystyrene round-bottom tubes. The cells were then analyzed on a Becton-Dickinson FACscan for the measurement of GFP fluorescence in comparison to the autofluorescence of an untransfected control.

### Production of EBV reporter construct.

A DNA fragment containing the cytomegalovirus (CMV) promoter , the enhanced green fluorescent protein (EGFP) and the simian virus 40 (SV40) polyadenylation sequence, was removed from the vector, pEGFP-N1 (Clontech), by restriction endonuclease digestion with Ase I and Afl II using the manufacturers recommended conditions (NEB). The DNA was electrophoresed on a 0.5% agarose gel to separate the fragment from the vector backbone. The DNA fragment was cut out of the gel and purified from the gel slice using the standard glass milk purification technique. The fragment was blunted using T4 DNA polymerase (NEB) according to the manufacturers conditions and purified by 1:1 (v/v) extraction with phenol:chloroform:isoamylalcohol (25:24:1) followed by ethanol precipitation.

The reporter cassette was then cloned into the Epstein-Barr virus (EBV) vector, p220.2 (described in International Patent Application WO 98/07876). P220.2 was restriction endonuclease digested with Hind III (a unique site in the multiple cloning sequence (MCS) of the vector), blunted and purified in the same way as described above. The reporter cassette was ligated into p220.2 using T4 DNA ligase (Promega). The ligation reaction was performed in a 10µl volume using 200ng of the linearised p220.2 and either a molar equivalent or 5 molar excess of the CMV-EGFP-SV40pA fragment, in 1 × ligation buffer (Promega). The reaction was incubated overnight at room temperature. 2.5µl of the ligations were transformed into electrocompetent DH5α *E.coli* cells by electroporation at 2.5kV, 400Ω, 25µF followed by the addition of 900µl of SOB medium and incubation at 37°C for 1 hour. 200µl of each of the transformations were plated on LB-ampicillin agar plates and incubated overnight at 37°C.

The resulting colonies were screened for the presence of the reporter cassette by colony polymerase chain reaction (PCR) with DNA primers in the CMV and EGFP sequence, using Taq polymerase (Advanced Biotechnologies) with the manufacturers standard conditions. Positive colonies were grown overnight in LB-ampicillin medium and were analysed as alkaline-lysis DNA minipreparations (Qiagen). The DNAs were screened for the correct orientation of the fragment using Bam HI restriction endonuclease digestion. The resultant construct was named p220.EGFP.

p220.EGFP was demonstrated to express EGFP by analysis on a Becton-Dickinson FACScan, after electroporation into K562 cells, using essentially the same method as described below.

### Production of EBV reporter constructs containing the hnRNPA2 16kb (RNP16) UCOE fragment.

A *Sal*I site was removed from p220.EGFP by partial restriction endonuclease digestion of the vector with Sal I, followed by blunting and religation of the vector, thus leaving a unique Sal I site in the multiple cloning site (MCS) of the vector which could be utilised for the cloning of the 16kb RNP fragment. The resultant vector was restriction endonuclease digested with Sal I, treated with calf intestinal phoshatase (to prevent recircularisation of the vector during the ligation) and purified by phenol:chlorofom extraction and ethanol precipitation.

The 16kb RNP fragment was removed from the vector, MA551, using the restriction endonuclease, Sal I, and was blunted, purified by electroelution and ligated into the linearised vector. The ligation reactions were set up in the same way as previously described (using a molar equivalent amount of the fragment), followed by transformation and screening of the colonies for the presence of the fragments. Colonies were screened as DNA minipreparations, with positive colonies being confirmed by agarose gel electrophoresis analysis. The correct orientation of the 16kbp RNP fragment was determined by restriction endonuclease analysis using Not I. The resultant construct was named p220.RNP16.

### Transfection of EBV reporter constructs into Hela cells.

Hela cells were transfected in 6-well plates with p220.EGFP and p220.RNP16, using the CL22 peptide-mediated delivery system described in International Patent Application WO 98/35984 and described below. After culture for 24 hours, hygromycin B (Calbiochem) selection was added to a final concentration of 400µg/ml. Hygromycin B-resistant colonies of cells were maintained in culture and analysed periodically for GFP expression on a Becton-Dickinson FACScan. Cells were routinely split into 24-well plates the day before analysis so that they were approximately 50% confluent on the day of analysis. For the expression time course, a marker region was set which contained the GFP-expressing population of cells and this marker was used to investigate the stability of GFP expression over time. Transfected Hela cells were also taken off hygromycin B selection to investigate the stability of GFP expression, in the absence/presence of the UCOE, without selection pressure.

### Cloning of CET200

PEGFPN1 was restricted with NheI/NotII and the following oligos were anealed and inserted to create the multiple cloning site (MCS):
5' CTAGCGTTCGAAGTTTAAACGC 3'
5' GGCCGCGTTTAAACTTCGAACG 3'

The resulting plasmid was restricted with AseI blunted and the 8.3kb HindIII fragment blunted RNP A2 fragment inserted. The resulting orientation was then determined creating the final vector CET200 (see Figure 49)

### Cloning CET201

pUC19 was restricted with EcoRI/ArI and blunted, removing one PvuI site thus creating a unique PvuI site for linearisation (pUC19Δ). The MCS was removed from pEGFPN1 by digestion with NheI/AgeI and blunted. This creates the NheI site. The CMV EGFP SV40 cassette was removed as a AfIII-blunt AseI fragment and inserted into pUC19Δ tthat had been restricted with PvuII and pGK puro bGH (from pGK-puro-BKS) was inserted withNdeI. The resulting vector was then restricted with NheI/NotI removing EGFP and the MCS inserted as described above. The MCS containing vector was then restricted with HindIII and the 8.3kb RNP HindIII fragment inserted creating the final vector CET210 (see Figure 49).

### Preparation of Plasmid Containing a UCOE

### Cloning of RNP-UCOE containing reporter constructs

**p8kb Hind BKS** contained a 8.3kb HindIII genomic fragment of the RNP locus which contained the promoters and first exons of RNPA2 and HP1H-γ genes.

**pCMV EGFP-IRES** was constructed by digesting pEGFP-N1 (Clontech, same as CMV-EGFP Figure 35) with KpnI and NotI to liberate the EGFP sequence, this was then ligated into pIRESneo (Clontech) that had been partially digested with KpnI and then NotI. This created a vector with the EGFP gene 3' to the CMV promoter and 5' to IREsneo.

**IntronA-CMV** was cloned by taking the 1.5kbp IntronA-CMV fragment from pTX0350 (a pUC based CMV IntronA-MAGE1 plasmid) with NruI (blunt cutter) and Hind III. pEGFP-NI was digested with Asel and the ends of the fragment were then end filled with Klenow and T4 DNA Polymerase. This was then digested with HindIII to obtain a 4.2 Kb fragment. Both fragments were then ligated overnight.

**p4.0CMV** was constructed by excising a 4kb fragment from p8kb Hind BKS with BamHI/HindIII/BstEII digestion. The ends of the fragment were then end-filled with Klenow and T4 DNA polymerase.

pEGFP-N1 (Clontech) was linearised with AseI, the ends blunted as above and then treated with calf intestinal phosphatase (CIP). Both fragments were then ligated overnight. The resultant clones were screened for both forward and reverse orientations of the 4kb UCOE insert.

**p7.5CMV** was constructed by excising the 8.3kb fragment from p8kb Hind BKS with HindIII digestion. The ends of the fragment were then end filled with Klenow and T4 DNA Polymerase. pEGFP-NI (Clontech) was linearised with AseI, the ends were blunted as above and then treated with calf intestinal phosphatase (CIP). Both fragments were then ligated overnight. The resultant clones were screened for both forward and reverse orientations of the 8.3kb UCOE insert.

**p16CMV** was constructed by excising a 16kbp fragment from MA551 (hnRNPA2 genomic clone containing 5kb 5' and 1.5kbp 3' sequence including the entire coding region) by Sal I digestion. The ends of the fragment were then end filled with Klenow and T4 DNA Polymerase. pEGFP-NI (Clontech) was linearised with AseI, the ends were blunted as above and then treated with calf intestinal phosphatase (CIP). Both fragments were then ligated overnight. The resultant clones were screened for both forward and reverse orientations of the 16kb UCOE insert.

### Transfection of HeLa cells using the CL22 Peptide

The CL22 peptide has the amino acid sequence:
NH₂-KKKKKKGGFLGFWRGENGRKTRSAYERMCNILKGK-COOH

The CL22 peptide was used as a transfecting agent in accordance with the methods described in W0 98/35984.

HeLa cells are routinely cultured in EF10 media, spliting a confluent flask 1:10 every 3 to 4 days. 24 Hours prior to transfection, cells were seeded at 5x10⁴ per well (6 well plate). Complexes were formed 1 hour prior to transfection by mixing equal volumes of DNA:CL22, which are at concentrations of 40µg/ml and 80µg/ml respectively in Hepes buffered saline (10mM Hepes pH7.4, 150mM NaCl), and incubated at room temperature for 1 hour. Media was removed from cells, which were then washed with 1% phosphate buffered saline. 2.5µg of DNA:complex (125µl) was then added to the cells and the volume made up to 1 ml with RAQ (RPMI media (Sigma), 0.1% human albumin, 137µM chloroquine (added fresh)) which gives a final concentration of chloroquine of 120µM. Cells and complex were incubated for 5 hours at 37°C. The complex was then removed and replaced with EF10 media (Minimal Essential medium (Sigma), 10% Foetal calf serum, 100 unit/ml penicillin/0.1 mg/ml streptomycin, 1x Non-Essential amino acids (Sigma)).

### Analysis of GFP expression in transfected HeLa cells

Cells were stripped off 6-well plates for expression analysis of GFP. Cells were washed with phosphate buffered saline (PBS; Gibco) and incubated in Trypsin/EDTA (Sigma) until they had detached from the surface of the plates. An excess of EF10 medium (Gibco) supplemented with 10% foetal calf serum (FCS; Sigma) was added to the cells and the cells transferred to 5ml polystyrene round-bottom tubes. The cells were then analysed on a Becton-Dickinson FACscan for the detection of GFP expression in comparison to the autofluorescence of the parental cell population.

### Preparation of total DNA samples

Inorder to examine the episomal DNA content of the transfected populations, a total preparation of cellular DNA was made. The cells were washed with PBS and then lysed with lysis buffer [10mM tris pH7.5, 10mM EDTA pH 8.0, 10mM NaCl and 0.5% Sarcosyl to which was added fresh Proteinase K 1mg/ml F/C]. The cell lysate was scrapped off the plate and transferred to an eppendorf tube with a wide bore pipette. Following overnight incubation at 65°C the cell lysate was phenol/chloroform extracted and ethanol precipitated. The DNA pellet was resuspended in TE pH8.0.

### Detection of Episomal DNA in total genomic DNA samples

Total genomic DNAs, prepared from transfected cells, 7 days after transfection, were restriction endonuclease digested using an endonuclease that linearised the DNA constructs used in the transfection and therefore any episomal DNA present in the sample. Apa L1 (NEB) was used for mock, CMV-EGFP, IntronA-CMV and 4.0CMV forward and reverse samples. BspLU11 I (Boehringer) was used for 7.5CMV forward and reverse samples. 10µl (20% of the sample) of total genomic DNA were digested with 30 units of restriction endonuclease, for 16 hours according to the manufacturers recommended conditions. The samples were electrophoresed for 400 volt/hours on a 0.6% agarose gel along with 100pg or 4ng of linearised plasmid controls. The gel was then transferred to Hybond-N Hybridisation transfer membrane (Amersham) by Southern blotting. Briefly, the gel was incubated in 0.25M HCl for 15 minutes to depurinate the DNA, followed by denaturation in 1.5M NaCl/0.5M NaOH for 45 minutes and neutralisation in 1.5M NaCl/0.5M Tris-Cl, pH7.0, for 45 minutes. The DNA was then transferred from the gel to the membrane by capillary blotting in 20X SSC (3M NaCl, 0.3M Na₃citrate-2H₂O, pH 7.0) for 16 hours. The filter was air-dried for 1 hour and cross-linked for 2 minutes using a UVP CL-100 ultraviolet crosslinker (GRI) at an energy setting of 1200. The membrane was probed using a radioactive EGFP probe using "Church hybridisation conditions". The membrane was prehybridised in 0.5M NaPi pH7.2, 1% SDS at 65°C for longer than 2 hours. An EGFP fragment of DNA was removed from pEGFP-N1 (Clontech) by restriction endonuclease digestion with Bgl II/Not I (NEB), separated by electrophoresis and purified from the gel slice using a GFX^{™} PCR DNA and Gel Band Purification kit (Amersham Pharmacia Biotech). 50ng of the EGFP fragment were labelled with α-³²P dCTP (3000Ci/mmol; Amersham) using a Megaprime DNA labeling kit (Amersham). The labelled probe was mixed with 100µl of 10mg/ml salmon sperm DNA, incubated at 95°C for 10 minutes and placed on ice followed by addition to the hybridisation. The membrane was hybridised for 16 hours at 65°C, followed by two 30 minute washes in 40mM NaPi pH7.2, 1% SDS at 65°C. The radiolabelled membrane was then analysed on a Cyclone storage phoshor system (Packard) after exposure on a super resolution phosphor screen.

### Fluorescence Microscopy

The transfected cells cultured in 6-well plates were viewed under fluorescence using a Zeiss Axiovert S100 inverted microscope. Photography was carried out at regular timepoints throughout using a Zeiss MC100 camera and Fujichrome Provia 400ASA film.

### EXAMPLE 1

### ANALYSIS OF THE HUMAN TBP GENE LOCUS

### Mapping the TBP gene domain

The human TBP gene is 20kb in length (Chalut *et al.,* 1995), located on chromosome 6q27-tel (Heng *et al.,* 1994) and is closely linked to the gene encoding the protein C5 which forms part of a ubiquitous proteosome (Figure 1A and C; Trachtulec, Z. *et al.,* 1997). The C5 gene is divergently transcribed from a position 1kb upstream from the cap site of TBP. TBP and C5 may therefore comprise dual promoters. This has important ramifications with regards to the construction of expression vectors based on TBP since dual promoters do not necessarily function with equal efficiency in both directions (see Gavalas and Zalkin, 1995).

Sequence analysis has revealed that the TBP/C5 promoter regions are contained within a methylation-free, CpG-island of 3.4kb. This extends from a *Fsp*I site within intron 1 of C5 and a *Hind*III site within intron 1 of TBP and encompasses the most 5' 1 kb sequences of the first intron of both genes as well as the 1.4kb region between their transcriptional start sites (Figure 1B).

The human TBP gene locus consists of 3 closely linked genes. The PSMB1 gene (also referred to herein as C5) is divergently transcribed from a position 1 kb upstream from the cap site of TBP. The 3' end of a recently identified gene, PDCD2 is located 5 kb downstream of TBP. These 3 transcription units span a total of 50 kb. Downstream of the PSMB 1 gene in the direction of the centromere, there is a region of at least 80kb which consists of blocks of repeat sequence DNA with no identifiable structural genes. Upstream of the PDCD2 gene toward the telomere there is a 30 kb stretch of repeat, non-coding sequences followed by a potential new transcription unit. The PDCD2 gene is approximately 150 kb from the start of the telomeric repeat region. This makes the TBP locus the first structural gene cluster from the telomere on the long arm of chromsome 6.

### Pattern of gene expression from the TBP domain

The tissue distribution of expression from within the TBP gene cluster was assessed using a commercially available dot-blot prepared with poly(A)⁺-RNA derived from a wide range of human tissues and cell types (Figure 35A). Hybridisation of this dot-blot with appropriate probes showed that the PSMB1 (Figure 35B), PDCD2 (Figure 35C) and TBP (Figure 35D) genes are all ubiquitously expressed. These data confirm that the TBP locus consists exclusively of a ubiquitously expressed chromatin domain.

### Mapping transgene integrity in mice harbouring pCP2-TLN

The pCYPAC-2 derived clone pCP2-TLN (Figure 1) which is 90kb in length was used to generate transgenic mice. This clone starts at a position 46kb downstream of the C5 gene (65kb 5' of TBP) and terminates 4.5kb 3' of TBP. This clone therefore possesses both C5 and TBP genes in their entirety.

Three transgenic lines with pCP2-TLN have been produced. The initial Southern blot analysis with probes derived from the ends of pCP2-TLN showed that line TLN:3 possesses two copies of the transgene (Figure 2a,b lanes TLN-3) in a head-to-tail configuration (Figure 3a, lanes TLN:3). However, one copy appears to have suffered a 5' deletion, which extends into the TBP promoter (Figure 4, lanes TLN:3). Line TLN:8 by end fragment analysis appeared to harbour 3 copies of pCP2-TLN (Figure 2a,b lanes TLN-8). Line TLN:28 appeared to harbour several copies at multiple integration sites (Figure 3a, lanes TLN:28).

A summary of the initial analysis of transgene copy number and integrity in these TLN mice is shown in Figure 3B.

Further analysis of the transgenic lines produced with pCP2-TLN has now shown that line TLN:3 contains two deleted copies of pCP2-TLN such that a single functional copy of the TBP and PSMB1 genes remains intact (Figure 3C, TLN:3). Line TLN:8 harbours two, tandem integrated copies of pCP2-TLN (Figure 3C, TLN:8). Line TLN:28 possesses 4 tandem arranged copies of pCP2-TLN (Fifure 4, TLN:28). The deletions at the 5' and 3' ends of the transgene tandem arrays in TLN:8 and TLN:28 still leave the PSMB1 and TBP genes intact.

As expected the methylation-free island of TBP/C5 is preserved in transgenic mice (data not shown) as has been observed for the 5' region of other genes which harbour a CpG-rich domain (e.g. murine Thy-1; Kolsto *et al.,* 1986)

### Expression analysis of the TBP and C5 transgenes on pCP2-TLN in mice

An RT-PCR based assay that would simultaneously detect both the endogenous murine as well as the human transgene TBP and C5 message was developed. Primers (TB-14 and TB-22) for the RT-PCR reactions were selected from a region of homology between the human and mouse TBP cDNA sequence (Figure 5b). This allows an RT-PCR product of 284 bp to be produced from both mRNAs by a single pair of primers. In order to distinguish between the human and mouse TBP products, minor base differences resulting in changes in the presence of restriction enzyme sites are exploited. Digestion with *Bsp*1407I cleaves the human PCR product, giving rise to a fragment of 221 nucleotides (nt) (Figure 6a). Similarly, from a region of homology between the human and mouse C5 cDNA sequence (Figure 5a), allowed the generation of an RT-PCR product of 350nt from both sequences. Cleavage with *Pst*I reduced the size of the product derived from the murine C5 mRNA to 173nt (Figure 7a)

Primers TB14 (Figure 5b) and C5RTF (Figure 5a) were end-labelled with ³²P resulting in the generation of radioactive products after the PCR reaction. These products are finally resolved by electrophoresis on denaturing polyacrylamide gels (Figures 6b-c and 7b).

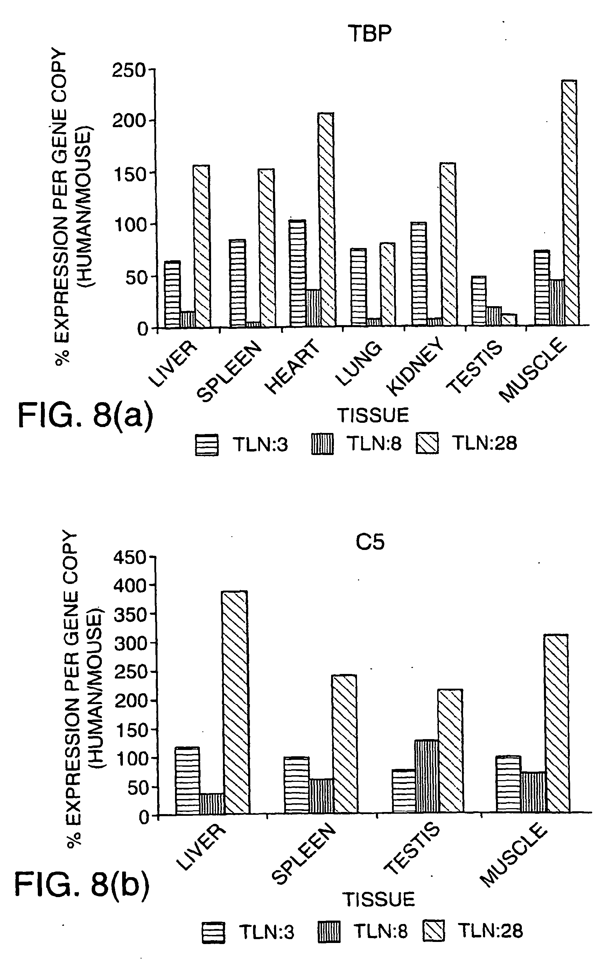

Total RNA (1µg) from various tissues of transgenic mouse lines TLN:3, TLN:8, and TLN:28, were subjected to the above analytical procedure and quantified by PhosphorImager analysis (Figure 8). All mice showed significant levels of expression of both the human TBP and C5 transgenes in all tissues analysed including TLN:3, which harbours a single intact copy of these two genes. Most importantly, a reproducible level of expression was observed between tissues in a given mouse line especially for C5. This indicates that the TLN clone in all likelihood possesses a ubiquitous chromatin opening capability. However, some variation in the level of expression per transgene copy number was observed between mouse lines. In addition, expression of TBP in line TLN:8 between tissues also varied from 5-40%. These results suggest that although TLN possesses a chromatin opening capability, the C5 and especially the TBP promoters are prone to positive and negative transcriptional interference. This in turn implies that the inherent transcriptional activating potential of the TBP and C5 regions on this clone are weak and therefore unable to always exert a dominant effect over position effects. This is in contrast to what seems to be a chromatin opening UCOE effect of this region, which is strong and appears to over-ride such positon effects. This hypothesis is supported by the observation that the weaker TBP promoter is more prone to variability; compare, for example, the ratio of TBP levels between spleen and muscle with that for C5 in line TLN:8 (Figure 8).

Transgene expression analysis as described previously, was carried out using tissues from mice that were between 2 and 6 months of age. The stability of transgene expression was also assessed in 23 month old mice from lines TLN:3 and TLN:8 by analysing PSMB1 mRNA. Similar results were obtained in both lines compared to that obtained with the younger animals. The result further demonstrates that the transgenes are maintaining a transcriptionally competent open chromatin structure.

### Expression Analysis of a 40kb sub-clone of the TBP locus

The reproducible, physiological levels of expression given by the pCP2-TLN clone in transgenic mice indicate that it possesses a ubiquitous chromatin opening capability. As a first step to fine mapping the region(s) of DNA responsible for this activity, we have begun to analyse a 40kb subclone (pCP2-TSN; Figure 1a) of the human TBP locus. The pCP2-TSN clone possesses 12kbp of both 5' and 3' flanking sequences surrounding the TBP gene. As a result it only harbours a complete TBP gene and a 3' truncated mutant of C5.

Previous work with the human β-globin LCR demonstrated that an initial indication for the presence of LCR activity may be obtained by comparing expression levels between stable transfected tissue culture cell clones harbouring a single copy of the transgene. It has been found that the more complete the LCR element, the higher the degree of reproducibility of expression between independent clones. Expression analysis of pCP2-TSN was conducted using this strategy to assess for the presence of LCR-type activity.

pCP2-TSN was first cloned into the cosmid vector pWE15 (Clontech) which possesses a neomycin resistance gene (Figure 9). The resulting pWE-TBP construct was then used to generate stable transfected clones of murine fibroblast L-cells. The transgene copy number of 23 clones was then determined by Southern blot analysis (Figure 10). A number of clones representing a range of copy numbers were then selected and analysed for transgene expression as described for the transgenic mice above. The results are summarised in Figure 11 and show that expression at or above physiological levels are obtained per copy of the transgene up to a number of eight. With copy numbers of 20 or more, expression levels per transgene are reduced to 30-40% of wild type.

These data demonstrate that reproducible, physiological levels of expression can be produced by pCP2-TSN at both single and multiple transgene copy numbers. This strongly suggests that this genomic clone possess a ubiquitous chromatin opening capability. There are clearly a number of clones (e.g. number 4, 33 and 6), which show a pronounced "positive" position effect giving rise to expression levels that are markedly greater than physiological per transgene copy. This would be the anticipated outcome in certain cases where integration of the transgene had taken place within already open, active chromatin. The nearby presence of a strong transcriptional enhancer under these circumstances would be expected to have a stimulatory effect on the inherently weak TBP promoter.

The stability of expression of the constructs was tested over a 60 day period. Expression levels were found to remain constant (Figure 36). This was even the case when drug selective pressure was removed (Figure 36, lanes marked -G418). In addition, expression remained stable through successive freeze and thaw cycles of the cells regardless of whether drug selective pressure was maintained.

### Expression Analysis of a 25kb sub-clone of the TBP locus

The 25 kb genomic clone (TPO) spanning the TBP gene with 1 kb 5' and 5 kb 3' flanking sequences (Figure 1C) was cloned into the polylinker region of a modified pBluescript vector harbouring a puromycin resistance gene to give pBL-TPO-puro as described above. The construct was used to generate stable transfected clones of murine fibroblast L-cells. The pBL-TPO-puro construct gave similar results to those obtained using the TSN construct (Figure 37). The data demonstrate that reproducible physiological levels of expression can be produced by both TSN and TPO at single and multiple transgenes copy numbers. The data is consistent with the genomic clones possessing a ubiquitous chromatin opening capability. This surmise is further enhanced by the finding that TPO clone numbers 7 (two copies), 29 (single copy) and 34 (two copies) are centromeric integration events (data shown below) demonstrating that the genomic fragment has the ability to express from within a heterochromatin enviroment.

There are clearly a number of clones (e.g. Figure 37, clone 11), which show a pronounced "positive" position effect giving rise to expression levels that are markedly greater than physiological per transgene copy. This would be the anticipated outcome in certain cases where integration of the transgene had taken place within already open, active chromatin. The nearby presence of a strong transcriptional enhancer under these circumstances would be expected to have a stimulatory effect on the inherently weak TBP promoter.

Similar results have also been obtained using Hela cells instead of CHO cells (data not shown).

### Mapping DNase I hypersensitive sites

All known LCR elements have been found to be regions of high, tissue-specific DNase I hypersensitivity, indicative of the highly open chromatin configuration which these elements are thought to generate. We have therefore begun to analyse for the presence of DNase I hypersensitive (HS) sites both within and around the human TBP gene. Figure 12 summaries a series of experiments using nuclei from the human myelogenous leukaemia cell line K562, which maps DNase I HS sites over a 40kb region starting from 12kb 5' and extending 4.5kb 3' of the TBP gene. The only HS sites that are evident throughout this region map to the immediate promoter regions of the C5 and TBP genes (Figure 12, top panel, *Hind*III digest/*Hind*III-*Xba*I probe). These HS sites correlate well to previously identified promoter elements important for TBP and C5 gene expression as determined by transient transfection assays (Tumara, T. *et al*., 1994; Foulds and Hawley, 1997). However, it would appear that if LCR-type elements are present within this locus, they are at a considerable distance from the transcriptional start sites of both the TBP and C5 genes. This places any LCR-type element outside of the 40kb clone spanning the TBP gene that has given an initial indication of ubiquitous chromatin opening capability.

### FISH Analysis

A total of 34 clones carrying 1-2 copies of the human TBP transgene were analyzed by FISH. The TBP transgene and the heterochromatin component of the mouse centromere, the gamma or major satellite, were detected with Fluorescein and Texas Red, respectively. This produced green and red fluorescent signals in the clones in which the transgene had integrated into the chromosome arm (see Figure 39A). However, in the case of centromeric integration both signals colocalized and a mixture of both colours could be detected as a yellow fluorescent signal. Two clones, 344-6 and 344-37, out of the 18 generated with pWE-TSN, showed the transgenic signal in the centromeric region. In clone 344-6, the TBP transgene had integrated in the centromere of a Robertsonian chromosome, whereas integration in clone 344-37 was in a typical mouse acrocentric chromosome.

Three clones, 440-7, 440-29, and 440-34, out of the 16 generated with pBL3-TPO-puro, showed centromeric integration in typical acrocentric chromosomes. Clone 440-29, which carried a single copy of the TBP transgene, showed the TBP signal clearly surrounded by heterochromatic satellite sequences (see Figure 39B and C). It was further shown that these clones continued to express TBP at physiological levels for at least 12 to 14 weeks in the absence of selection (data not shown).

These results show that a single copy of the 25kb fragment of the TBP locus (TPO) is capable of ensuring physiological expression even in the context of a heterochromatic location (i.e. centromeric integration), and thus provides formal proof of chromatin opening (Sabbattini P, Georgiou A, Sinclair C, Dillon N (1999) Analysis of mice with single and multiple copies of transgenes reveals a novel arrangement for the λ5-VpreB1 locus control region. Molecular and Cellular Biology 19: 671-679).

### EXAMPLE 2

### ANALYSIS OF THE HUMAN HNRNP A2 GENE LOCUS

### Mapping the hnRNP A2 gene domain

The hnRNP A2 gene is composed of 12 exons spanning 10kb and is highly homologous to the hnRNP-A1 gene in its coding sequence and overall intron/exon structure indicating that it may have arisen by gene duplication (Biamonti et al., 1994). However, unlike the A1 gene no A2-specific pseudogenes have been found (Burd et al., 1989; Biamonti et al., 1994). In addition, the A1 and A2 genes are not genetically linked being on human chromosomes 12q13.1 (Saccone et al., 1992) and 7p15 (Biamonti et al., 1994) respectively. Figure 13A depicts a genetic map of the human hnRNP A2 locus present on the 160kb pCYPAC-2 derived clone MA160. This genomic fragment possesses 110kb 5' and 50kb of 3' flanking sequences. The DNA sequence of the 4.5 kb region upstream of the known transcriptional start site of the hnRNP-A2 was determined. This identified the position of the gene for the heterochromatin-associated protein HP1γ to be divergently transcribed from a position approximately 1-2 kb 5' of the hnRNP-A2 cap site (Figure 13C). Southern blot analysis indicates that the entire HP1γ gene is contained within a region of 10 kb (data not shown).

Therefore the TBP and hnRNP-A2 gene loci share the common feature of closely linked, divergently transcribed promotors.

The pattern of expression of the HP1γ gene within human tissues was assessed on a dot-blot prepared with poly(A)⁺-RNA derived from a wide range of human tissues and cell types. The results (Figure 38) show that the gene, like that for hnRNP-A2 is also ubiquitously expressed. The two genes can therefore be seen to form a ubiquitously expressed gene domain similar to that of the TBP locus.

### Functional analysis of the hnRNP A2 locus in transgenic mice

MA160 (Figure 13A) was used to generate transgenic mice. Southern blot analysis of the two founders that have bred through to the F1 stage has shown that these lines possess 1-2 copies of the transgene (data not shown).

A similar RT-PCR based assay to that used for TBP was used to analyse expression of the human hnRNP A2 transgene. The cDNA sequence of the murine hnRNP A2 is not known. Therefore, we could not select a region of homology between human and mouse hnRNP A2 by sequence comparison for RT-PCR amplification. We initially chose two primers Hn9 and Hn11, which correspond to sequences within exons 10 and 12 respectively of human hnRNP A2 (Figure 14A) and gives rise to an RT-PCR product of 270bp. However, we found that these two primers gave an identical sized product from both human and mouse RNA preparations (Figure 14B) indicating a region of homology between these two species. Tests with a range of restriction enzymes also revealed that *Hind*III is able to cut the murine (Figure 14B, lane HindIII M) but not the human (Figure 14B, lane HindIII H) product to give a fragment of 170bp.

Total RNA (1µg) prepared from various tissues of an F1 transgenic mice of line Hn35 and Hn55, were then analysed using the above method with ³²P-end labelled 5' Hn9 (Figure 16). PhosphorImager analysis was used to quantify the ratio of human to mouse RT-PCR products. The results (Figure 17A) show that reproducible, physiological levels of expression per transgene copy number are obtained in all tissue types analysed.

### Analysis of 60kb subclone of the hnRNP A2 locus in transgenic mice

The data obtained with the MA160 pCYPAC-derived clone indicate that this genomic fragment possesses a ubiquitous chromatin opening capability. In order to further define the location of the DNA region(s) responsible for this activity, transgenic mice were generated with a 60kb *Aat*II sub-fragment (Aa60) obtained from MA160 (Figure 13B). This fragment possesses 30kb 5' and 20kb 3' flanking sequences around the hnRNPA-2 gene.

Three transgenic mice (Aa7, Aa23 and Aa31) have been generated to date with the Aa60 fragment, two of which (Aa23 and 31) have bred through to establish lines. Estimated transgene copy numbers are: Aa7, 3; Aa23, 1-2; Aa31, 1-2).

Total RNA (1µg) from a range of tissues was analysed for transgene expression as described above. The results are shown in Figure 15 and quantified by PhosphorImager (Figure 17B). These data show that all transgenic mice express at a reproducible level per transgene copy number in all tissues analysed. This indicated that the ubiquitous chromatin opening capacity shown by MA160 is preserved on the Aa60 sub-fragment.

### Mapping of DNase I hypersensitive sites

The results of preliminary experiments to map DNase I HS sites over a 20-25kb region 5' of the transcriptional start point of the human hnRNP A2 gene are shown in Figure 18. A 766bp probe from exon 2 on a double restriction enzyme digest with *Aat*II and *Cla*I, gave a series of three HS sites (Figure 18, upper panel) corresponding to positions -1.1, -0.7 and - 0.1kb 5' of the hnRNP A2 gene (Figure 18, lower panel). We have also extended the analysis to 12-13 kb downstream of the transcriptional start of hnRNP-A2 and no further HS sites where identified.

As in the case of the TBP/C5 locus, these HS sites correspond to the 1-2kb region between the promoter of hnRNP A2 and the HP1H-γ gene. No LCR-type HS sites were detected indicating that the chromatin opening capacity of this locus is not associated with this type of element.

The data presented clearly show we have been able to obtain reproducible, ubiquitous, physiological levels of expression with two different gene loci (TBP and hnRNP A2) in all tissues of transgenic mice. This indicates that genetic control elements, not derived from an LCR, with a ubiquitous chromatin opening capability do indeed exist.

It is important to note that the data herein presented demonstrate a totally different function to the previously published results using promoter-enhancer combinations from other ubiquitously expressed genes such as human β-actin (e.g. see Ray, P. *et al.*, 1991; Yamashita *et al.*, 1993; Deprimo *et al.*, 1996), murine hydroxy-methylglutaryl CoA reductase (Mehtali *et al.*, 1990), murine adenosine deaminase (Winston *et al.*, 1992 and 1996), human ornithine decarboxylase (Halmekytö *et al.*, 1991) and murine phosphoglycerate kinase-1 (McBurney *et al.*, 1994). In these earlier studies high levels of expression were observed in only a subset of tissues and a chromatin opening function was not demonstrated or tested for.

In the case of the TBP gene, expression data from tissue culture cells (Figure 11) indicate that this ubiquitous chromatin opening capacity is contained within a 40kb genomic fragment with 12kb of 5' and 3' flanking sequences (pCP2-TSN, Figure 1a).

Transgenic mouse data with a 60kb fragment spanning the hnRNP A2 gene (Aa60; Figure 13B), indicate that the region with a ubiquitous chromatin opening capacity is contained on this fragment (Figures 15-17).

The only DNase I HS sites that have been mapped to these regions to date correspond to classical promoter rather than LCR-type elements. Therefore, the regions of DNA which act as ubiquitous chromatin opening elements (UCOEs) do not meet the definition of LCR elements which are associated with genes that are expressed in a tissue-specific or restricted manner. UCOEs and their activities can therefore clearly be distinguished from LCRs and LCR derived elements.

### Expression Vector Development

Sub-fragments of the 60kb RNP region are assayed for UCOE activity using reporter based assays.

Expression vectors containing sub-fragments located in the dual promoter region between RNP and HP1H-γ were designed using both GFP and a Neo^{R} reporter genes, as described above and as shown in Figure 22. These include a control vector with the RNP promoter driving GFP/Neo expression (RNP), a vector comprising the 5.5kb fragment upstream of the RNP promoter region and the RNP promoter (5.5RNP), vectors constructed using a splice acceptor strategy wherein the splice acceptor/branch consensus sequences (derived from exon 2 of the RNP gene) were cloned in front of the GFP gene (ensuring that the entire CpG island including sequences from RNP intron 1 can be tested in the same reporter-based assay), resulting in exon 1/part of intron 1 upstream of GFP (7.5RNP), carrying 7.5kb of the RNP gene preceeding the GFP gene, and a vector comprising the 1.5kb fragment upstream of the RNP promoter region and the RNP promoter (1.5RNP).

Expression vectors comprising the heterologous promoter CMV are also described above and are shown in Figure 23. These include control vectors with the CMV promoter driving GFP/Neo expression with an internal ribosome binding site (CMV-EGFP-IRES) and without an internal binding site (CMV-EGFP), a vector comprising the 5.5kb fragment upstream of the RNP promoter region and the CMV promoter driving GFP/Neo expression (5.5CMV), a vector comprising 4.0kb sequence encompassing the RNP and the HP1H-γ promoters and the CMV promoter driving GFP/Neo expression (4.0CMV), and a vector comprising 7.5kb sequences of the RNP gene including exon 1 and part of intron 1, and the CMV promoter driving GFP-Neo expression.

These constructs were transfected into CHO cells by electroporation, as described above. Addition of the 5.5kb region in front of the RNP promoter resulted in a 3.5-fold increase in number of G418^{R} colonies, Figure 24. Transfection of these same constructs into COS7 cells using a nucleic acid condensing peptide delivery strategy showed an increase in colony numbers closer to 7-fold (data not shown).

A 1.5-fold increase in colony numbers was also observed after transfection of the CMV-based vectors (i.e. CMV vs. 5.5CMV) into CHO cells, Figure 24.

Ring cloning of colonies from these transfections resulted in stable G418^{R} cell lines which could then be analysed for GFP expression levels. The FACS data is shown in Figure 25. Addition of the upstream sequences resulted in a 3.5-fold increase in GFP expression when assayed with the endogenous promoter (RNP vs 5.5 RNP). An increase in GFP expression is also seen with addition of the 5.5kb sequence in front of the heterologous CMV promoter (CMV vs 5.5CMV).

Extension of the constructs to include the entire methylation free island showed no increase in the number of G418^{R} colonies as compared with 5.5RNP, but there was an increase in the average median GFP fluorescence (5.5RNP cf. 7.5RNP; see Figure 26).

GFP expression of individual clones and restricted pools (approx. 100 colonies) were followed over time culturing the cells with/without G418 selection. Clones generated with the RNP promoter alone showed dramatic instability, with the percentage of GFP expressing cells rapidly decreasing over time. Clones expressing GFP from the 5.5RNP construct in comparison were stable for more than 3 months. Although CMV-GFP pools initially show better stability, after prolonged culturing in the absence of G418 a decrease in the number of GFP expressing cells was evident, in comparison to the 5.5CMV populations which remained completely stable. Figures 27 and 28 show FACs profiles of these populations clearly indicating a shift to the left i.e. an increasing proportion of nonfluorescent cells with the CMV-GFP construct. In contrast the 5.5CMV-GFP pools show a stable uniform peak of expression over time. The percentage of low or non-expressing cells is estimated from a gated population M1.

The studies on the RNP locus have narrowed in on a 5.5kb region covering the dual promoters of the RNP and HP1H-γ genes. Extension of this fragment in the 3' direction (7.5RNP or 8.5RNP) shows an enhancement in the level of gene expression and may relate to maintaining the methylation free islands intact. It has also been found that minimisation of the 5.5kb sequences to a 1.5kb region (1.5RNP, Figure 23) does not dramatically affect the outcome of reporter transfection studies, in terms of both the numbers of G418R colonies and expression as determined by FACs analysis (Figure 29). However, 1.5RNP does not confer the stability of gene expression as shown by 5.5RNP and 7.5RNP. Figure 30 shows the percentage of GFP expressing cells rapidly reduces over 68 days.

The construct 4.0CMV was designed so that the entire 4kb of sequence representing the CpG methylation free island remained intact. In addition, the cassette was inserted in front of CMV-EGFP (4.0CMV-EGFP-F (forward) and 4.0CMV-EGFP-R (reverse)) in both orientations. Figure 31 shows a dramatic enhancement (greater than 10-fold) of GFP median fluorescence, as compared to the standard CMV-GFP construct, CMV-EGFP. It is also shown that this boost of GFP expression occurs when the 4kb cassette is in both the forward and reverse orientations.

In terms of stability of gene expression, the vectors containing the upstream 5.5kb RNP sequences when transfected into CHO cells and followed over time show a definite advantage. Most importantly this stability is not only limited to the endogenous promoter but also confers a stability advantage to the heterologous and widely used CMV promoter.

Figure 32 shows CMV based constructs 4.0CMV and 7.5CMV with control vector CMV-EGFP transfected into CHO cells and analysed at day 13 post-transfection following G418 selection. A substantial increase (15-20 fold) in median fluorescence can be seen by adding the 4.0 or the 7.5kb fragments from the RNP locus in front of the CMV promoter. This increase was independent of the orientation of the fragment (data not shown).

Figure 33 shows the percentage of GFP expressing cells in the same G418 selected pools as in Figure 32. It can be seen that inclusion of the 4.0 and the 7.5 kb fragments enhances the percentage of GFP positive cells in the G418 selected population. In addition, the populations appear relatively stable over time, although from previous experiments it was evident that CMV-EGFP instability is only apparent after approximately 60 days in culture.

Figure 34 shows colony numbers after transfection if CHO cells with equivalent molar amounts of various constructs. The 7.5CMV constructs show approximately 2.5-fold more colonies than the control vector CMV-EGFP. These observations are consistent with 7.5CMV-F ensuring an enhanced number of productive integration events and therefore with there being a chromatin opening/maintaining capacity to the 7.5kb fragment.

### Adenovirus vector containing a UCOE

At the present time adenovirus (Ad) is the vector system giving the most efficient delivery of genes to many cell types of interest for gene therapy. Many of the most promising gene therapies in clinical development use this vector system, notably vectors derived from Ad subtype 5. The utility of Ad for human gene therapy could be substantially increased by improving expression of the therapeutic genes in two main ways. The first involves increasing the level of transgene expression in order to obtain the maximum effect with the minimum dose, and this applies whichever promoter is used. The second involves improving tissue specific or tumour-specific promoters, such that they retain specificity but give stronger expression in the permissive cells. Although several promoters giving good specificity for particular tissues or tumour types are known, the level of expression they give in the permissive cells is generally too weak to be of real therapeutic benefit. An example of this is the promoter of the mouse alpha-foetoprotein (AFP) gene, which gives expression that is weak but very specific for hepatoma (liver cancer) cells (Bui et al, 1997, Human Gene Therapy, 8, 2173-2182). Such tumour-specific promoters are of particular interest for Gene-Directed Enzyme Prodrug Therapy (GDEPT) for cancer, which exploits gene delivery to accomplish targeted chemotherapy. In GDEPT a gene encoding a prodrug converting enzyme is delivered to tumour cells, for example by injecting the delivery vector into tumours. Subsequent administration of a relatively harmless prodrug converts this into a potent cytotoxic drug which kills the cells expressing the enzyme *in situ*. An example concerns the enzyme nitroreductase (NTR) and the prodrug CB 1954 (Bridgewater et al, 1995, Eur. J. Cancer, 31A, 2362-2370). Adenovirus vectors give the most efficient delivery of genes encoding such enzymes, for example by direct injection into tumours.

### Construction of an Ad expressing NTR from the AFP promoter and a UCOE.

A recombinant type 5 adenovirus vector was made which expresses the NTR gene from the AFP promoter preceded by the 4kb RNP UCOE (the sequence of Figure 20 between nucleotides 4102 and 8286). The 4kb UCOE was first cloned as a Pme1 fragment into pTXO379, an intermediate vector which carries the NTR gene preceded by the AFP promoter (Bui et al, 1997, Human Gene Therapy, 8, 2173-2182) and flanked by Ad5 sequences (1-359, 3525-10589), by blunt end ligation into the Cla1 site located 5' to the AFP promoter. Restriction digestion was used to confirm the presence of a single UCOE copy and to establish the orientation of the UCOE. A recombinant Ad construct was then generated using the plasmid pTXO384 which contains the UCOE fragment in reverse orientation and the Ad packaging cell line Per.C6, which was developed and supplied by Introgene (Fallaux et al, 1998, Human Gene Therapy, 9, 1909-1917). The procedure supplied by Introgene was used for viral rescue. Essentially pTXO384 was linearised with Swa1 and co-transfected into

Per.C6 cells with Swal-linearised backbone vector pPS1160, which carries the right end of Ad5 and a region of overlap with pTXO384 such that a recombinant Ad is generated by homologous recombination. Virus produced by homologous recombination in the transfected cells was pooled and designated CTL208.

### NTR expression in cell lines in vitro

Larger scale virus preparations were made using standard procedures for CTL208, and two other recombinant Ad viruses. These were CTL203, which carries the NTR gene preceded by the AFP promoter and minimal enhancer but no UCOE fragment, and CTL102 which carries the NTR gene preceded by the CMV promoter. The CMV promoter is commonly used in recombinant Ad vectors to give strong expression in a wide range of tissue and tumour types. CTL203 and CTL102 share the same Ad5 backbone as CTL208 and were identical to it except in the elements used for transcription of the NTR gene.

CTL203, 208 and 102 were then used to transduce two cell lines *in vitro* to investigate the level and specificity of NTR expression. These were the primary human hepatoma cell line HepG2 which expresses AFP, and KLN205, a mouse squamous cell carcinoma line which does not express AFP. Exponentially growing cells were harvested from tissue culture plates by brief trypsinisation, resuspended in infection medium at 1.25x10⁴ viable cells/ml and plated into 6 well plates. The viruses were added to the wells before attachment at a multiplicity of 50, and for CTL203 at multiplicities of 100 and 500 also. After 90 mins the foetal calf serum concentration was adjusted to 10% and the cells incubated for a total of 24 hours. Cell lysates were made from the infected cells by hypotonic lysis, then cell debris cleared by centrifugation in eppendorf tubes. An ELISA was performed to quantify the NTR protein in the supernatants. This involved coating Nunc-Immuno Maxisorp Assay Plates with recombinant NTR, adding 50 1 of each hypotonic lysate per well in duplicate and incubating overnight at 4°C. The samples were then washed 3X with 0.5% Tween in PBS and incubated with a sheep anti-NTR polyclonal antiserum (100 1 per well of a 1 in 2000 dilution in PBS/Tween for 30 mins at room temperature. After washing off excess primary antibody HRP-conjugated secondary antibody was applied, this being donkey antisheep (100 1 per well of 1 in 5000 in PBS/Tween). After a further 30 min incubation the samples were washed with PBS before development with 100 1 per well of TMB substrate (1ml TMB solution, 1mg/ml in DMSO + 9ml of 0.05M phosphate-citrate buffer + 2µl of 30%v/v H₂O₂ per 10ml) for 10 mins at room temperature. The reactions were stopped by addition of 25µl of 2M H₂SO₄ per well and read at 450nm using a plate reader.

Figure 46 shows the results of these ELISAs. It shows that CTL203, with NTR expressed from the AFP promoter/enhancer, gave weak but specific NTR expression, detectable only in the AFP positive cell line. CTL102 (with NTR expressed from the CMV promoter) gave much higher and non-specific expression, with very similar levels of NTR in both cell lines. Strikingly, AFP positive HepG2 cells infected with CTL208 (UCOE + AFP promoter driving expression of NTR) expressed NTR at a higher level then CTL102 infected cells, whereas CTL208 infected AFP negative KLN205 cells expressed significantly less NTR than those infected with CTL102. These data show that the UCOE dramatically enhances expression in the context of Ad, with partial retention of specificity.

### NTR expression and anti-tumour effects in vivo

Tumour-specific promoters are preferable to non-specific promoters for cancer gene therapy from the safety viewpoint, because they will give lower expression of the transgene in normal tissues. This is particularly important for Ad-based gene therapies because after injection into tumours some of the virus tends to escape from the tumour and following systemic dissemination tends to transduce normal tissues. In particular Ad gives very efficient transduction of liver cells, such that liver damage is usually the dose-limiting toxicity for Ad gene therapies. In the case of GDEPT the use of strong promoters able to give expression in normal tissues, such as the CMV promoter, can lead to killing of normal liver cells expressing NTR. This problem can potentially be avoided or minimised using tumour-specific promoters, which would be advantageous providing these give sufficiently strong expression in the tumour cells to give anti-tumour effects. CTL208 was therefore compared to CTL102 for NTR gene expression in tumour cells and liver cells following injection into tumours in mice, and for anti-tumour effects. The congenitally athymic nude mouse strain BALB/c nu/nu was used. The mice were males free of specifc pathogens, aged eight to twelve weeks at the commencement of the experiments, and maintained in microisolator cages equipped with filter tops. Exponentially growing HepG2 cells cultured *in vitro* were used as tumour inocula. The cells were cultured in shake flasks, harvested by trypsinisation and centrifugation for 5 min at 800 g, washed and resuspended in sterile saline solution. Cell viability was estimated by trypan blue dye exclusion, and only single cell suspensions of greater than 90% viability were used. Mice were injected subcutaneously in the flank with 2-5x10⁶ cells, under general anaesthesia, induced by intraperitoneal injection of 0.2 ml of a xylizine (Chanelle Animal Health Ltd, Liverpool, UK) and ketamine (Willows Francis Veterinary, Crawley, UK) mixture at a concentration of 1 mg/ml and 10 mg/ml respectively. In the first experiment CTL102 or CTL208 were injected into sub-cutaneous HepG2 tumours of size 25-60mm² (size expressed as surface area determined by multiplying the longest diameter with its greatest perpendicular diameter, length x width=mm²) growing in nude mice. Single doses of 7.5x10⁹ particles were used for each virus. The animals were sacrificed 48 hours later, their tumours and livers excised, fixed in buffered 4% formalin/PBS for 24 hours and processed for paraffinembedding and sectioning using standard protocols. Serial 3µm sections were cut and immunostained to detect cells expressing NTR by indirect immunoperoxidase staining using a sheep anti-NTR antiserum (Polyclonal Antibodies Ltd) and VECTASTAIN Elite ABC kit (Vector Labs). These histological sections were examined using standard microscopic equipment and the percentage of cells expressing NTR in the entire livers and tumours were estimated by microscopy. Figure 47 shows the results for each mouse. It demonstrates that the UCOE in combination with the (otherwise weak) AFP promoter gives strong NTR expression in AFP positive tumours in mice, such that on average CTL208 gives very similar numbers of tumour cells expressing NTR at detectable levels as CTL102 following injection into tumours. Intra-tumoral injection of CTL102, however, led to NTR expression detectable in the liver for 5 out of 6 animals for CTL102, but 0 out of 6 for CTL208. This result confirms that in CTL208 the UCOE-AFP promoter combination gives expression in AFP positive tumour cells similar to or stronger than the CMV promoter, but shows much less expression in (AFP negative) normal tissues.

To confirm that the UCOE elevates expression from the AFP promoter to therapeutically useful levels CTL208 and CTL102 were compared for their ability to confer anti-tumour effects in combination with the prodrug CB1954. Nude mice bearing sub-cutaneous HepG2 tumours of size 25 to 60 mm² were given single injections of CTL102 or CTL208, at doses of either 7.5x10⁹ or 2x10¹⁰ particles. 48 hours later CB1954 administration to the mice commenced. CB1954 (Oxford Asymmetry, Oxford, UK) was dissolved in DMSO (Sigma, St Louis, Mo, USA) to give a concentration of 20 mg/ml. Immediately prior to dosing this solution was diluted 1:5 in sterile saline solution to give a final concentration of 4 mg/ml. Mice received five equal daily doses intraperitoneally without anaesthesia. For a control group of mice the tumours were injected with PBS instead of virus 48 hours before commencing prodrug administration. Tumour size was measured daily using vernier calipers for the next 27 days. Figure 48 shows the results. For the control group given CB 1954 and neither virus, 7/7 tumours continued to grow rapidly. Tumour regressions were observed in some of the mice in all the groups given both NTR expressing virus and CB1954. With CTL102 regressions were observed in 3/8 mice given the lower dose, and 4/8 mice given the higher dose. With CTL208 regressions were observed in 5/8 and 6/8 mice respectively. These results confirm that, in CTL208, the UCOE elevates NTR expression from the AFP promoter in permissive tumour cells to levels which exceed those given by the strong CMV promoter and this results in a superior anti-tumour effect in a mouse model of the clinical situation for GDEPT.

These results demonstrate two important and useful properties of the UCOE. First, it substantially improves expression in the context of Ad, a non-integrating vector of great potential in gene therapy. Second, it elevates expression from weak but specific promoters to much more useful levels with retention of useful specificity.

### FISH Analysis

Copy number was determined in 31 16 RNP-EGFP clones in mouse Ltk cells. Due to the low amount of DNA used in the transfection (0.5-1.0 µg), the percentage of single copy clones was very high (83%). Moreover, EGFP expression varied more than two-fold within the single copy clones, indicating that the transgene was susceptible to positive and negative position effects. Nonetheless, three single copy clones had integrated in centromeric heterochromatin (Figure 42), indicating that this construct is able to open chromatin. Clones F1 and G6 showed the 16RNP-EGFP transgene had integrated in one of the centromeres of metacentric chromosomes originated by Robertsonian translocations (Figure B, C), whereas in clone I3, integration had occurred in the centromere of a typical mouse acrocentric chromosome (Figure D).

### Expression of Erythropoietin (EPO) In Vectors CET300 and CET301

### Construction of EPO expression vectors CET300 and CET301.

The erythropoietin (EPO) coding sequence was amplified by polymerase chain reaction (PCR) from a human fetal liver Quick-Clone™ cDNA library (Clontech, Palo Alto, US.) using primers EP2 (5'-CAGGTCGCTGAGGGAC-3') and EP4 (5'-CTCGACGGGGTTCAGG-3'). The resulting 705 bp product, which included the entire open reading frame, was subcloned into the vector pCR3.1 using the Eukaryotic TA cloning kit (Invitrogen, Groningen, The Netherlands), to create the vector pCR-EPO. The EPO sequence was verified by automated DNA sequencing on both strands. A 790 bp *Nhe*I-*Eco*RV fragment, containing the EPO coding sequence, was excised from pCR-EPO and subcloned between the *Nhe*I and *Pme*I sites of the vectors CET200 and CET201 (containing the 7.5 kb RNP fragments in the forward and reverse orientations respectively), to generate the vectors CET300 and CET301 respectively. A control vector, pCMV-EPO, was generated by excising the EGFP coding sequence from pEGFP-N1 as a *Nhe*I-*Not*I fragment and replacing it with a *Nhe*I-*Not*I fragment from pCR-EPO containing the EPO coding sequence.

### Expression of erythropoietin in CHO cells.

Plasmids CET300, CET301 and pCMV-EPO were linearised using the restriction endonuclease *Dra*III. Restricted DNA was then purified by extraction with phenolchloroform followed by ethanol precipitation. DNA was resuspended in sterile water and equimolar amounts of the plasmids were electroporated into CHO cells. Viable cells were plated in 225 cm³ culture flasks and stable transfected cells were selected by replacing the medium after 24 hrs for complete medium containing 0.6 mg/ml G418. Cells were grown in this medium until G418-resistant colonies were present (about 10 days after electroporation). The flasks were then stripped and cells were seeded at 10⁶ cells/well in a 6 well dish containing 1ml of complete medium. After 48 hrs the medium was removed and the levels of erythropoietin in the media were quantitated by enzyme linked immunosorbent assay (ELISA) using a Quantikine^{®} IVD^{®} Human EPO immunoassay kit (R & D systems, Minneapolis, US). The levels of EPO produced by the constructs CET300, CET301 and pCMV-EPO were 1780 U/ml, 1040 U/ml and 128 U/ml respectively (Figure 40). Therefore, constructs CET300 and CET301, containing the 7.5 kb RNP fragment in forward and reverse orientations, produced EPO in the above experiment at levels approximately 14-fold and 8-fold higher, respectively, than the control plasmid pCMV-EPO which contains the strong ubiquitous CMV promoter to drive expression of EPO.

### GFP expression in Hela cells transfected with EBV reporter constructs with or without the 16kb UCOE fragment of hnRNPA2.

In the initial experiment with cells maintained on hygromycin selection, the RNP16 UCOE-containing construct (p220.RNP16) gave high level, homogeneous expression of EGFP by day 23, whereas a more heterogeneous pattern of EGFP expression was observed with p220.EGFP (construct without the UCOE). EGFP expression in the p220.EGFP-transfected pools was gradually lost, whereas expression remained stable for 160 days with the p220.RNP16-transfected pools.

Three repeat experiments demonstrated the same pattern of high level, homogeneous EGFP expression in p220.RNP16-transfected pools, with heterogeneous expression again observed in the p220.EGFP-transfected pools. As with the initial experiment, the expression of EGFP was stable with the RNP 16 UCOE and was unstable without the UCOE, with expression dropping dramatically by 30-40 days (Figure 43).

A further experiment was performed wherein hygromycin selection was removed at day 27. The results show that even without selection EGFP expression is stable with the RNP16 UCOE and was unstable without the UCOE (Figure 44).

### EXAMPLE 3

### Plasmid Containing A UCOE

Figure 50 shows the constructs generated and fragments used in comparison to the hnRNPA2 endogenous genomic locus.

Figure 51 shows a graph of the FACs analysis with median fluorescence of the transiently transfected HeLa populations. The cells were transfected using the CL22 peptide condensed reporter plasmids as indicated above. It can be seen that the duration of expression of the control CMV-GFP reporter construct is short-lived and dramatically decreases from 24 to 48 hours post-transfection.

In contrast to the control, the UCOE containing plasmid 7.5CMV-F continues to show significant GFP expression over an extended period of time, at least 9 days post-transfection. In repeat experiments GFP expression can be seen at 14 days post-transfection.

Figure 52 shows representative low magnification field of views of the transiently transfected HeLa cell populations. The data correlates with the FACs analyses and enables the cells to be visibly followed over a similar time-course. At 24 hours post-transfection significant numbers of GFP positive cells are visible in both the control CMV-GFP and 7.5CMV transient populations (Figure 52 A and B). In fact it can be seen that at 24 hours there were more GFP positive cells in the control population than in the 7.5CMV transfected population. This is due to the fact that the quantity of input DNA in both cases was not gene dosage corrected, resulting in significantly more copies of the control plasmid per transfection. However, at 6 days post-transfection there were very few if any positive fluorescent cells left in the CMV-EGFP control population (Figure 52C). In contrast 6 days post-transfection the 7.5CMV transfected Hela cells continued to show significant numbers of GFP expressing cells (Figure 52D). In fact even 14 days after transfection positively fluorescing cells could easily be detected (data not shown).

Total DNA was recovered from various time points throughout the experiment, linearised, run on a gel and blotted (see Matetrials and Methods). Interestingly at day 6 even in the control population of cells where little or no expression of GFP was detected, the plasmid could be readily detected in an unintegrated state (data not shown). This would suggest that the rapid loss in gene expression seen with the CMV-GFP control plasmid is not due to chronic loss of the plasmid template but rather to a mechanism of chromatin shut-down of gene expression.

### Transient Transfection of CHO cells with erythropoietin expression vectors.

Supercoiled forms of plasmids CET300, CET301 and CMV-EPO were electroporated into CHO cells using standard conditions (975µF, 250V). Viable cells were then seeded at 10⁶ cells in a 6-well dish containing 1 ml of complete CHO medium. The medium was then removed at 24 hr intervals and replaced with 1 ml of fresh medium. Media samples were collected in this fashion for 9 days and erythropoietin levels were then quantitated by ELISA using a Quantikine^{®} IVD^{®} Human EPO immunoassay kit (R & D systems, Minneapolis, US). The attached figure shows a time course of erythropoietin expression by cells transfected with CET300, CET301 and CMV-EPO plasmids. Erythropoietin expression continued to rise for 48 hrs in all cell populations. Thereafter, erythropoietin expression by cells transfected with CMV-EPO fell on a daily basis. Whereas, levels of EPO expression by cells transfected with CET300 or CET301 continued to rise throughout the 9-day period (Figure45).

### REFERENCES

Altschul, S. F., Madden, T. L., Schäffer, J. Z., Zhang, Z., Miller, W., and Lipman, D. J. (1997). Gapped BLAST and PSI-BLAST: a new generation of protein database search programs. Nucleic Acids Res. 25: 3389-3402.
Antoniou, M. (1991). Induction of erythroid-specific expression in murine erythroleukaemia (MEL) cell lines. In: Methods in Molecular Biology, Vol. 7: Gene Transfer and Expression Protocols. Ed. E. J. Murray, Humana Press Inc., Clifton, NJ, U.S.A. pp.421-434.
Antoniou, M. and Grosveld, F. (1990). The β-globin gene dominant control region interacts differently with distal and proximal promoter elements. Genes Dev. 4: 1007-1012.
Archer, T.K., Lefebvre, P., Wolford, R.G. and Hager, G.L. (1992) Transcription factor loading on the MMTV promoter: a bimodal mechanism for promoter activation. Science 255: 1573-1576.
Aronow, B.J., Ebert, C.A., Valerius, M.T., Potter, S.S., Wiginton, D.A., Witte, D.P. and Hutton, J.J. (1995) Dissecting a Locus Control Region: Facilitation of Enhancer Function by Extended Enhancer-Flanking Sequences. Mol. Cell. Biol. 15: 1123-1135.
Auffray, C., and Rougeon, F. (1980). Purification of mouse immunoglobulin heavy-chain RNAs from total myeloma tumor RNA. Eur. J. Biochem. 107: 303-324.
Biamonti G, Ruggiu M, Saccone S, Della Valle G, Riva S (1994) Two homologous genes, originated by duplication, encode the human hnRNP proteins A2 and A1. Nucl. Acids Res. 22: 1996-2002.
Bimboim, H. C., and Dolly, J. (1979). A rapid alkaline extraction procedure for screening recombinant plasmid DNA. Nucleic Acids Res. 7: 1513.
Blom van Assendelft, G., Hanscombe, O., Grosveld, F., and Greaves, D.R. (1989). The β-globin dominant control region activates homologous and heterologous promoters in a tissue-specific manner. Cell 56: 969-977.
Bonifer C, Vidal M, Grosveld F, Sippel AE (1990) Tissue specific and position independent expression of the complete gene domain for chicken lysozyme in transgenic mice. EMBO J. 9: 2843-2848.
Bonifer, C., Yannoutsos, N., Grosveld, G. and Sippel, A.E. (1994) Dissection of the locus control function located on the chicken lysozyme gene domain in transgenic mice. Nucleic Acids Res. 22: 4202-4210.
Brines RD and Klaus GG (1993) Polyclonal activation of immature B cells by preactivated T cells: the role of IL-4 and CD40 ligand. Int Immunol 5: 1445-1450.
Burd CG, Swanson MS, Gorlach M, Dreyfuss G (1989) Primary structures of the heterogeneous nuclear ribonucleoprotein A2, B1, and C2 proteins: a diversity of RNA binding proteins is generated by small peptide inserts. Proc. Natl. Acad. Sci. USA 86: 9788-9792.
Carson, S. and Wiles, M. V. (1993). Far upstream regions of class II MHC Ea are necessary for position-independent, copy-dependent expression of Ea transgene. Nucl. Acids Res. 21: 2065-2072.
Chalut, C., Gallois, Y., Poterszman, A., Moncollin, V., and Egly, J.-M. (1995). Genomic structure of the human TATA-box-binding protein (TBP). Gene 161: 277-282.
Chu, G., Hayakawa, H., and Berg, P. (1987). Electroporation for the efficient transfection of mammalian cells with DNA. Nucleic Acids Res. 15: 1311-1326.
Church, G. M., and Gilbert, W. (1984). Genomic sequencing. Proc. Natl. Acad. Sci. USA 81: 1991-1995.
Collis, P., Antoniou, M. and Grosveld, F. (1990). Definition of the minimal requirements within the human β-globin gene and the dominant control region for high level expression. EMBO J. 9: 233-240.
Cooper, M.J. and Miron, S., 1993, Efficient episomal expression vector for human transitional carcinoma cells, Hum. Gene Ther. 4: 557-566.
Dai, Y., Roman, M., Naviaux, R. K. and Verma, I. M. (1992) Gene Therapy via primary myoblasts: long-term expression of factor IX protein following transplantation in vivo. Proc. Natl. Acad. Sci. USA 89: 10892-10895.
De Benedetti, A. and Rhoads, R.E., 1991, A novel BK virus-based episomal vector for expression of foreign genes in mammalian cells, Nucl. Acids Res., 19: 1925-1931.
Deprimo, S.E., Stambrook, P.J. and Stringer, J.R. (1996) Human placental alkaline phosphatase as a histochemical marker of gene expression in transgenic mice. Transgenic Res. 5: 459-466.
Diaz, P., Cado, D. and Winoto, A. (1994). A locus control region in the T cell receptor α/δ locus. Immunity 1: 207-217.
Dillon, N., and Grosveld, F. (1993). Transcriptional analysis using transgenic animals. In Gene Transcription: A practical approach, B. D. Hames and S. J. Higgins, eds. (Oxford: IRL Press), pp. 153-188.
Dillon, N. and Grosveld, F. (1994). Chromatin domains as potential units of eukaryotic gene function. Curr. Opin. Genet. Develop. 4: 260-264.
Dillon, N., Trimbom, T., Strouboulis, J., Fraser, P. and Grosveld, F. (1997) The effect of distance on long-range chromatin interactions. Mol. Cell 1: 131-139.
Earle, W. R., Schilling, E. L., Stark, T. H., Straus, N. P., Brown, M. F., and Shelton, E. (1943). Production of malignancy in vitro. IV. The mouse fibroblast cultures and changes seen in the living cells. J. Natl. Cancer Inst. 4: 165-212.
Ellis, J., Tan-Un, K.C., Harper, A., Michalovich, D., Yannoutsos, N., Philipsen, S. and Grosveld, F. (1996). A dominant chromatin-opening activity in 5' hypersensitive site 3 of the human β-globin locus control region. EMBO J. 15: 562-568.
Festenstein, R., Tolaini, M., Corbella, P., Mamalaki, C., Parrington, J., Fox, M., Miliou, A., Jones, M and Kioussis, D. (1996). Locus control region function and heterochromatininduced position effect variegation. Science 271: 1123-1125.
Flotte, T.R. and Carter, B.J. (1995) Adeno-associated virus vectors for gene therapy. Gene Ther. 2: 357-362.
Foulds, C.E. and Hawley, D.K. (1997) Analysis of the human TATA binding protein promoter and identification of an ets site critical for activity. Nucl. Acids Res. 25: 2485-2494,
Forrester, W. C., Takegawa, S., Papayannopouplou, T., Stamatoyannopoulos, G., and Groudine, M. (1987). Evidence for a locus activation region: the formation of developmentally stable hypersensitive sites in globin-expressing hybrids. Nucleic Acids Res. 15: 10159-10177.
Freshney, R. I. (1994). In Culture of animal cells: a manual of basic techniques (New York: Wiley-Liss, Inc.), pp. 169-171.
Gavalas, A. and Zalkin, H. (1995) Analysis of the chicken GPAT/AIRC bidirectional promoter for de novo purine nucleotide synthesis. J. Biol. Chem. 270: 2403-2410.
Gilliland, G., Perrin, S., Blanchard, K., and Bunn, H. F. (1990). Analysis of cytokine mRNA and DNA: Detection and quantitation by competitive polymerase chain reaction. Proc. Natl. Acad. Sci. USA 87: 2725-2729.
Greaves, D. R., Wilson, F. D., Lang, G. and Kioussis, D. (1989). Human CD2 3' flanking sequences confer high-level, T cell specific, position-independent gene expression in transgenic mice. Cell 56: 979-986.
Grosveld, F., Blom van Assendelft, G. B., Greaves, D. R. and Kollias, G. (1987). Position-independent high level expression of the human β-globin gene in transgenic mice. Cell 51: 975-985.
Grosveld, F., Dillon, N. and Higgs, D.R. (1993) The regulation of human globin gene expression. Baillieres Clin. Haematol. 6: 31-55.
Hammekytö, M., Alhonen, L., Wahlfors, J., Sinervirta, R., Janne, O. A., and Janne, J. (1991). Position-independent, abberant expression of the human ornithine decarboxylase gene in transgenic mice. Biochem. Biophys. Res. Comm. 180: 262-267.
Hanscombe, O., Whyatt, D., Fraser, P., Yannoutsos, N., Greaves, D., Dillon, N. and Grosveld, F. (1991) Importance of globin gene order for correct developmental expression. Genes Dev. 5: 1387-1394.
Hartman, P. S. (1991). Transillumination can profoundly reduce transformation frequencies. BioTechniques 11: 747-748.
Heng HH, Xiao H, Shi XM, Greenblatt J, Tsui LC (1994) Genes encoding general initiation factors for RNA polymerase II transcription are dispersed in the human genome. Hum Mol Genet. 3: 61-64.
Hong, N.A., Cado, D., Mitchell, J., Ortiz, B.D., Hsieh, S.N. and Winoto, A. (1997) A targeted mutation at the T cell receptor α locus impairs T cell development and reveals the presence of the nearby anti-apoptosis gene Dad-1. Mol. Cell. Biol. 17: 2151-2157.
Ioannou, P. A., Amemiya, C. T., Gamer, J., Kroisel, P. M., Shizuya, H., Chen, C., Batzer, M. A., and de Jong, P. J. (1994). A new bacteriophage P1-derrived vector for the propagation of large human DNA fragments. Nat. Genet. 6: 84-89.
Jarman, A.P., Wood, W.G., Sharpe, J.A., Gourdon, G., Ayyub, H. and Higgs, D.R. (1991) Characterization of the major regulatory element upstream of the human alpha-globin gene cluster. Mol. Cell. Biol. 11: 4679-4689.
Jones, B.K., Monks, B.R., Liebhaber, S.A. and Cooke, N.E. (1995) The Human Growth Hormone Gene is Regulated by a Multicomponent Locus Control Region. Mol. Cell. Biol. 15: 7010-7021.
Kaufman, R.J. (1990) Methods in Enzymology 185: 537-566.
Kolsto A.-B., Kollias, G., Giguere, V., Isobe, K.-I., Prydz, H. and Grosveld, F. (1986) The maintenance of methylation-free islands in transgenic mice. Nucl. Acids Res. 14: 9667-9678
Kotin, RM (1994) Prosspects for the use of adeno-associated virus as a vector for human gene therapy. Hum. Gene Ther. 5: 793-801.
Kozu T, Henrich B, Schafer KP (1995) Structure and expression of the gene (HNRPA2B1) encoding the human hnRNP protein A2/B1. Genomics 25: 365-371.
Lake, R.A., Wotton, D. and Owen, M.J. (1990) A 3' transcriptional enhancer regulates tissue-specific expression of the human CD2 gene. EMBO J. 9: 3129-3136.
Lang, G., Wotton, D., Owen, M.J., Sewell, W.A., Brown, M.H., Mason, D.Y., Crumpton, M.J. and Kioussis, D. (1988) The structure of the human CD2 gene and its expression in transgenic mice. EMBO J. 7: 1675-1682.
Larsen, F., Gundersen, G., Lopez, R., and Prydz, H. (1992). CpG islands as gene markers in the human genome. Genomics 13: 1095-1107.
Lee CC, Pons F, Jones PG, Bies RD, Schlang AM, Leger JJ, Caskey CT (1993) Mdx transgenic mouse: restoration of recombinant dystrophin to the dystrophic muscle. Hum. Gene Ther. 4: 273-287.
Lennon, G. G., Auffray, C., Polymeropoulos, M., and Soares, M. B. (1996). The I.M.A.G.E. Consortium: An intergrated molecular analysis of genomes and their expression. Genomics 33: 151-152.
Lozzio, C. B., and Lozzio, B. B. (1975). Human chronic myelogenous leukemia cell-line with positive Philadelphia chromosome. Blood 45: 321-334.
McBurney, M.W., Staines, W.A., Boekelheide, K., Parry, D., Jardine, K. and Pickavance, L. (1994) Murine PGK-1 promoter drives widespread but not uniform expression in transgenci mice. Devel. Dynam. 200: 278-293.
Mehtali, M., LeMeur, M. and Lathe, R. (1990) The methylation-free status of a housekeeping transgene is lost at high copy number. Gene 91: 179-184.
Yeoman H and Mellor AL (1992) Tolerance and MHC restriction in transgenic mice expressing a MHC class I gene in erythroid cells. Int Immunol 4: 59-65.
Michelsen, B. K. (1995). Transformation of Escherichia coli increases 260-fold upon inactivation of T4 DNA ligase. Anal. Biochem. 225: 172-174.
Miller, A.D. (1992) Retroviral vectors. Curr. Top. Microbiol. Immunol. 158: 1-24.
Miller, A.D., Miller, D.G., Garcia, J.V. and Lynch, C.M. (1993) Use of retroviral vectors for gene transfer and expression. Meth. Enzymol. 217: 581-599.
Milot, E., Strouboulis, J., Trimbom, T., Wijgerde, M., de Boer, E., Langeveld, A., Tan-Un, K., Vergeer, W., Yannoutsos, N., Grosveld, F. and Fraser, P. (1996). Heterochromatin effects on the frequency and duration of LCR-mediated gene transcription. Cell 87: 105-114.
Montoliu, L., Umland, T. and Schütz, G. (1996). A locus control region at -12 kb of the tyrosinase gene. EMBO J. 15: 6026-6034.
Muzyczka, N. (1992) Use of adeno-associated virus as a general transduction vector for mammalian cells, Curr. Top. Microbiol. Immunol., 158: 97-129.
Needham, M., Egerton, M., Millest, A., Evans, S., Popplewell, M., Cerillo, G., McPheat, J., Monk, A., Jack, A., Johnstone, D. and Hollis, M. (1995). Further development of the locus control region/murine erythroleukemia expression system: high level expression and characterisation of recombinant human calcitonin receptor. Protein Expression and Purification 6: 124-131.
Needham, M., Gooding, C., Hudson, K., Antoniou, M., Grosveld, F. and Hollis, M. (1992). LCR/MEL: A versatile system for high-level expression of heterologous proteins in erythroid cells. Nucl. Acids Res. 20: 997-1003.
Ogilvy, S., Elefanty, A. G., Visvader, J., Bath, M. L., Harris, A. W., and Adams, J. M. (1998). Transcriptional regulation of vav, a gene expressed throughout the hematopoietic compartment. Blood 91: 419-430.
Ortiz, B.D., Cado, D., Chen, V., Diaz, P.W. and Winoto, A. (1997) Adjacent DNA elements dominantly restrict the ubiquitous activity of a novel chromatin-opening region to specific tissues. EMBO J. 16: 5037-5045.
Peterson, M. G., Tanese, N., Pugh, B. F., and Tijan, R. (1990). Functional domains and upstream activation properties of cloned human TATA binding protein. Science 248: 1625-1630.
Philipsen, S., Talbot, D., Fraser, P. and Grosveld, F. (1990) The β-globin dominant control region: hypersensitive site 2, EMBO J., 9: 2159-2167.
Piirsoo, M., Ustav, E., Mandel, T., Stenlund, A. and Ustav, M. (1996) Cis and trans requirements for stable episomal maintenance of the BPV-1 replicator. EMBO J. 15: 1-11.
Pruzina, S., Hanscombe, O., Whyatt, D., Grosveld, F. and Philipsen, S. (1991) Hypersensitive site 4 of the human β-globin locus control region, Nucl. Acids Res., 19: 1413-1419.
Raguz, S., Hobbs, C., Yagüe, E., Ioannou, P.A., Walsh, F.S. and Antoniou, M. (1998) Muscle-specific locus control region activity associated with the human desmin gene. Develop. Biol. in press.
Ray P, Higgins KM, Tan JC, Chu TY, Yee NS, Nguyen H, Lacy E, Besmer P (1991) Ectopic expression of a c-kitW42 minigene in transgenic mice: recapitulation of W phenotypes and evidence for c-kit function in melanoblast progenitors. Genes Dev. 5: 2265-2273,
Reeves, R., Gorman, C.M. and Howard, B. (1985) Minichromosome assembly of nonintegrated plasmid DNA transfected into mammalian cells. Nucl. Acids Res. 13: 3599-3615.
Reitmann, M., Lee, E., Westphal, H., and Felsenfeld, G. (1993). An enhancer / locus control region is not sufficient to open chromatin. Mol. Cell. Biol. 13: 3990-3998.
Saccone S, Biamonti G, Maugeri S, Bassi MT, Bunone G, Riva S, Della Valle G (1992) Assignment of the human heterogeneous nuclear ribonucleoprotein A1 gene (HNRPA1) to chromosome 12q13.1 by cDNA competitive in situ hybridization. Genomics 12: 171-174.
Sambrook, J., Fritsch, E. F., and Maniatis, T. (1989). Molecular Cloning: A Laboratory Manual (Cold Spring Harbor, NY: Cold Spring Harbor Laboratory Press).
Smith, C.L., Archer, T.K., Hamlin-Green, G. and Hager, G.L. (1993) Newly expressed progesterone receptor cannot activate stable, replicated mouse mammary tumor virus templates but acquires transactivation potential upon continuous expression. Proc. Natl. Acad. Sci. USA 90: 11202-11206.
Southern, E. M. (1975). Detection of specific sequences among DNA fragments separated by gel electrophoresis. J. Mol. Biol. 98: 503-517.
Sun, T.Q., Fernstermacher, D.A. and Vos, J.M. (1994) Human artificial episomal chromosomes for cloning large DNA fragments in human cells. Nat. Genet. 8, 33-41.
Talbot, D., Philipsen, S., Fraser, P. and Grosveld, F. (1990) Detailed analysis of the site 3 region of the human β-globin dominant control region, EMBO J., 9: 2169-2178.
Tamura T, Osaka F, Kawamura Y, Higuti T, Ishida N, Nothwang HG, Tsurumi C, Tanaka K, Ichihara A (1994) Isolation and characterization of alpha-type HC3 and betatype HC5 subunit genes of human proteasomes. J. Mol. Biol. 244: 117-124.
Tartof, K. D., and Hobbs, C. A. (1987). Improved media for growing plasmid and cosmid clones. Bethesda Res. Lab. Focus 9: 12.
Tewari, R., Gillemans, N., Wijerde, M., Nuez, B., von Lindem, M., Grosveld, F. and Philipsen, S. (1998) Erythroid Krüppel-like factor (EKLF) is active in primitive and definitive erythroid cells and is required for the function of 5'HS3 of the b-globin locus control region. EMBO J. 17: 2334-2341.
Trachtulec Z, Hamvas RM, Forejt J, Lehrach HR, Vincek V, Klein J (1997) Linkage of TATA-binding protein and proteasome subunit C5 genes in mice and humans reveals synteny conserved between mammals and invertebrates. Genomics 44: 1-7.
Vyas P, Vickers MA, Simmons DL, Ayyub H, Craddock CF, Higgs DR (1992) Cis-acting sequences regulating expression of the human alpha-globin cluster lie within constitutively open chromatin. Cell 69: 781-793.
Wijgerde, M., Grosveld, F. and Fraser, P. (1995). Transcription complex stability and chromatin dynamics in vivo. Nature 377: 209-213.
Winston, J. H., Hanten, G. R., Overbeek, P. A., and Kellems, R. E. (1992) 5' flanking sequences of the murine adenosine deaminase gene direct expression of a reporter gene to specific prenatal and postnatal tissues in transgenic mice. J. Biol. Chem. 267, 13472-13479.
Winston, J. H., Hong, L., Datta, S.K. and Kellems, R. E. (1996) An intron 1 regulatory region from the murine adenosine deaminase gene can activate heterologous promoters for ubiquitous expression in transgenic mice. Som. Cell Mol. Genet. 22: 261-278.
Yamashita, T., Kasai, N., Miyoshi, I., Sasaki, N., Maki, K., Sakai, M., Nishi, S. and Namioka, S. (1993) High level expression of human alpha-fetoprotein in transgenic mice. Biochem. Biophys. Res. Comm. 191: 715-720.
Yates, J.L., Warren, N. and Sugden, B. (1985) Stable replication of plasmids derived from Epstein-Barr virus in various mammalian cells. Nature 313: 812-815.
Zhumabekov T, Corbella P, Tolaini M, Kioussis D (1995) Improved version of a human CD2 minigene based vector for T cell-specific expression in transgenic mice. J Immunol Methods 185: 133-140.

## Claims

1. A method of obtaining a desired gene product comprising culturing a host cell, wherein the host cell comprises a vector, and the vector comprises a polynucleotide, the polynucleotide comprising:
(i) an extended methylation-free CpG-island comprising dual or bidirectional promoters that are divergently transcribed, wherein the extended methylation-free CpG-island extends for more than 300bp and further comprises an average GC content of 60%;
(ii) an expressible gene encoding a desired gene product, wherein the expressible gene is operably-linked to the CpG-island; and
(iii) a promoter, operably-linked to the expressible gene, wherein the promoter is not naturally operably-linked to the CpG-island;
wherein the extended methylation-free CpG island opens chromatin or maintains chromatin in an open state and facilitates reproducible activation of transcription of the expressible gene.

2. The method of claim 1, wherein the extended methylation-free CpG island comprises at least one endogenous promoter.

3. The method of claim 1 or claim 2, wherein the extended methylation-free CpG island comprises dual promoters that transcribe divergently.

4. The method of any of claims 1 to 3, wherein the extended methylation-free CpG island extends for more than 500 bp.

5. The method of any of claims 1 to 4, wherein the extended methylation-free CpG island comprises a 44kb DNA fragment spanning the human TATA binding protein gene and 12kb each of the 5' and 3' flanking sequence.

6. The method of any of claims 1 to 4, wherein the extended methylation-free CpG island comprises a 25kb DNA fragment spanning the human TATA binding protein gene with 1 kb 5' and 5kb 3' flanking sequence.

7. The method of any of claims 1 to 4, wherein the extended methylation-free CpG island comprises the sequence according to Figure 21 or a functional fragment thereof.

8. The method of any of claims 1 to 4, wherein the extended methylation-free CpG island comprises a 60kb DNA fragment spanning the human heterogeneous nuclear ribonucleoprotein A2 gene with 30kb 5' and 20kb 3' flanking sequence.

9. The method of any of claims 1 to 4, wherein the extended methylation-free CpG island comprises a 16kb DNA fragment spanning the human heterogeneous nuclear ribonucleoprotein A2gene with 5kb 5' and 1.5kb 3' flanking sequence.

10. The method of any of claims 1 to 9, wherein the vector comprises a heterologous promoter which is a tissue specific promoter or a substantially ubiquitous promoter.

11. The method of claim 10, wherein the promoter is a substantially ubiquitous promoter and is a CMV promoter.

12. The method of any of claims 1 to 11, wherein the expressible gene encodes a therapeutic polypeptide.

13. The method of claim 12, wherein the expressible gene encodes a therapeutic antibody or fragment thereof.

14. The method of any of claims 1 to 13 wherein the extended methylation-free CpG-island increases the expression of the operably linked expressible gene.

15. Use of an isolated polynucleotide comprising an extended methylation-free CpG island to increase the expression of an endogenous gene, said use comprising inserting the polynucleotide into the genome of a cell in a position operably associated with the endogenous gene thereby increasing the level of expression of the gene;
wherein the endogenous gene is operably-linked to a promoter that is not naturally operably-linked to the extended methylation-free CpG island;
wherein the extended methylation-free CpG island comprises dual or bidirectional promoters that are divergently transcribed;
wherein the extended methylation-free CpG island extends for more than 300bp and further comprises an average GC content of 60%; and
wherein the extended methylation-free CpG island opens chromatin or maintains chromatin in an open state and facilitates reproducible activation of transcription of the endogenous gene.

16. A polynucleotide comprising:
(i) an extended methylation-free CpG-island comprising dual or bidirectional promoters that are divergently transcribed, wherein the extended methylation-free CpG-island extends for more than 300bp and further comprises an average GC content of 60%;
(ii) an expressible gene encoding a desired gene product, wherein the expressible gene is operably-linked to the CpG-island; and
(iii) a promoter, operably-linked to the expressible gene, wherein the promoter is not naturally operably-linked to said CpG-island;
for use as a medicament, wherein the extended methylation-free CpG island opens chromatin or maintains chromatin in an open state and facilitates reproducible activation of transcription of the expressible gene.

17. A polynucleotide comprising:
(i) an extended methylation-free CpG-island comprising dual or bidirectional promoters that are divergently transcribed , wherein the extended methylation-free CpG-island extends for more than 300bp and further comprises an average GC content of 60%;
(ii) an expressible gene encoding a desired gene product, wherein the expressible gene is operably-linked to the CpG-island; and
(iii) a promoter, operably-linked to the expressible gene, wherein the promoter is not naturally operably-linked to the CpG-island;
for use in the treatment of Duchenne muscular dystrophy, wherein the extended methylation-free CpG island opens chromatin or maintains chromatin in an open state and facilitates reproducible activation of transcription of the expressible gene.

18. An isolated polynucleotide comprising:
(i) an extended methylation-free CpG island comprising dual or bidirectional promoters that are divergently transcribed , wherein the extended methylation-free CpG-island extends for more than 300bp and further comprises an average GC content of 60%;
(ii) a nucleotide sequence encoding a therapeutic antibody, wherein the nucleotide sequence is operably-linked to the extended methylation-free CpG island; and
(iii) a heterologous promoter operably linked to the nucleotide sequence;
wherein the extended methylation-free CpG island opens chromatin or maintains chromatin in an open state and facilitates reproducible activation of transcription of the nucleotide sequence.

19. A vector comprising a polynucleotide as claimed in any one of claims 16 to 18.

## Patentansprüche

1. Verfahren zum Erhalten eines gewünschten Genprodukts, welches das Kultivieren einer Wirtszelle umfasst, wobei die Wirtszelle einen Vektor umfasst, und wobei der Vektor ein Polynulcleotid umfasst, wobei das Polynukleotid folgendes umfasst:
(i) eine erweiterte methylierungsfreie CpG-Insel, die duale oder bidirektionale, divergent transkribierte Promoter umfasst, wobei sich die erweiterte methylierungsfreie CpG-Insel über mehr als 300 bp erstreckt, und wobei sie ferner einen durchschnittlichen GC-Anteil von 60 % aufweist;
(ii) ein exprimierbares Gen, das für ein gewünschtes Genprodukt codiert, wobei das exprimierbare Gen funktionsfähig mit der CpG-Insel verknüpft ist; und
(iii) einen Promoter, der funktionsfähig mit dem exprimierbaren Gen verknüpft ist, wobei der Promoter nicht natürlich mit der CpG-Insel funktionsfähig verknüpft ist;
wobei die erweiterte methylierungsfreie CpG-Insel Chromatin öffnet oder Chromatin in einem offenen Zustand hält und die reproduzierbare Aktivierung der Transkription des exprimierbaren Gens erleichtert.

2. Verfahren nach Anspruch 1, wobei die erweiterte methylierungsfreie CpG-Insel mindestens einen endogenen Promoter umfasst.

3. Verfahren nach Anspruch 1 oder Anspruch 2, wobei die erweiterte methylierungsfreie CpG-Insel duale Promoter umfasst, die divergent transkribieren.

4. Verfahren nach einem der Ansprüche 1 bis 3, wobei sich die erweiterte methylierungsfreie CpG-Insel über mehr als 500 bp erstreckt.

5. Verfahren nach einem der Ansprüche 1 bis 4, wobei die erweiterte methylierungsfreie CpG-Insel ein DNA-Fragment von 44 kb umfasst, das das menschliche TATA-Bindungsproteingen umspannt und jeweils 12 kb der 5'- und 3'-Flankierungssequenz.

6. Verfahren nach einem der Ansprüche 1 bis 4, wobei die erweiterte methylierungsfreie CpG-Insel ein DNA-Fragment von 25 kb umfasst, das das menschliche TATA-Bindungsproteingen umspannt, mit einer 1 kb 5'- und 5 kb 3'-Flankierungssequenz.

7. Verfahren nach einem der Ansprüche 1 bis 4, wobei die erweiterte methylierungsfreie CpG-Insel die Sequenz aus Figur 21 umfasst oder ein funktionales Fragment dieser.

8. Verfahren nach einem der Ansprüche 1 bis 4, wobei die erweiterte methylierungsfreie CpG-Insel ein DNA-Fragment von 60 kb umfasst, das das menschliche heterogene nukleare Ribonukleoprotein-A2-Gen umspannt, mit einer 30 kb 5'- und 20 kb 3'-Flankierungssequenz.

9. Verfahren nach einem der Ansprüche 1 bis 4, wobei die erweiterte methylierungsfreie CpG-Insel ein DNA-Fragment von 16 kb umfasst, das das menschliche heterogene nukleare Ribonukleoprotein-A2-Gen umspannt, mit einer 5 kb 5'- und 1,5 kb 3'-Flankierungssequenz.

10. Verfahren nach einem der Ansprüche 1 bis 9, wobei der Vektor einen heterologen Promoter umfasst, bei dem es sich um einen gewebespezifischen Promoter oder um einen im Wesentlichen ubiquitären Promoter handelt.

11. Verfahren nach Anspruch 10, wobei es sich bei dem Promoter um einen im Wesentlichen ubiquitären Promoter und einen CMV-Promoter handelt.

12. Verfahren nach einem der Ansprüche 1 bis 11, wobei das exprimierbare Gen für ein therapeutisches Polypeptid codiert.

13. Verfahren nach Anspruch 12, wobei das exprimierbare Gen für einen therapeutischen Antikörper oder ein Fragment dessen codiert.

14. Verfahren nach einem der Ansprüche 1 bis 13, wobei die erweiterte methylierungsfreie CpG-Insel die Expression des funktionsfähig verknüpften exprimierbaren Gens erhöht.

15. Verwendung eines isolierten Polynukleotids, das eine erweiterte methylierungsfreie CpG-Insel umfasst, um die Expression eines endogenen Gens zu erhöhen, wobei die genannte Verwendung das Einführen des Polynukleotids in das Genom einer Zelle an eine Position umfasst, die funktionsfähig dem endogenen Gen zugeordnet ist, wodurch das Maß der Expression des Gens erhöht wird;
wobei das endogene Gen funktionsfähig mit einem Promoter verknüpft ist, der nicht natürlich funktionsfähige mit der erweiterten methylierungsfreien CpG-Insel verknüpft ist;
wobei die erweiterte methylierungsfreie CpG-Insel duale oder bidirektionale Promoter umfasst, die divergent transkribiert sind;
wobei sich die erweiterte methylierungsfreie CpG-Insel über mehr als 300 bp erstreckt, und wobei sie ferner einen durchschnittlichen GC-Anteil von 60 % aufweist; und
wobei die erweiterte methylierungsfreie CpG-Insel Chromatin öffnet oder Chromatin in einem offenen Zustand hält und die reproduzierbare Aktivierung der Transkription des endogenen Gens erleichtert.

16. Polynukleotid, das folgendes umfasst:
(i) eine erweiterte methylierungsfreie CpG-Insel, die duale oder bidirektionale, divergent transkribierte Promoter umfasst, wobei sich die erweiterte methylierungsfreie CpG-Insel über mehr als 300 bp erstreckt, und wobei sie ferner einen durchschnittlichen GC-Anteil von 60 % aufweist;
(ii) ein exprimierbares Gen, das für ein gewünschtes Genprodukt codiert, wobei das exprimierbare Gen funktionsfähig mit der CpG-Insel verknüpft ist; und
(iii) einen Promoter, der funktionsfähig mit dem exprimierbaren Gen verknüpft ist, wobei der Promoter nicht natürlich mit der CpG-Insel funktionsfähig verknüpft ist;
zur Verwendung als ein Arzneimittel, wobei die erweiterte methylierungsfreie CpG-Insel Chromatin öffnet oder Chromatin in einem offenen Zustand hält und die reproduzierbare Aktivierung der Transkription des exprimierbaren Gens erleichtert.

17. Polynukleotid, das folgendes umfasst:
(i) eine erweiterte methylierungsfreie CpG-Insel, die duale oder bidirektionale, divergent transkribierte Promoter umfasst, wobei sich die erweiterte methylierungsfreie CpG-Insel über mehr als 300 bp erstreckt, und wobei sie ferner einen durchschnittlichen GC-Anteil von 60 % aufweist;
(ii) ein exprimierbares Gen, das für ein gewünschtes Genprodukt codiert, wobei das exprimierbare Gen funktionsfähig mit der CpG-Insel verknüpft ist; und
(iii) einen Promoter, der funktionsfähig mit dem exprimierbaren Gen verknüpft ist, wobei der Promoter nicht natürlich mit der CpG-Insel funktionsfähig verknüpft ist;
zur Verwendung in der Behandlung der Duchenne Muskeldystrophie, wobei die erweiterte methylierungsfreie CpG-Insel Chromatin öffnet oder Chromatin in einem offenen Zustand hält und die reproduzierbare Aktivierung der Transkription des exprimierbaren Gens erleichtert.

18. Isoliertes Polynukleotid, das folgendes umfasst:
(i) eine erweiterte methylierungsfreie CpG-Insel, die duale oder bidirektionale, divergent transkribierte Promoter umfasst, wobei sich die erweiterte methylierungsfreie CpG-Insel über mehr als 300 bp erstreckt, und wobei sie ferner einen durchschnittlichen GC-Anteil von 60 % aufweist;
(ii) eine Nukleotidsequenz, die für einen therapeutischen Antikörper codiert, wobei die Nukleotidsequenz funktionsfähig mit der erweiterten methylierungsfreien CpG-Insel verknüpft ist; und
(iii) einen heterologen Promoter, der funktionsfähig mit der Nukleotidsequenz verknüpft ist;
wobei die erweiterte methylierungsfreie CpG-Insel Chromatin öffnet oder Chromatin in einem offenen Zustand hält und die reproduzierbare Aktivierung der Transkription des exprimierbaren Gens erleichtert.

19. Vektor, der ein Polynukleotid nach einem der Ansprüche 16 bis 18 umfasst.

## Revendications

1. Procédé d'obtention d'un produit génique souhaité comprenant la mise en culture d'une cellule hôte, dans lequel la cellule hôte comprend un vecteur et le vecteur comprend un polynucléotide, le polynucléotide comprenant :
(i) un îlot CpG étendu exempt de méthylation comprenant des promoteurs doubles ou bidirectionnels qui sont transcrits de manière divergente, dans lequel l'îlot CpG étendu exempt de méthylation s'étend sur plus de 300 pb et comprend en outre une teneur en GC moyenne de 60 % ;
(ii) un gène exprimable codant pour un produit génique souhaité, dans lequel le gène exprimable est lié de manière opérationnelle à l'îlot CpG ; et
(iii) un promoteur lié de manière opérationnelle au gène exprimable, dans lequel le promoteur n'est pas naturellement lié de manière opérationnelle à l'îlot CpG ;
dans lequel l'îlot CpG étendu exempt de méthylation ouvre de la chromatine ou maintient la chromatine dans un état ouvert et facilite l'activation reproductible de la transcription du gène exprimable.

2. Procédé selon la revendication 1, dans lequel l'îlot CpG étendu exempt de méthylation comprend au moins un promoteur endogène.

3. Procédé selon la revendication 1 ou la revendication 2, dans lequel l'îlot CpG étendu exempt de méthylation comprend des promoteurs doubles qui transcrivent de manière divergente.

4. Procédé selon l'une quelconque des revendications 1 à 3, dans lequel l'îlot CpG étendu exempt de méthylation s'étend sur plus de 500 pb.

5. Procédé selon l'une quelconque des revendications 1 à 4, dans lequel l'îlot CpG étendu exempt de méthylation comprend un fragment d'ADN de 44 kb couvrant le gène de la protéine de liaison TATA humaine et 12 kb de chacune des séquences flanquantes en 5' et 3'.

6. Procédé selon l'une quelconque des revendications 1 à 4, dans lequel l'îlot CpG étendu exempt de méthylation comprend un fragment d'ADN de 25 kb couvrant le gène de la protéine de liaison TATA humaine avec 1 kb de la séquence flanquante en 5' et 5 kb de la séquence flanquante en 3'.

7. Procédé selon l'une quelconque des revendications 1 à 4, dans lequel l'îlot CpG étendu exempt de méthylation comprend la séquence selon la Figure 21 ou un fragment fonctionnel de celle-ci.

8. Procédé selon l'une quelconque des revendications 1 à 4, dans lequel l'îlot CpG étendu exempt de méthylation comprend un fragment d'ADN de 60 kb couvrant le gène A2 de ribonucléoprotéine nucléaire hétérogène humaine avec 30 kb de la séquence flanquante en 5' et 20 kb de la séquence flanquante en 3'.

9. Procédé selon l'une quelconque des revendications 1 à 4, dans lequel l'îlot CpG étendu exempt de méthylation comprend un fragment d'ADN de 16 kb couvrant le gène A2 de ribonucléoprotéine nucléaire hétérogène humaine avec 5 kb de la séquence flanquante en 5' et 1,5 kb de la séquence flanquante en 3'.

10. Procédé selon l'une quelconque des revendications 1 à 9, dans lequel le vecteur comprend un promoteur hétérologue qui est un promoteur spécifique à un tissu ou un promoteur essentiellement ubiquitaire.

11. Procédé selon la revendication 10, dans lequel le promoteur est un promoteur essentiellement ubiquitaire et est un promoteur de CMV.

12. Procédé selon l'une quelconque des revendications 1 à 11, dans lequel le gène exprimable code pour un polypeptide thérapeutique.

13. Procédé selon la revendication 12, dans lequel le gène exprimable code pour un anticorps thérapeutique ou fragment de celui-ci.

14. Procédé selon l'une quelconque des revendications 1 à 13, dans lequel l'îlot CpG étendu exempt de méthylation augmente l'expression du gène exprimable lié de manière opérationnelle.

15. Utilisation d'un polynucléotide isolé comprenant un îlot CpG étendu exempt de méthylation pour augmenter l'expression d'un gène endogène, ladite utilisation comprenant l'insertion du polynucléotide dans le génome d'une cellule en une position associée de manière opérationnelle au gène endogène, augmentant de ce fait le niveau d'expression du gène ;
dans laquelle le gène endogène est lié de manière opérationnelle à un promoteur qui n'est pas naturellement lié de manière opérationnelle à l'îlot CpG étendu exempt de méthylation ; dans laquelle l'îlot CpG étendu exempt de méthylation comprend des promoteurs doubles ou bidirectionnels qui sont transcrits de manière divergente ;
dans laquelle l'îlot CpG étendu exempt de méthylation s'étend sur plus de 300 pb et comprend en outre une teneur en GC moyenne de 60 % ; et
dans laquelle l'îlot CpG étendu exempt de méthylation ouvre de la chromatine ou maintient la chromatine dans un état ouvert et facilite l'activation reproductible de la transcription du gène endogène.

16. Polynucléotide comprenant :
(i) un îlot CpG étendu exempt de méthylation comprenant des promoteurs doubles ou bidirectionnels qui sont transcrits de manière divergente, dans lequel l'îlot CpG étendu exempt de méthylation s'étend sur plus de 300 pb et comprend en outre une teneur en GC moyenne de 60 % ;
(ii) un gène exprimable codant pour un produit génique souhaité, dans lequel le gène exprimable est lié de manière opérationnelle à l'îlot CpG ; et
(iii) un promoteur lié de manière opérationnelle au gène exprimable, dans lequel le promoteur n'est pas naturellement lié de manière opérationnelle audit îlot CpG ;
destiné à être utilisé comme médicament, dans lequel l'îlot CpG étendu exempt de méthylation ouvre de la chromatine ou maintient la chromatine dans un état ouvert et facilite l'activation reproductible de la transcription du gène exprimable.

17. Polynucléotide comprenant:
(i) un îlot CpG étendu exempt de méthylation comprenant des promoteurs doubles ou bidirectionnels qui sont transcrits de manière divergente, dans lequel l'îlot CpG étendu exempt de méthylation s'étend sur plus de 300 pb et comprend en outre une teneur en GC moyenne de 60 % ;
(ii) un gène exprimable codant pour un produit génique souhaité, dans lequel le gène exprimable est lié de manière opérationnelle à l'îlot CpG ; et
(iii) un promoteur lié de manière opérationnelle au gène exprimable, dans lequel le promoteur n'est pas naturellement lié de manière opérationnelle à l'îlot CpG ;
destiné à être utilisé dans le traitement de la dystrophie musculaire de Duchenne, dans lequel l'îlot CpG étendu exempt de méthylation ouvre de la chromatine ou maintient la chromatine dans un état ouvert et facilite l'activation reproductible de la transcription du gène exprimable.

18. Polynucléotide isolé comprenant :
(i) un îlot CpG étendu exempt de méthylation comprenant des promoteurs doubles ou bidirectionnels qui sont transcrits de manière divergente, dans lequel l'îlot CpG étendu exempt de méthylation s'étend sur plus de 300 pb et comprend en outre une teneur en GC moyenne de 60 % ;
(ii) une séquence nucléotidique codant pour un anticorps thérapeutique, dans lequel la séquence nucléotidique est liée de manière opérationnelle à l'îlot CpG étendu exempt de méthylation ; et
(iii) un promoteur hétérologue lié de manière opérationnelle à la séquence nucléotidique ; dans lequel l'îlot CpG étendu exempt de méthylation ouvre de la chromatine ou maintient la chromatine dans un état ouvert et facilite l'activation reproductible de la transcription de la séquence nucléotidique.

19. Vecteur comprenant un polynucléotide selon l'une quelconque des revendications 16 à 18.
